# EUROPÄISCHE PATENTSCHRIFT

(11) **EP 0 415 196 B1**
(45) Veröffentlichungstag und Bekanntmachung des Hinweises auf die Patenterteilung: **24.03.1993**
(21) Anmeldenummer: 90115742.0
(22) Anmeldetag: 17.08.1990
(51) Int. Cl.: C07D 249/14, C07D 405/12, A01N 47/38

(54) **Substituierte 4,5-Diamino-1,2,4-triazol-3-(thi)one**
Substituted 4,5-diamino-1,2,4-triazol-3-(thi)ones
4,5-Diamino-1,2,4-triazol-3-(thi)ones substituées

(30) Priorität: 30.08.1989 DE 3928662
(43) Veröffentlichungstag der Anmeldung: 06.03.1991
(73) Patentinhaber: BAYER AG, 51368 Leverkusen (DE)
(72) Erfinder: Müller, Klaus-Helmut, Dr., D-4000 Düsseldorf 13 (DE); Findeisen, Kurt, Dr., D-5090 Leverkusen 1 (DE); Haug, Michael, Dr., D-5060 Bergisch Gladbach 2 (DE); Heinemann, Ulrich, Dr., D-5653 Leichlingen 1 (DE); Kluth, Joachim, Dr., D-4018 Langenfeld (DE); König, Klaus, Dr., D-5068 Odenthal (DE); Santel, Hans-Joachim, Dr., D-5090 Leverkusen 1 (DE); Lürssen, Klaus, Dr., D-5060 Bergisch Gladbach 2 (DE); Schmidt, Robert, Dr., D-5060 Bergisch Gladbach 2 (DE)

(56) Entgegenhaltungen:
- EP-A- 0 283 876
- EP-A- 0 294 666
- PATENT ABSTRACTS OF JAPAN, Band 2, Nr. 24, 16. Februar 1978, Seite 4198 C 77; & JP-A-52 125 168 (NIPPON SODA K.K.) 10-02-1977
- JOURNAL OF THE CHEMICAL SOCIETY, (C), 1968, Seiten 2099-2107, London, GB; F. KURZER et al.: "Heterocyclic compounds from urea derivatives. Part XIV. The interaction of thiocarbohydrazide and diarylcarbodi-imides"
- EUROPEAN JOURNAL OF MEDICINAL CHEMISTRY - CHIMICA THERAPEUTICA, Band 21, Nr. 3, 1986, Seiten 235-244, Paris, FR; H. EMILSSON et al.: "Synthesis and antihypertensive activity of 5-substituted 3-amino-4-(2,6-dichlorobenzylideneamino)-4H-1,2,4-triazoles and the corresponding 3,4-diamino-4H-1,2,4-triazoles"
- JUSTUS LIEBIGS ANNALEN DER CHEMIE, Band 702, 21. März 1967, Seiten 101-111, Weinheim, DE; H. GEHLEN et al.: "Über die Bildung von 1-acyl-5-carbamoyl-bwz.-thiocarbamoyl-diaminoguanidinen und ihr Verhalten beim Kochen in Wasser und in Salzsäure"

## Beschreibung

Die Erfindung betrifft neue substituierte 4,5-Diamino-1,2,4-triazol-3-(thi)one, mehrere Verfahren und neue Zwischenprodukte zu ihrer Herstellung sowie ihre Verwendung als Herbizide.

Es ist bekannt, daß bestimmte substituierte Triazolone, wie z.B. 4-Amino-5-methyl-2-phenylaminocar- bonyl-2,4-dihydro-3H-1,2,4-triazol-3-on, herbizide Eigenschaften aufweisen (vgl. EP-A294666). Die Herbizidwirkung dieser bekannten Verbindung ist jedoch nicht in allen Belangen zufriedenstellend. Weiterhin werden in der EP-A 28 38 76 substituierte Triazolinone und deren herbizide Eigenschaften beschrieben.

Es wurden nun neue substituierte 4,5-Diamino-1,2,4-Triazol-3-(thi)one der allgemeinen Formel (I) 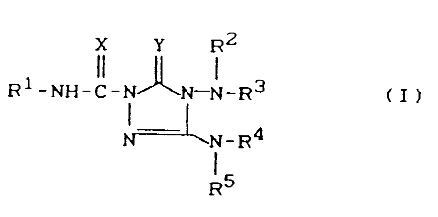 in welcher
R¹ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, für Phenoxyalkyl mit 1 bis 4 Kohlenstoffatomen im Alkylteil, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für jeweils gegebenenfalls einfache bis dreifache gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl-bzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oderverschiedenene Halogenatomen und geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; R¹ außerdem für im Heterocyclylteil gegebenenfalls einfach bis dreifach, gleich oder verschieden substituiertes Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro , sowie jeweils geradkettiges oder verzweigtes Alkyl, Alkoxy, Alkylthio, Halogenalkyl, Halogenalkoxy, Halogenalkylthio oderAlkoxycarbonyl mit jeweils 1 bis 5 Kohlenstoffatomen und gegebenenfalls 1 bis 9 gleichen oder verschiedenen Halogenatomen; R¹ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen oder für jeweils gegebenenfalls einfach bis dreifach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im geradkettigen oder verzweigten Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oderAlkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder R¹ für Benzyl mit im Phenylteil ankondensierter -O-CH₂-O- Gruppe steht,
R², R³, R⁴ und R⁵ gleich oder verschieden sind und unabhängig voneinander für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanoalkyl mit 1 bis 8 Kohlenstoffatomen, Alkoxyalkyl oder Alkylthioalkyl mit jeweils bis zu 4 Kohlenstoffatomen in den einzelnen Alkylteilen, für jeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen, die Reste R² bis R⁵ weiter für jeweils gegebenenfalls einfach bis dreifach gleich oder verschieden substituiertes Aryl oder Aralkyl stehen mit jeweils 6 oder 10 Kohlenstoffatomen im Arylteil und gegebenenfalls 1 bis 4 Kohlenstoffatomen im geradkettigen oder verzweigten Alkylteil, wobei als Substituenten infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio,Alkylsulfinyl,Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 Halogenatomen, in welcher weiter auch jeweils zwei dieser Reste - R² und R³ oder R⁴ und R^{5 -} zusammen für geradkettiges oder verzweigtes Alkandiyl oder Oxaalkandiyl mit jeweils 2 bis 6 Kohlenstoffatomen stehen können, und R⁵ auch fürgeradkettiges oderverzweigtes Alkoxy mit 1 bis 8 Kohlenstoffatomen stehen kann,
X für Sauerstoff oder Schwefel steht und
Y für Sauerstoff oder Schwefel steht,

gefunden.

Die Verbindungen der Formel (I) können gegebenenfalls in Abhängigkeit von der Art der Substituenten R¹, R², R³, R⁴ und R⁵ als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiter wurde gefunden, daß man die neuen substituierten 4,5-Diamino-1,2,4-triazol-3-(thi)one der allgemeinen Formel (I) 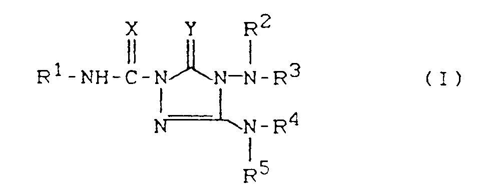 in welcher
R¹, R², R³, R⁴, R⁵, X und Y
   die oben genannten Bedeutungen haben, erhält, wenn man
   (a) 4,5-Diamino-1,2,4-triazol-3-(thi)one der allgemeinen Formel (11) 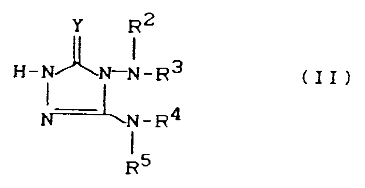 in welcher
      R², R³, R⁴, R⁵ und Y die oben angegebenen Bedeutungen haben,
         mit Iso(thio)cyanaten der allgemeinen Formel (III) 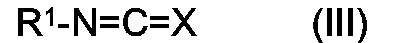 in welcher
      R¹ und X die oben angegebenen Bedeutungen haben,

      gegebenenfalls in Gegenwart eines Verdünnungsmittels und gegebenenfalls in Gegenwart eines Reaktionshilfsmittels umsetzt,
      oder wenn man
   (b) 4-Alkylidenamino-5-amino-1,2,4-triazol-3-(thi)one der allgemeinen Formel (IV) 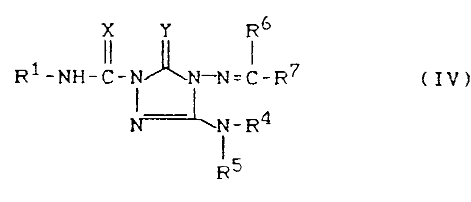 in welcher
      R¹, R⁴, R⁵, X und Y die oben angegebenen Bedeutungen haben und
      R⁶ und R⁷ gleich oder verschieden sind und unabhängig voneinanderfürWasserstoff, geradkettiges oder verzweigtes Alkyl mit 1 bis 4 Kohlenstoffatomen, Phenyl oder Benzyl stehen

      mit wäßrigen Säuren, gegebenenfalls in Gegenwart organischer Lösungsmittel, umsetzt,
      oder wenn man
   (c) substituierte 1,2,4-Triazol-3-(thi)one der allgemeinen Formel (V) 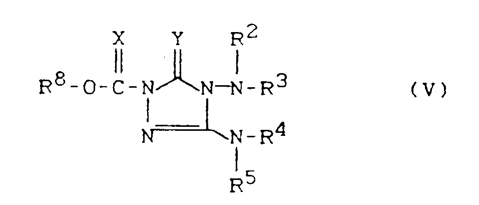 in welcher
      R², R³, R⁴, R⁵, X und Y die oben angegebenen Bedeutungen haben und
      R⁸ für Methyl, Ethyl, Propyl, Isopropyl, Butyl, Isobutyl, sec-Butyl, tert.- Butyl, Phenyl oder Benzyl steht,

      mit Aminoverbindungen der allgemeinen Formel (VI) 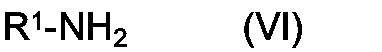 in welcher
      R¹ die oben angegebene Bedeutung hat,

      gegebenenfalls in Gegenwart eines Verdünnungsmittels und gegebenenfalls in Gegenwart eines Reaktionshilfsmittels umsetzt,
      oder wenn man
   (d) 4,5-Diamino-1,2,4-triazol-3-(thi)one der allgemeinen Formel (II) 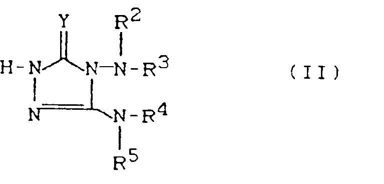 in welcher
      R², R³, R⁴, R⁵ und Y die oben angegebenen Bedeutungen haben,
         mit (Thio)Urethanen der allgemeinen Formel (VII) 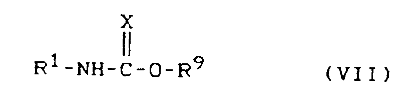 in welcher
      R¹ und X die oben angegebenen Bedeutungen haben und
      R⁹ für Methyl, Ethyl, Propyl, Isopropyl, Butyl, Isobutyl sec-Butyl, tert.-Butyl, Phenyl oder Benzyl steht,

      gegebenenfalls in Gegenwart eines Verdünnungsmittels und gegebenenfalls in Gegenwart eines Reaktionshilfsmittels umsetzt,
      oder wenn man
   (e) 4-Oxyalkylidenamino-5-amino-1,2,4-triazol-3-(thi)one der allgemeinen Formel (VIII) 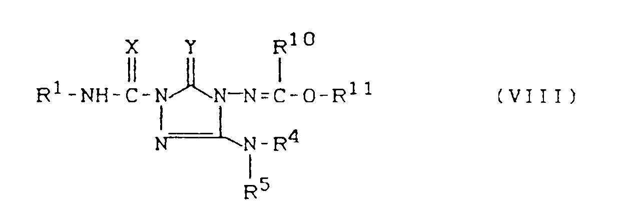 in welcher
      R¹, R⁴, R⁵, X und Y die oben angegebenen Bedeutungen haben und
      R¹⁰ für Wasserstoff, Methyl, Ethyl, Propyl, Phenyl, oder Benzyl und
      R11 für Methyl, Ethyl, Propyl oder Benzyl steht,

      mit Hydridkomplexen der allgemeinen Formel (IX) 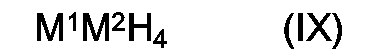 in welcher
      M¹ für Lithium, Natrium oder Kalium steht und
      M² für Bor oder Aluminium steht,

      gegebenenfalls in Gegenwart eines Verdünnungsmittels umsetzt,
      oder wenn man
   (f) 4-Alkylidenamino-5-amino-1,2,4-triazol-3-(thi)one der allgemeinen Formel (IV) 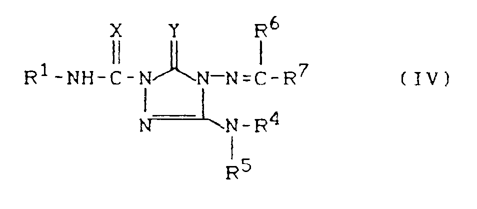 in welcher
      R¹, R⁴, R⁵, X und Y die oben angegebenen Bedeutungen haben und
      R⁶ und R⁷ gleich oder verschieden sind und unabhängig voneinander, die oben angegebene Bedeutung haben,

      mit Reduktionsmitteln, gegebenenfalls in Gegenwart von Katalysatoren und gegebenenfalls in Gegenwart von Verdünnungsmitteln umsetzt.

Schließlich wurde gefunden, daß die neuen substituierten 4,5-Diamino-1,2,4-triazol-3-one der allgemeinen Formel (I) interessante herbizide Eigenschaften aufweisen.

Überraschenderweise zeigen die erfindungsgemäßen substituierten 4,5-Diamino-1 ,2,4-triazol-3-one der allgemeinen Formel (I) erheblich stärkere Herbizidwirkung gegenüber Problemunkräutern als die nach Struktur und Wirkprofil naheliegende Verbindung 4-Amino-5-methyl-2-phenylaminocarbonyl-2,4-dihydro-3H-1,2,4-triazol-3-on.

In den Definitionen stehen die aromatischen Reste wie beispielsweise Aryl, Aryloxy, Aralkyl vorzugsweise für Phenyl oder Naphthyl, insbesondere für Phenyl. Die aliphatischen Kohlenstoffketten sind, auch wenn es nicht ausdrücklich angegeben ist, jeweils geradkettig oder verzweigt.

Der Substituent Heterocyclylalkyl in R¹ bedeutet vorzugsweise Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen -insbesondere Stickstoff, Sauerstoff und/oder Schwefel - im Heterocyclylteil, wobei der Heterocyclylteil einfach oder mehrfach, insbesondere einfach, zweifach oder dreifach, gleich oder verschieden substituiert sein kann mit Halogen, Cyano, Nitro sowie Cₗ-C₅-Alkyl, C₁-C₅-Alkoxy, C₁-C₅-Alkylthio, Halogen-C₁-C₅-alkyl, Halogen-C₁-C₅-alkoxy, Halogen-C₁-C₅-alkylthio oder C₁-C₅-Alkoxycarbonyl. Der Heterocyclylteil kann insbesondere substituiert sein mit Fluor, Chlor, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio. Besonders bevorzugt steht der Substituent Heterocyclylalkyl in R¹ für im Heterocyclylteil gegebenenfalls ein bis dreifach, gleich oderverschieden substituiertes Heterocyclylmethyl, Heterocyclylpropyl oder Heterocyclylethyl, wobei als Heterocyclen jeweils infrage kommen: 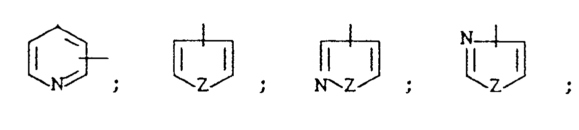 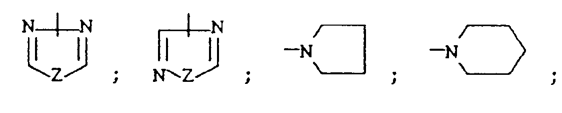 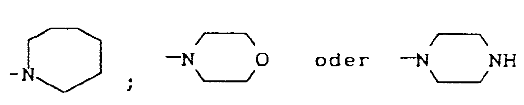 wobei Zjeweils für Sauerstoff oder Schwefel steht und wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio.

Die erfindungsgemäßen substituierten 4,5-Diamino-1,2,4-triazol-3-(thi)one sind durch die Formel (I) allgemein definiert. Bevorzugt sind die Verbindungen der Formel (1), bei welchen
R¹ für Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl, Dodecyl, für Allyl, jeweils geradkettiges oder verzweigtes Butenyl, Pentenyl oder Hexenyl, Propargyl, jeweils geradkettiges oder verzweigtes Butinyl, Pentinyl oder Hexinyl, für geradkettiges oder verzweigtes Halogenalkyl mit 1 bis 8 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 8 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxy-alkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom, Methyl, Ethyl, n- oder i-Propyl, n-, i-, s- oder t-Butyl, Cyano, Methandiyl, Ethandiyl, Butandiyl oder Butadiendiyl oder Dichlorallyl;
R¹ weiterhin für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen: 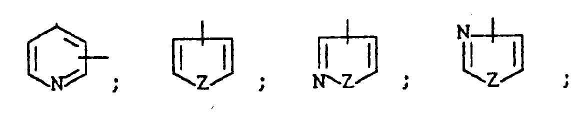 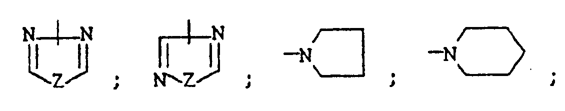 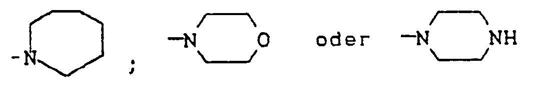 wobei Z jeweils für Sauerstoff oder Schwefel steht und wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
R¹ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl steht, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy,
R² für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl, Cyclohexyl, Phenyl oder Benzyl steht,
R³ für Wasserstoff, Methyl, Ethyl, Propyl, Butyl, Allyl, Propargyl, Cyanoethyl, Methoxyethyl oder gegebenenfalls durch Chlor substituiertes Benzyl steht, oder zusammen mit R² für Butan-1,4-diyl oder Pentan-1,5-diyl steht,
R⁴ für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl, Cyclohexyl, Phenyl oder Benzyl steht,
R⁵ für Wasserstoff, Methyl, Ethyl, Propyl, Butyl, Allyl, Propargyl, Cyanoethyl, Methoxyethyl, Methoxy oder Benzyl steht, oder zusammen mit R⁴ für Butan-1,4-diyl, Pentan-1,5-diyl oder 3-Oxa-pentan-1,5-diyI steht,
X für Sauerstoff oder Schwefel steht und
Y für Sauerstoff oder Schwefel steht.

Insbesondere sei in allen Definition die folgenden Bedeutungen hervorgehoben, wobei
R² für Wasserstoff steht,
R³ für Wasserstoff oder Methyl steht,
R⁴ für Wasserstoff, Methyl oder Ethyl steht,
R⁵ für Wasserstoff, Methyl, Ethyl, n- oder iso-Propyl steht, oder R⁴ und R⁵ zusammen für Butan-1,4-diyl steht,
X für Sauersstoff oder Schwefel steht oder
Y für Sauerstoff steht.

Beispiele für die Verbindungen der Formel (I) sind in der nachstehenden Tabelle 1 aufgeführt.

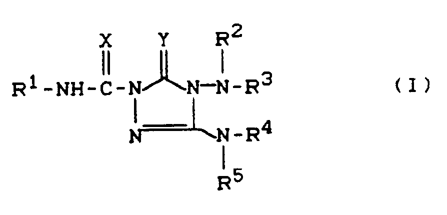

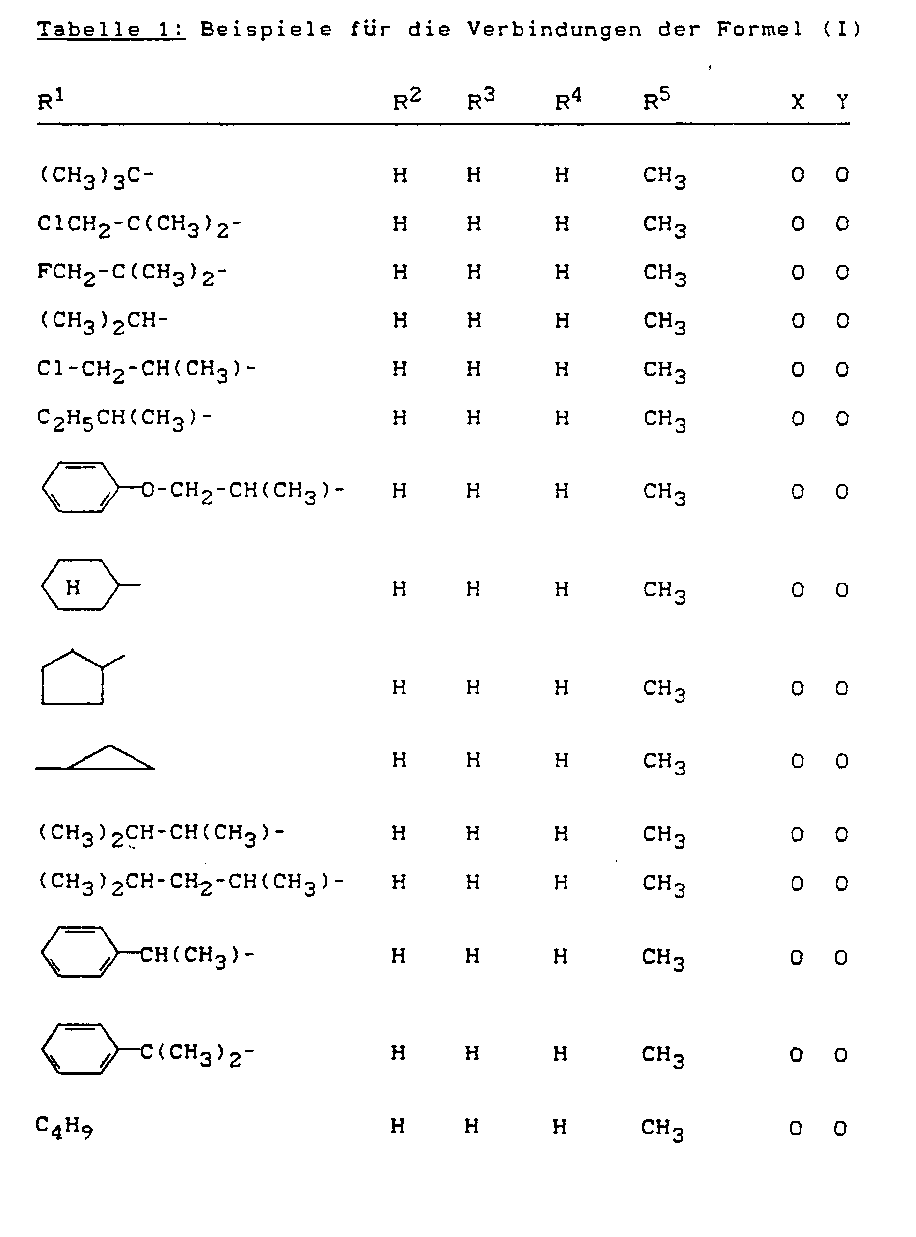

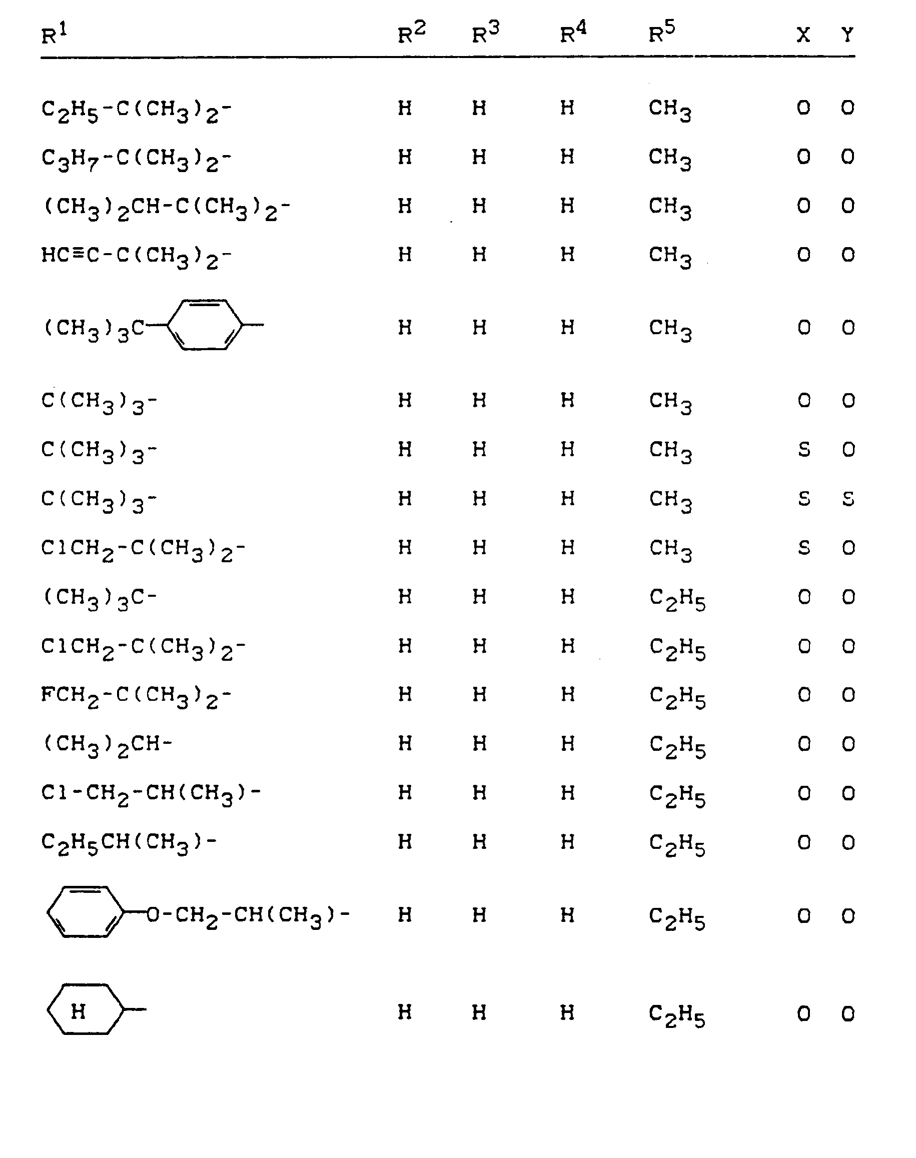

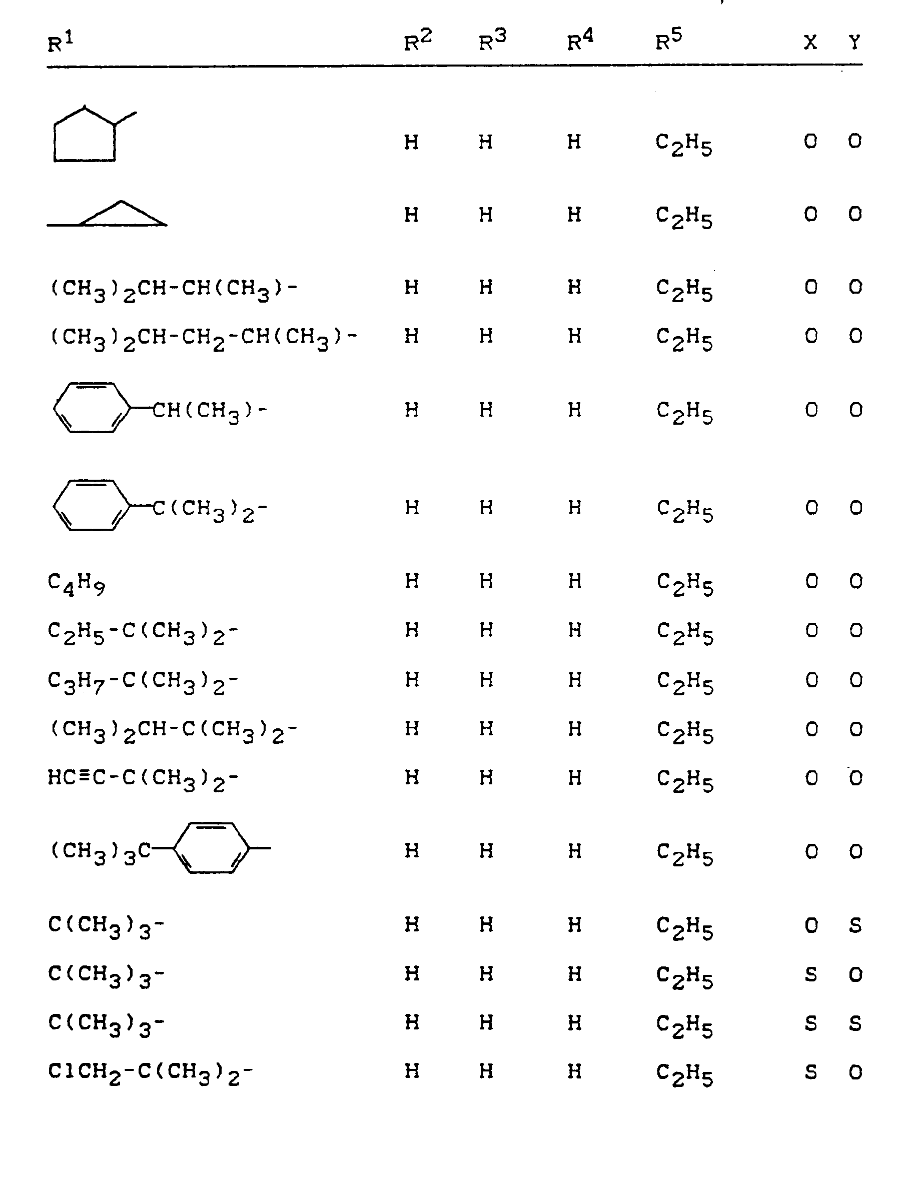

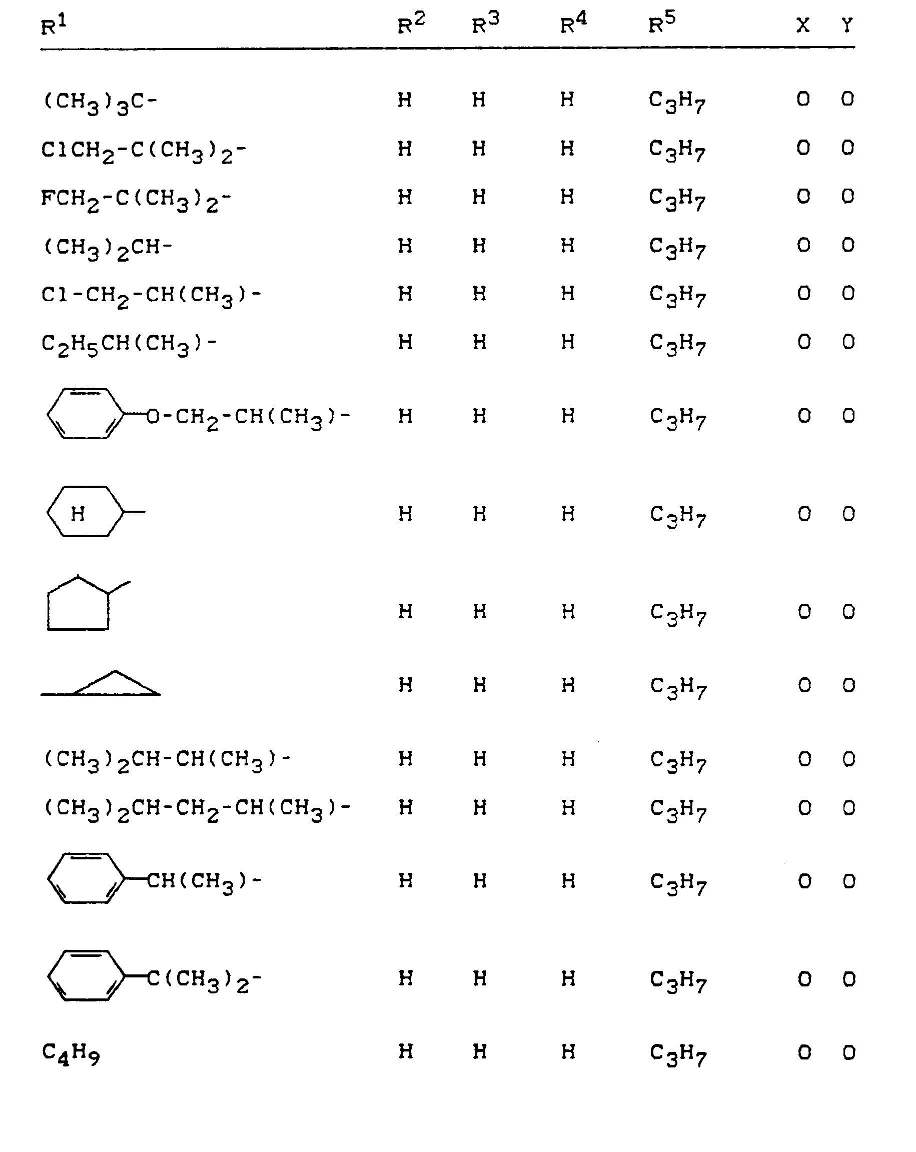

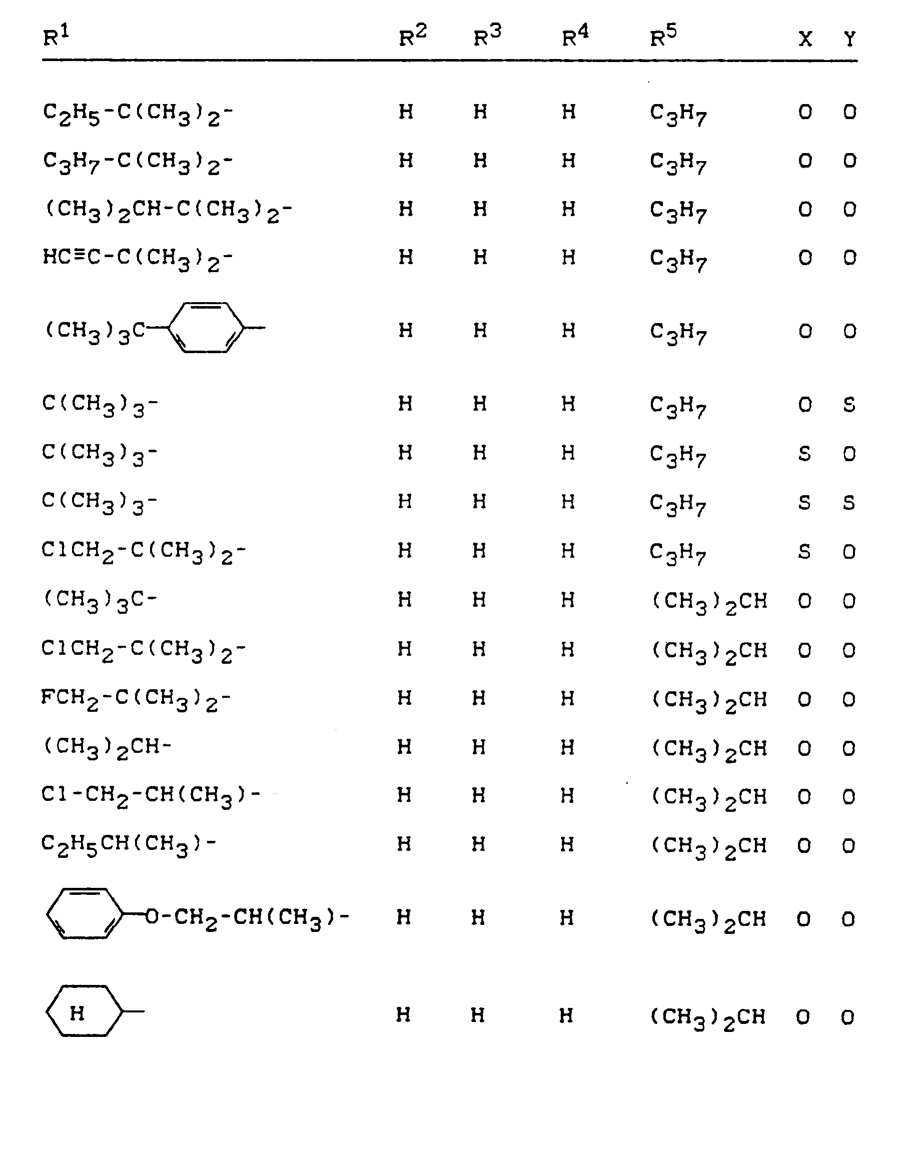

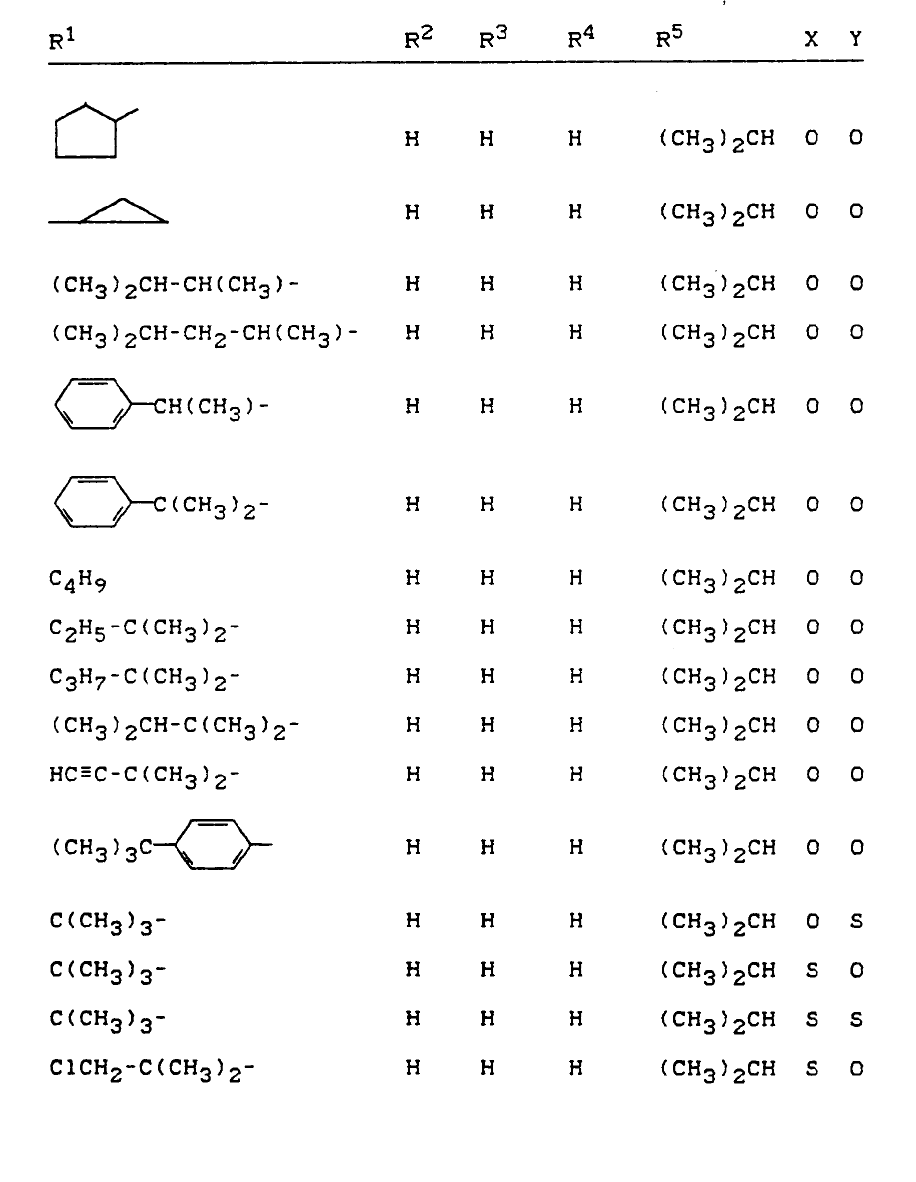

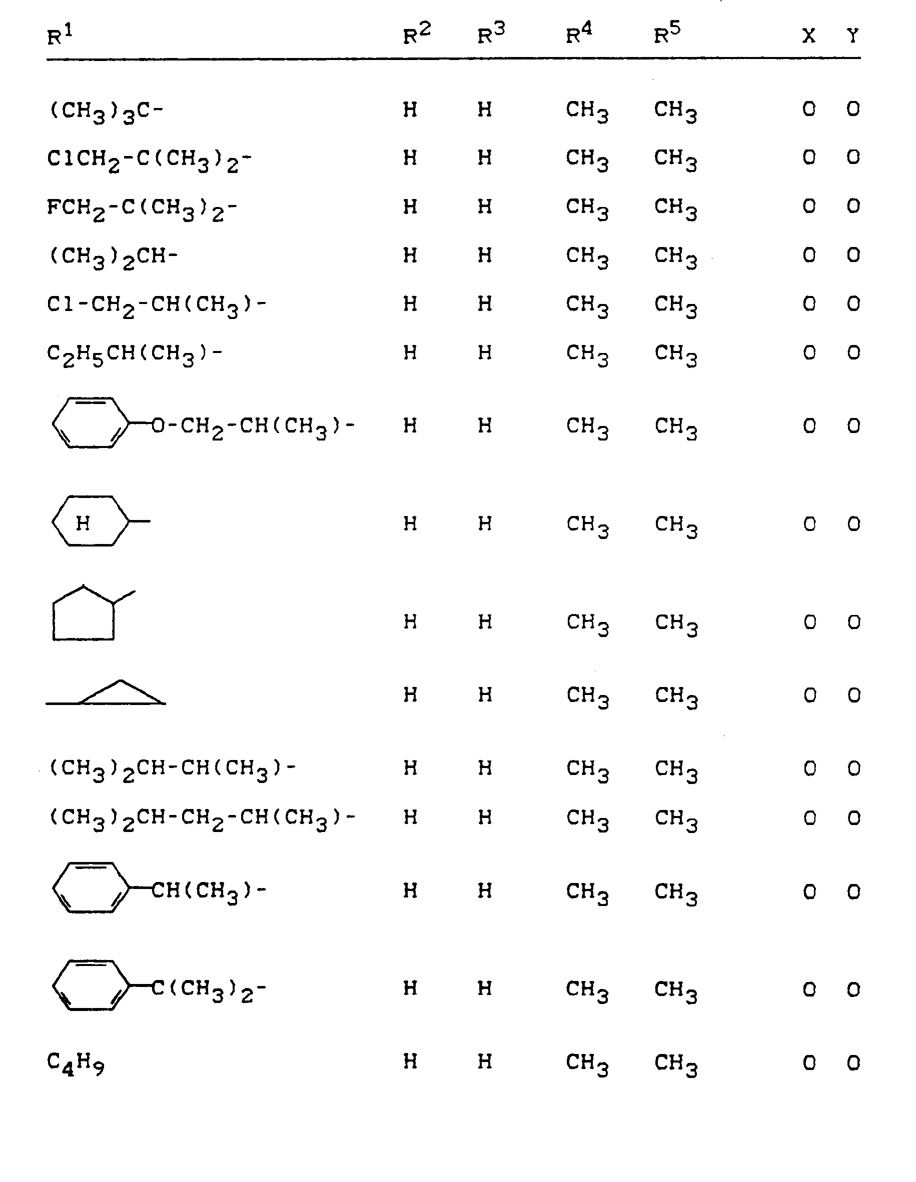

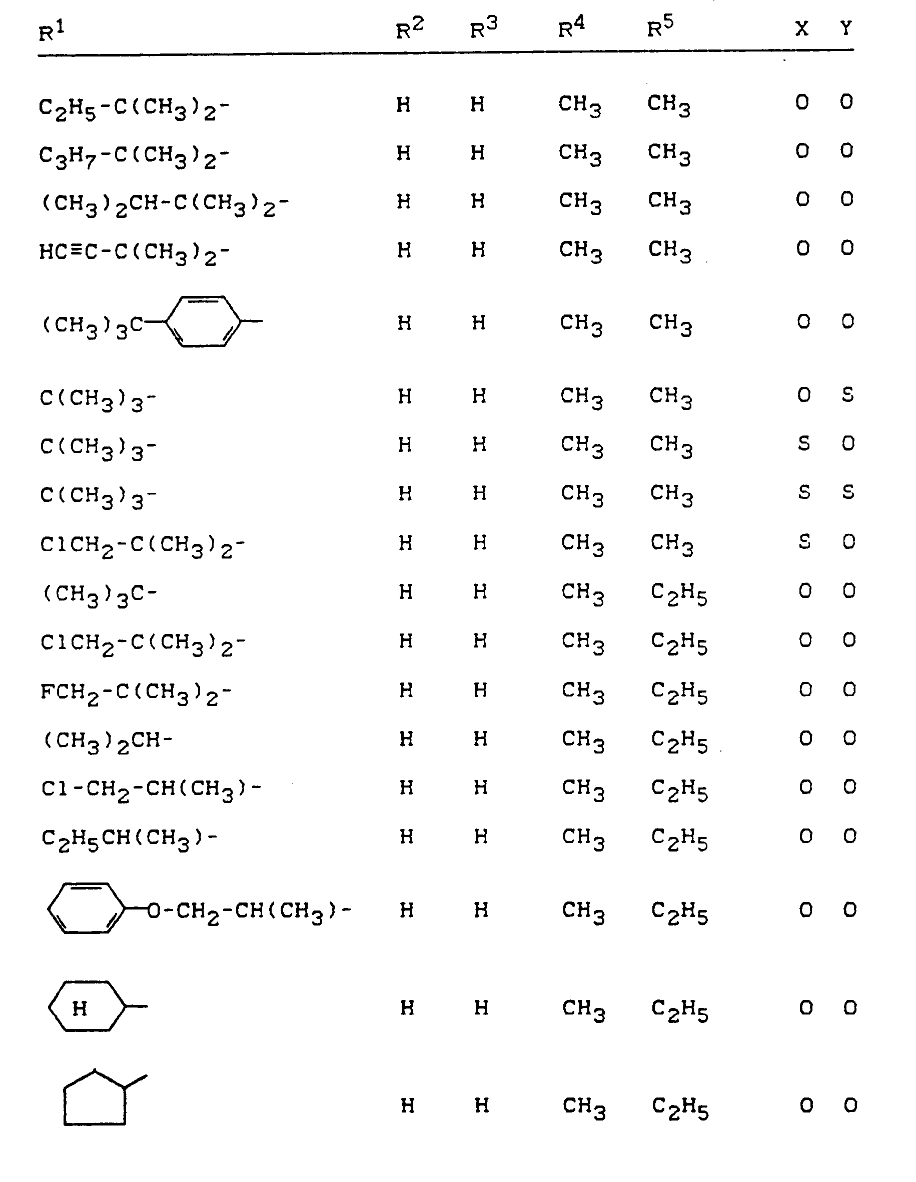

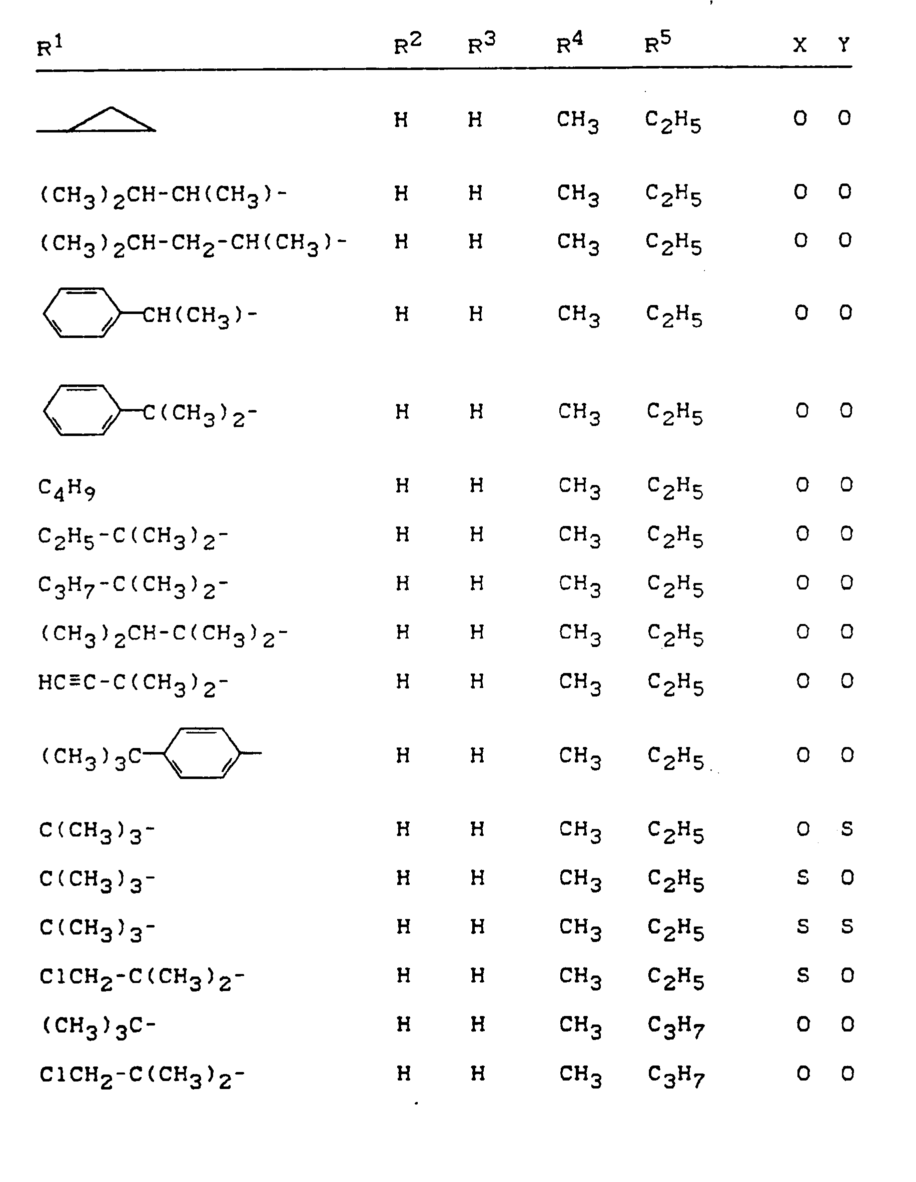

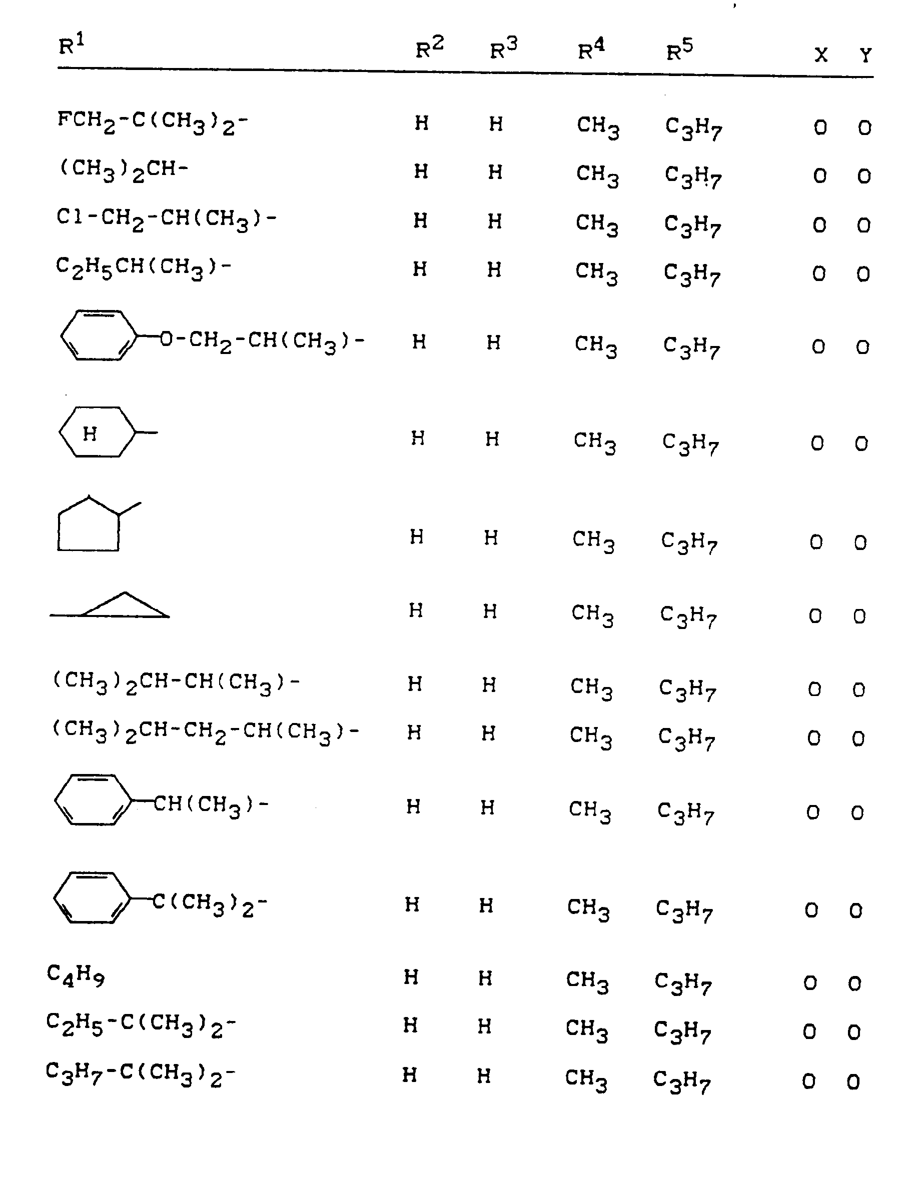

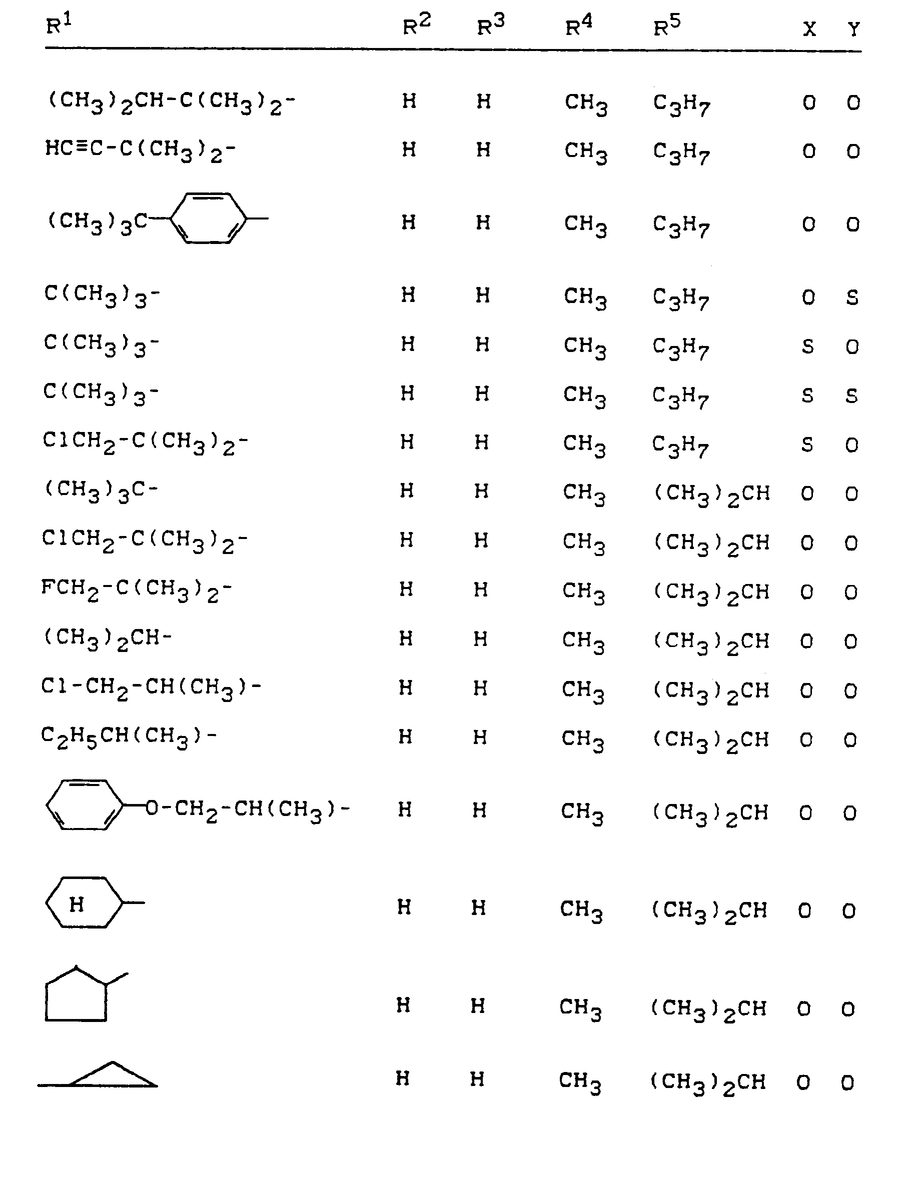

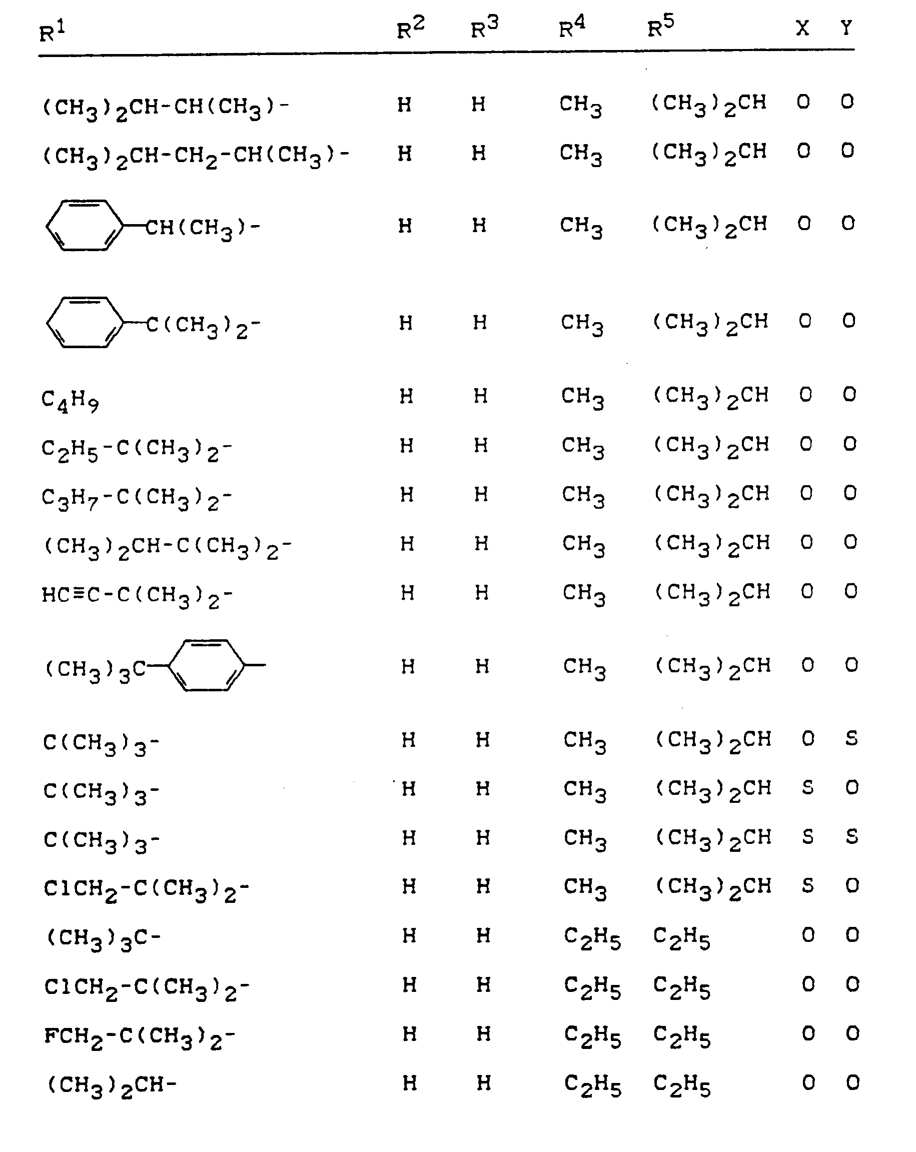

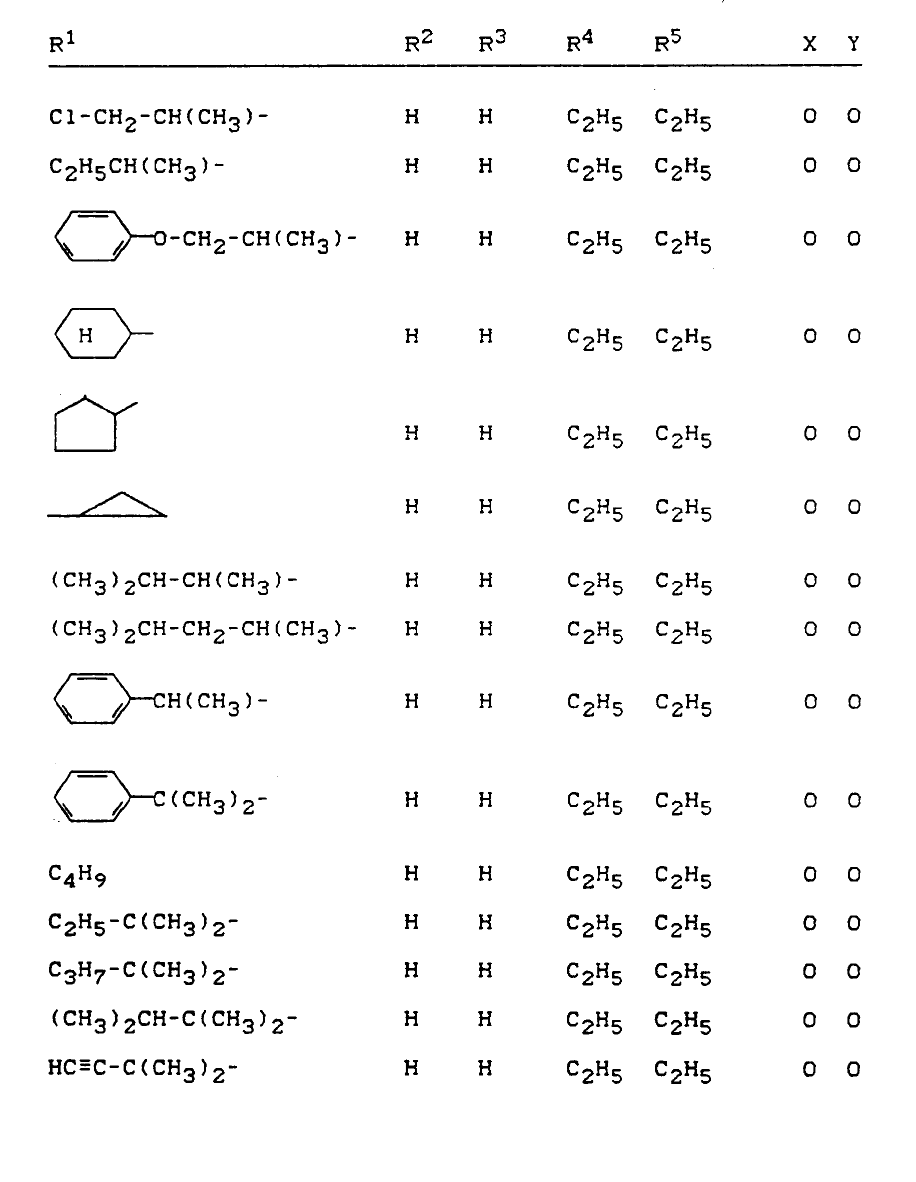

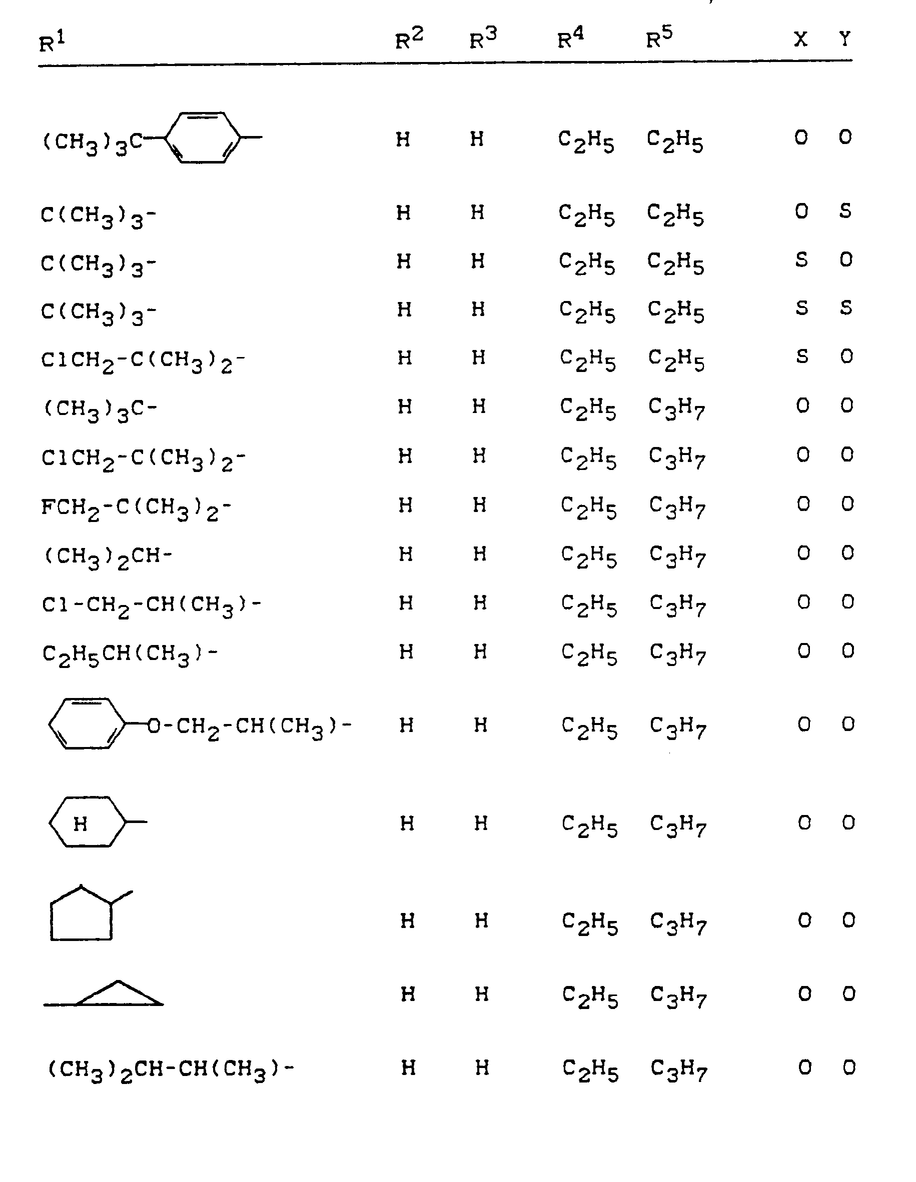

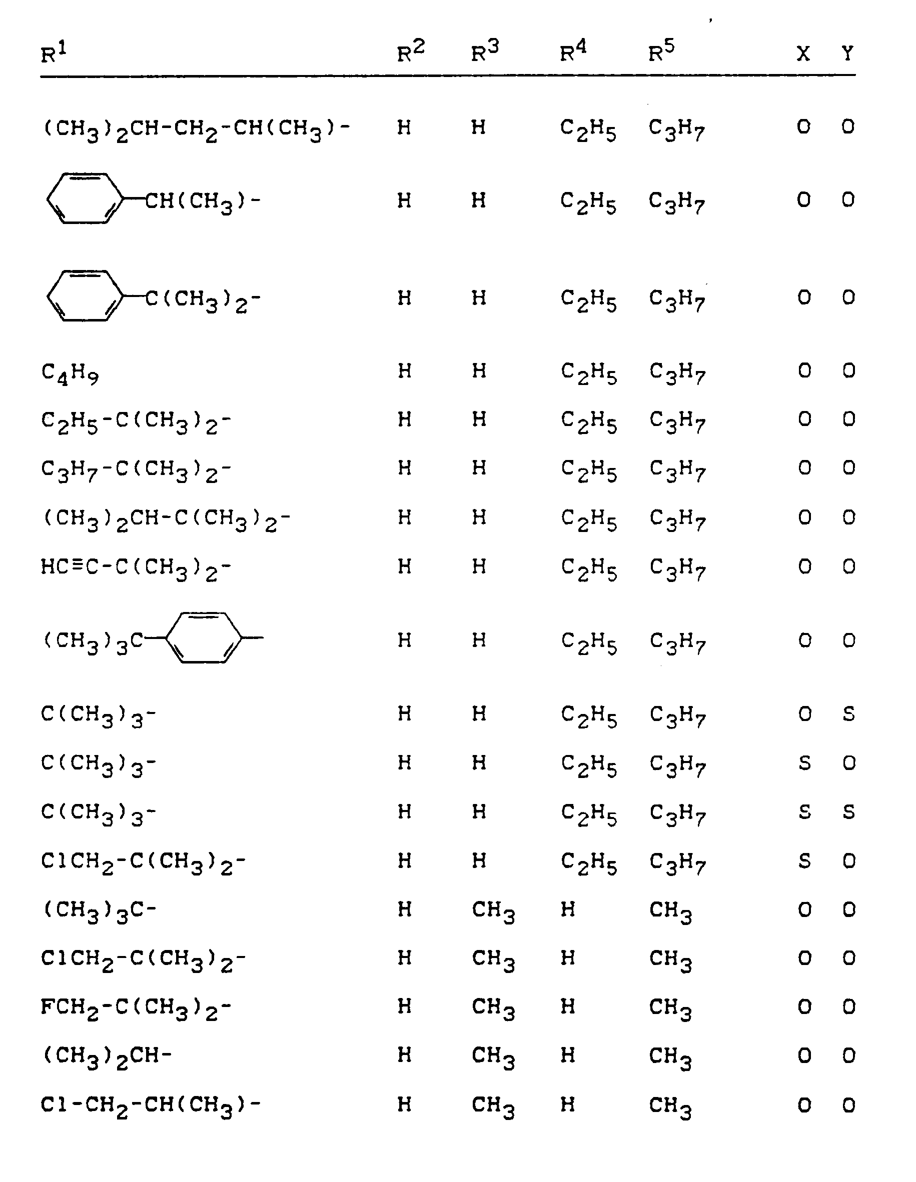

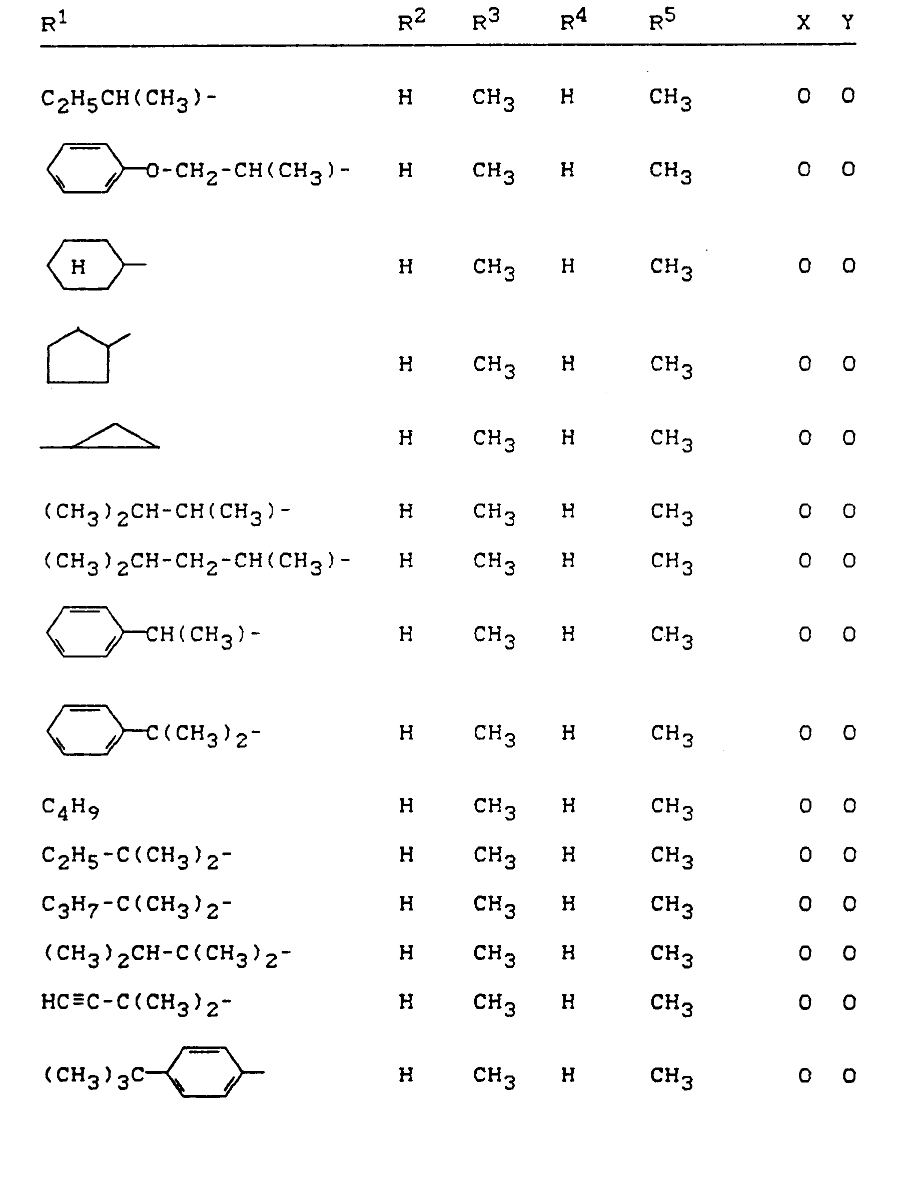

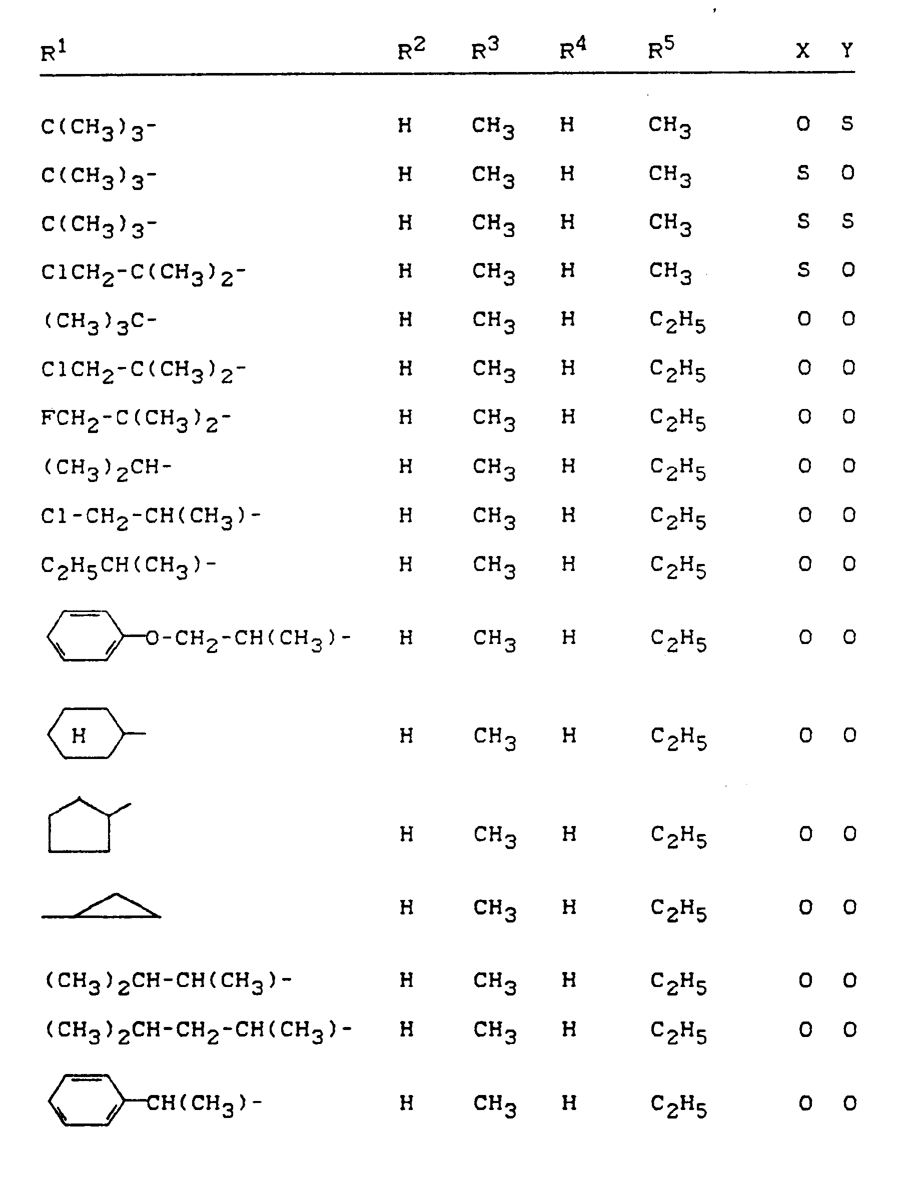

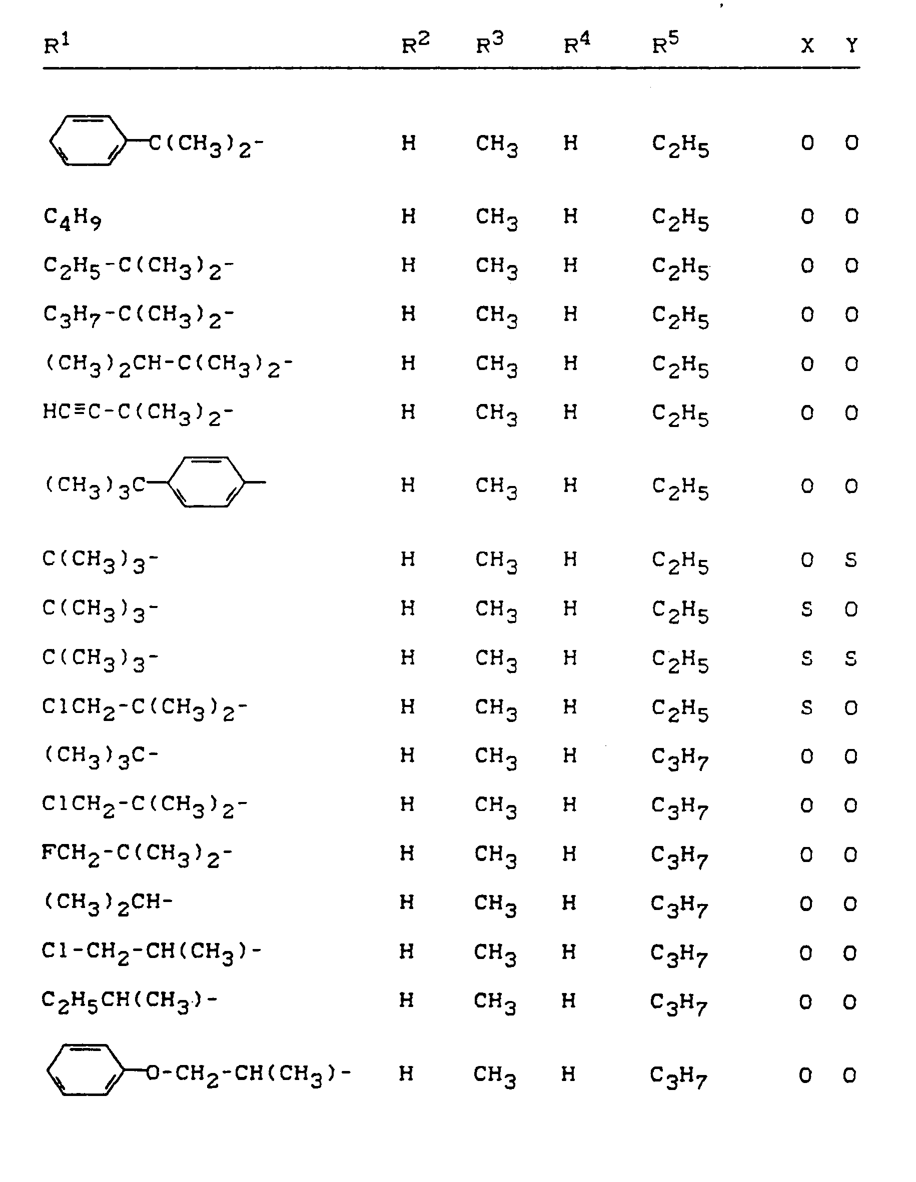

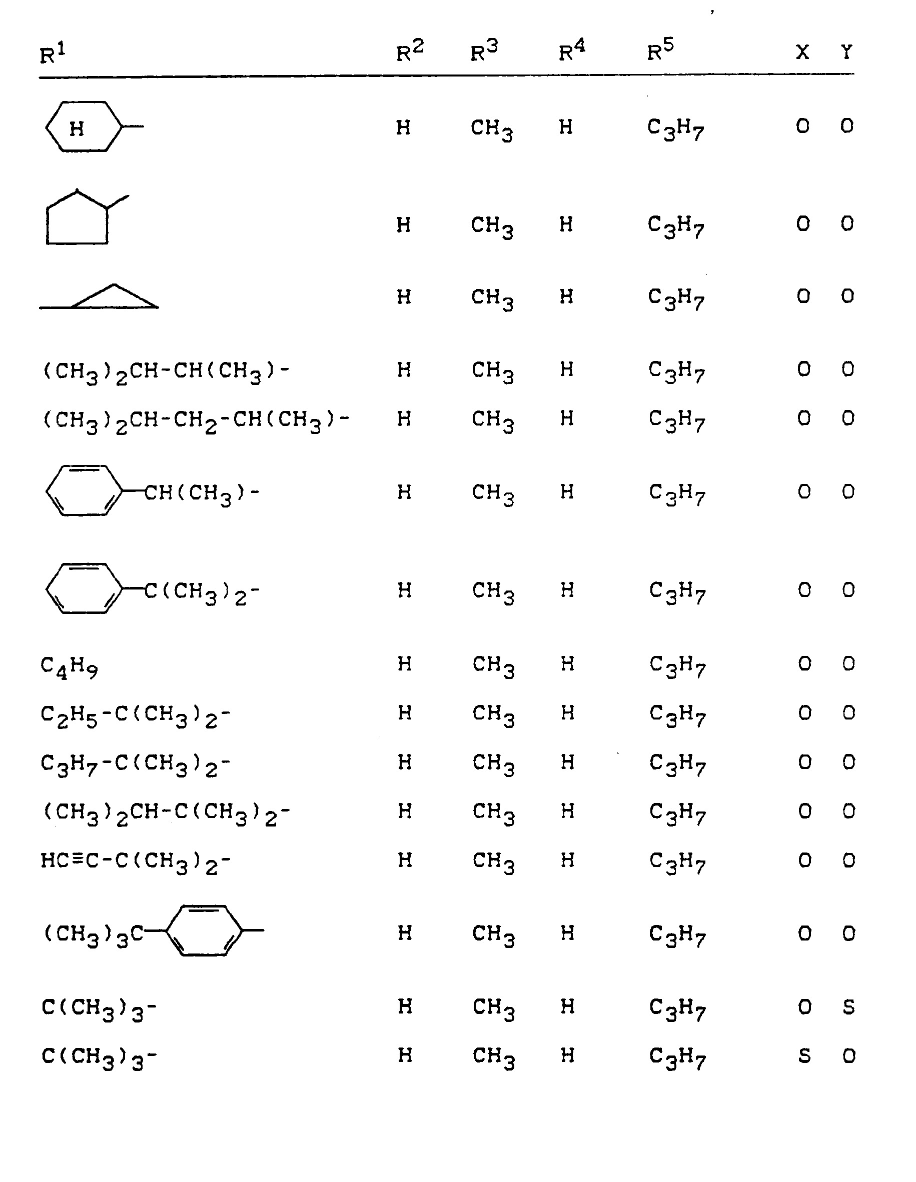

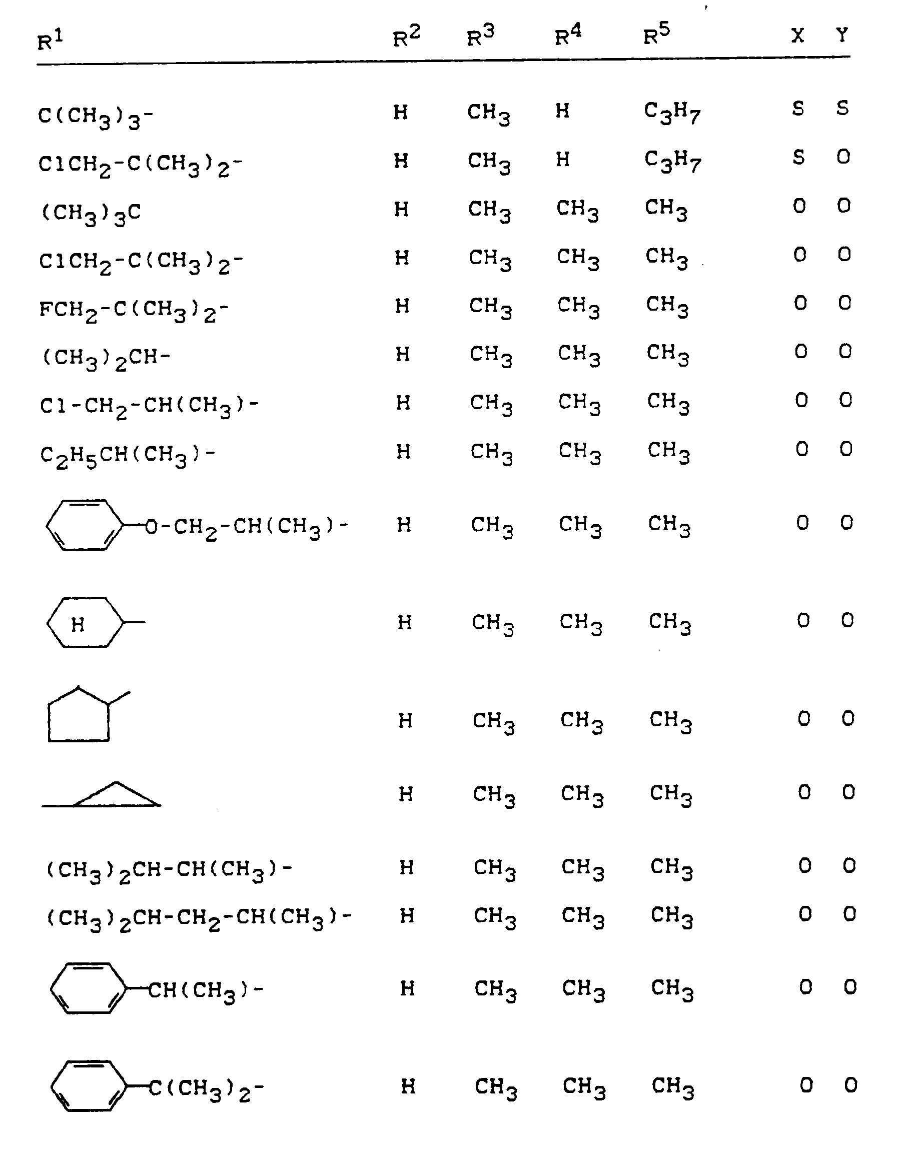

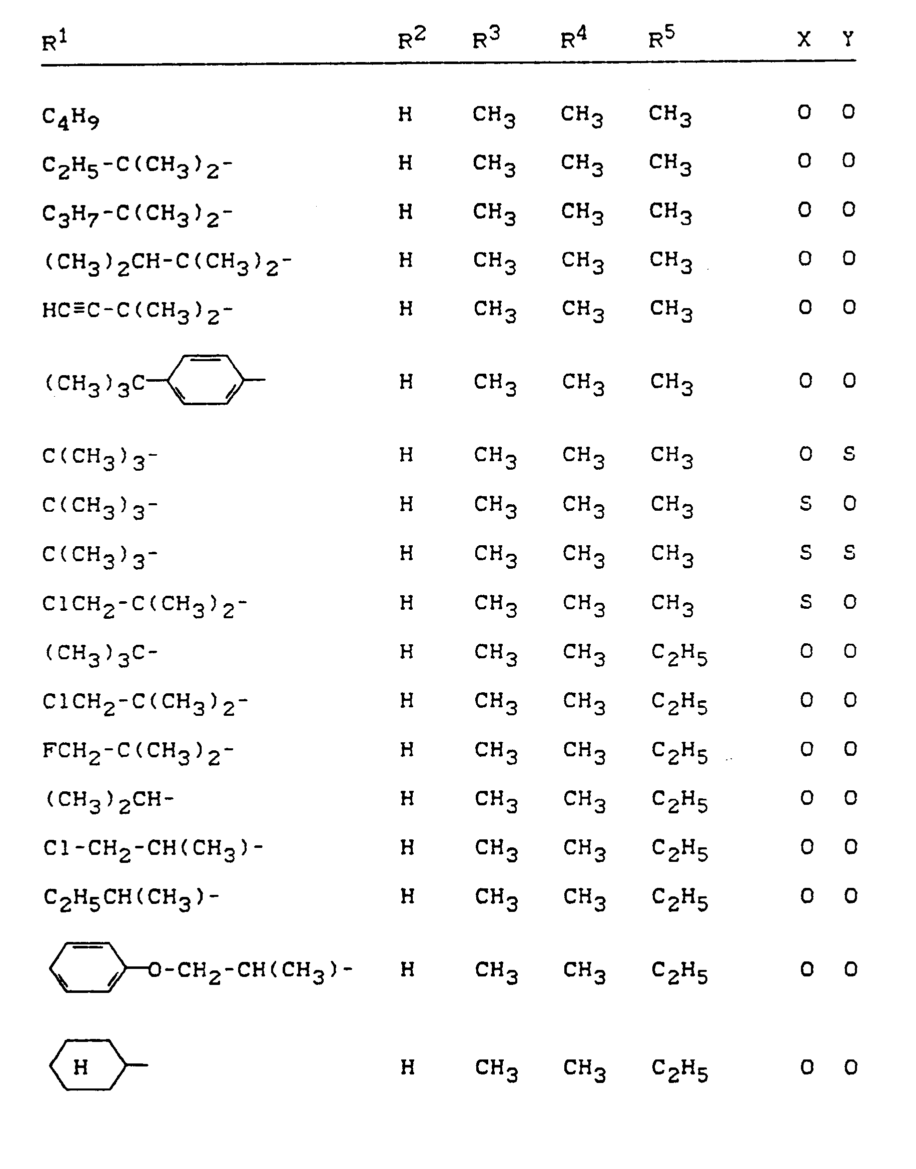

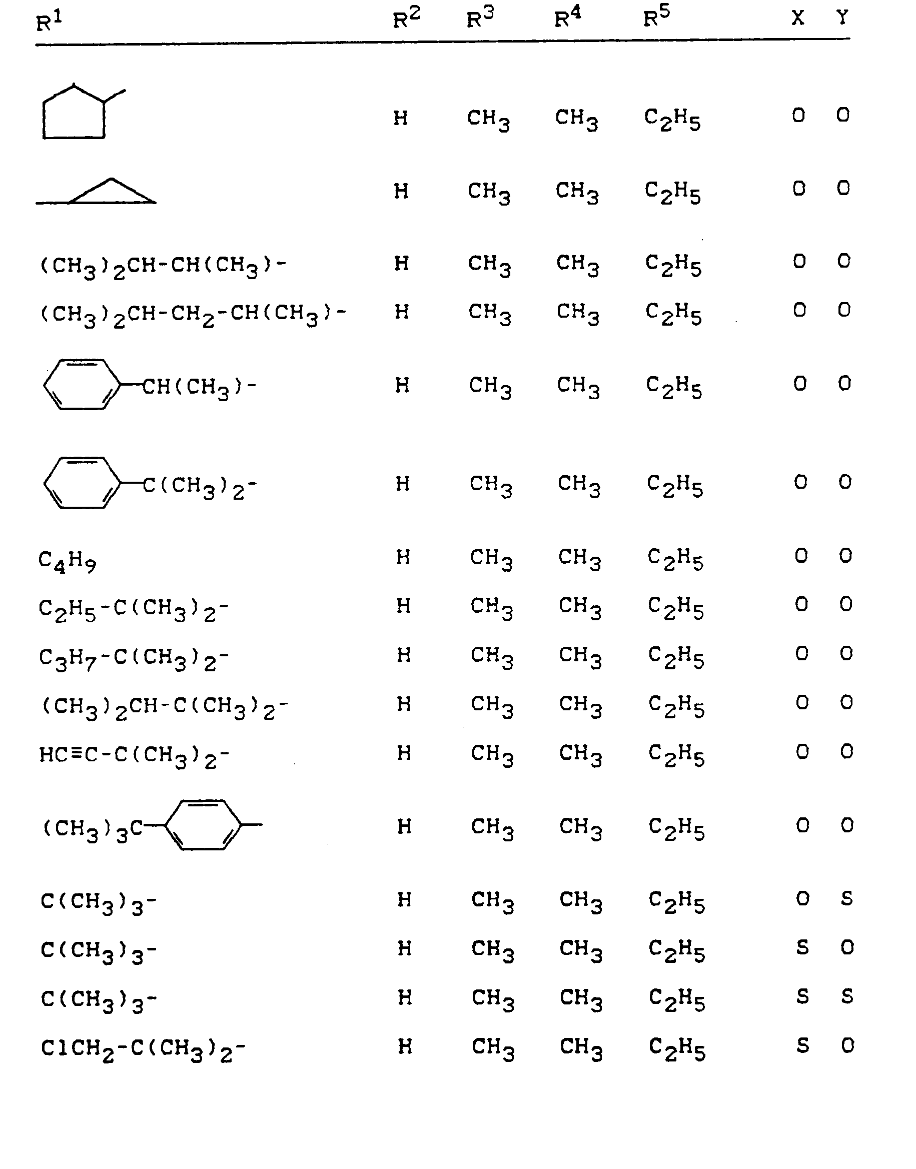

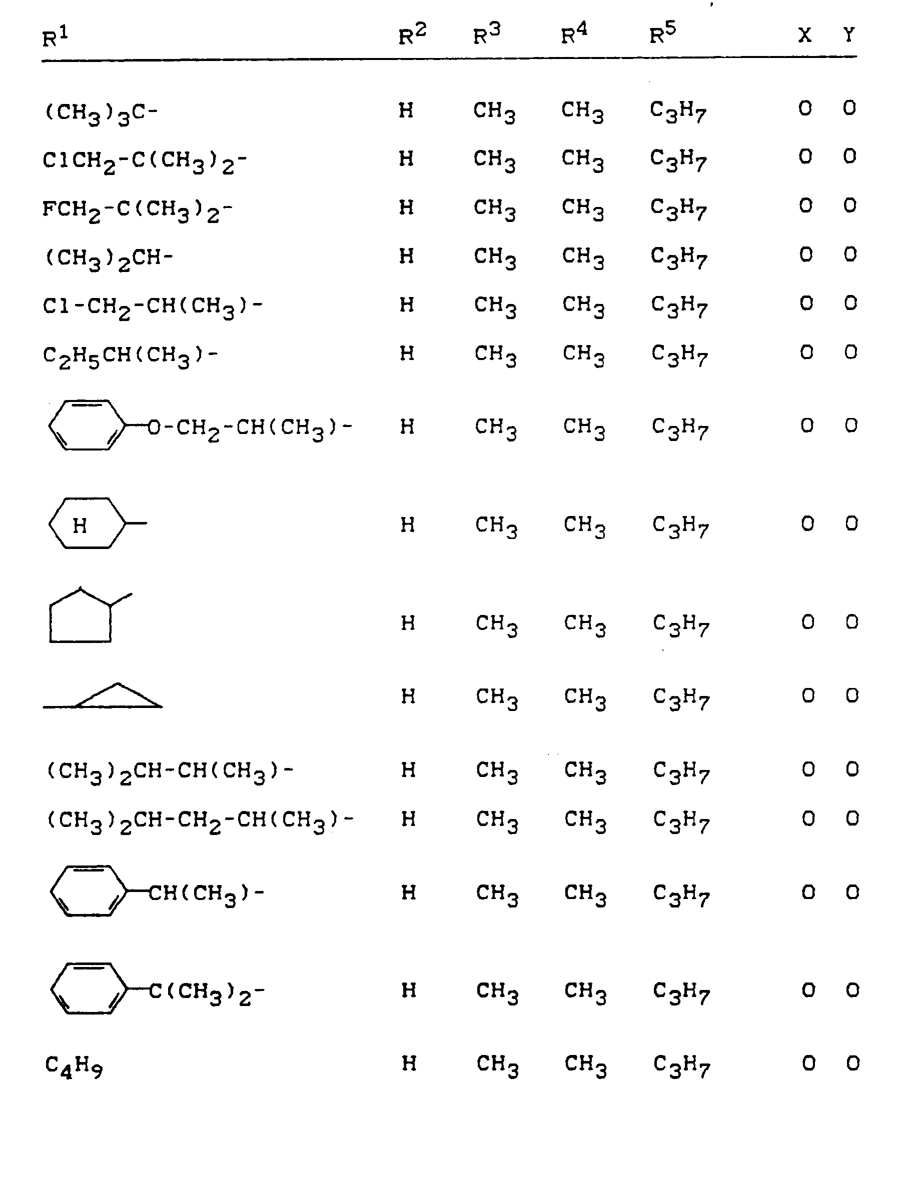

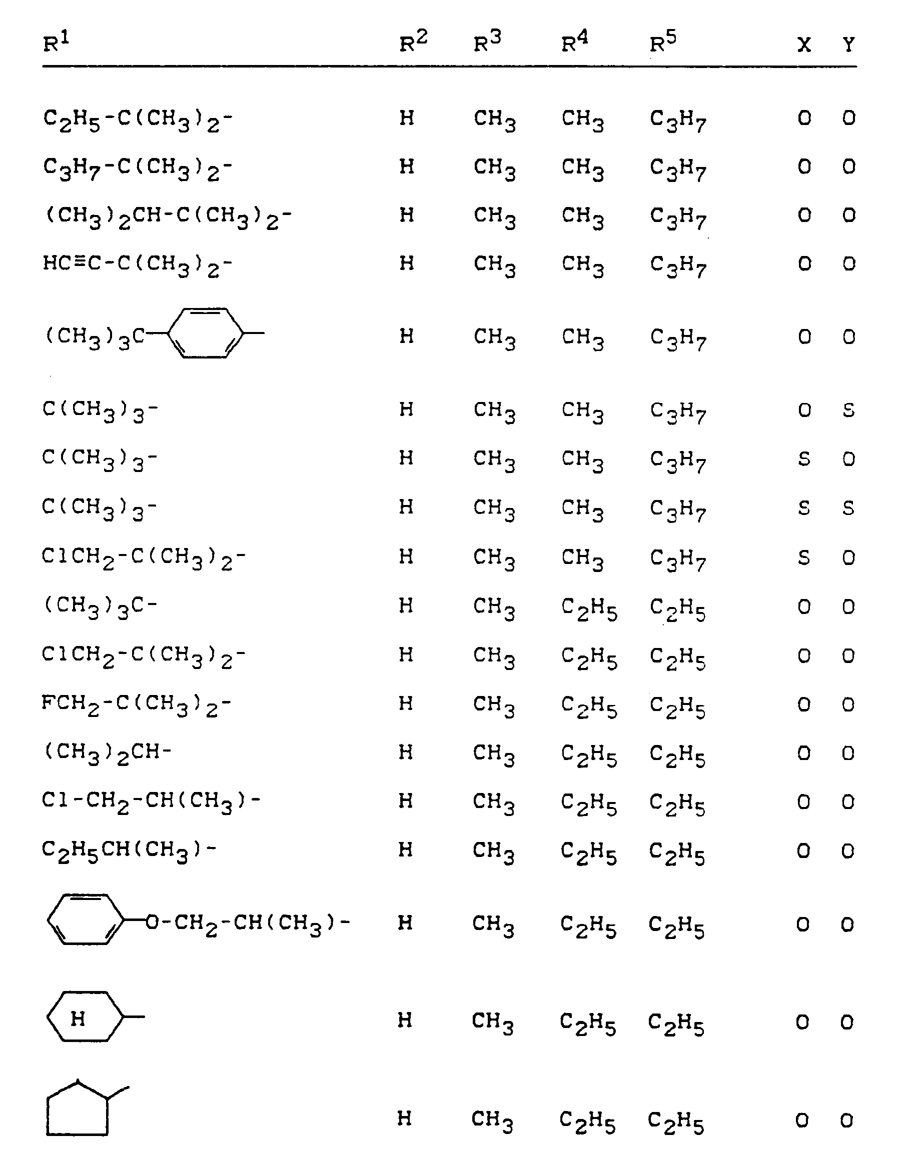

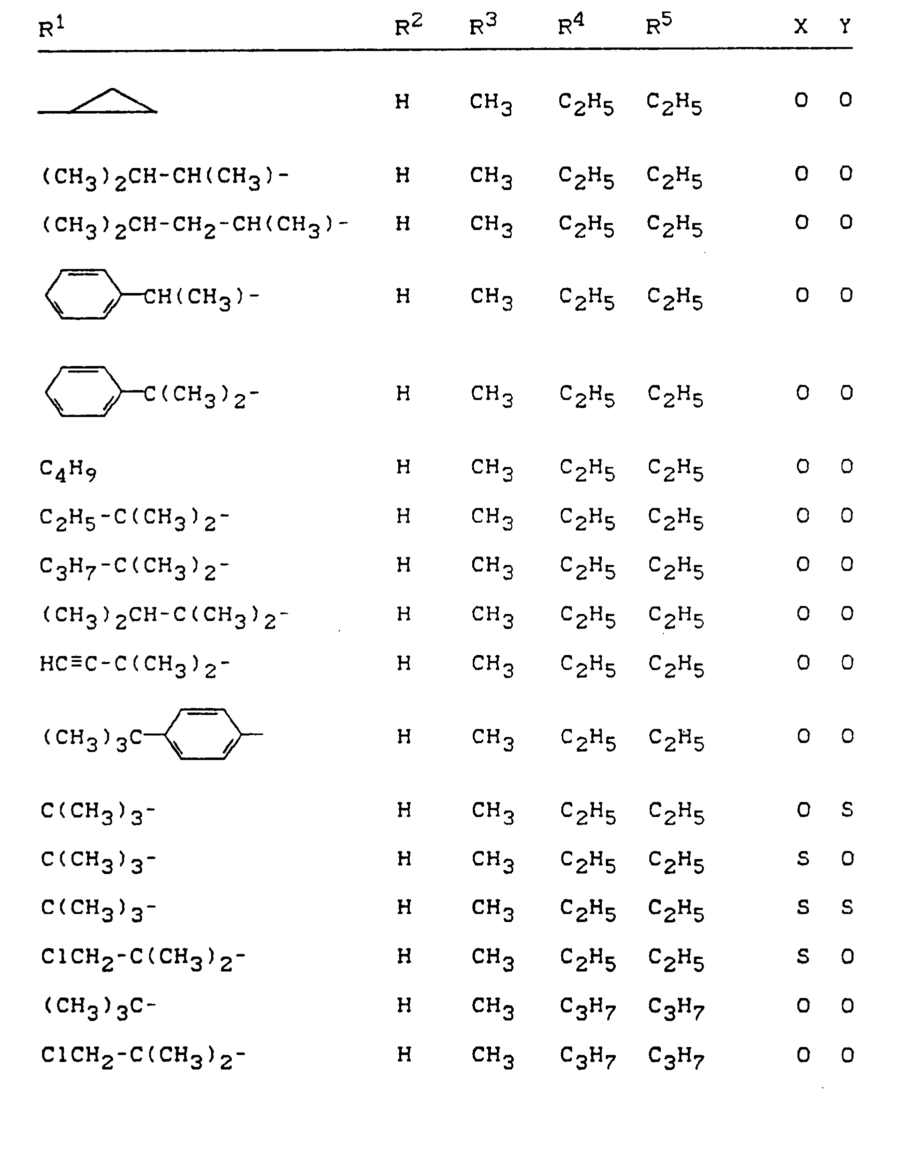

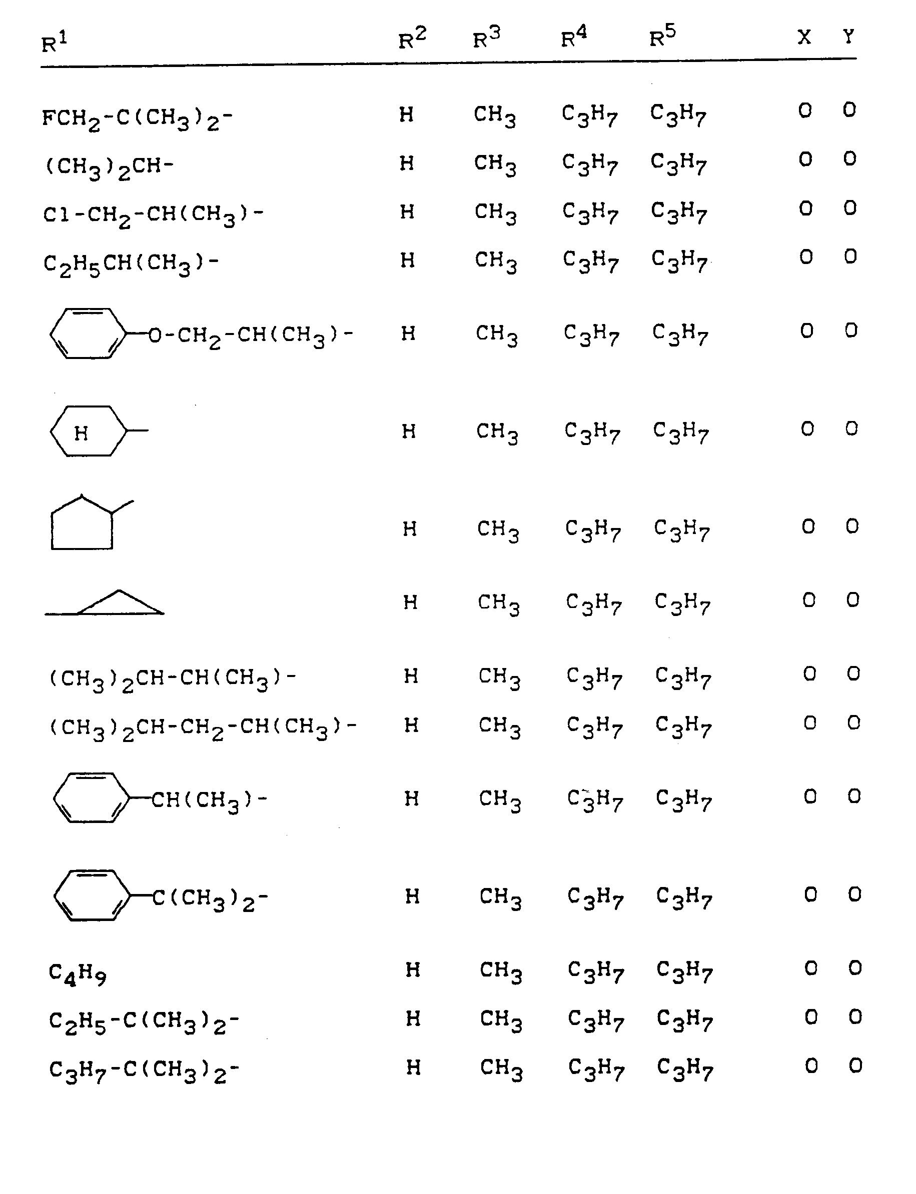

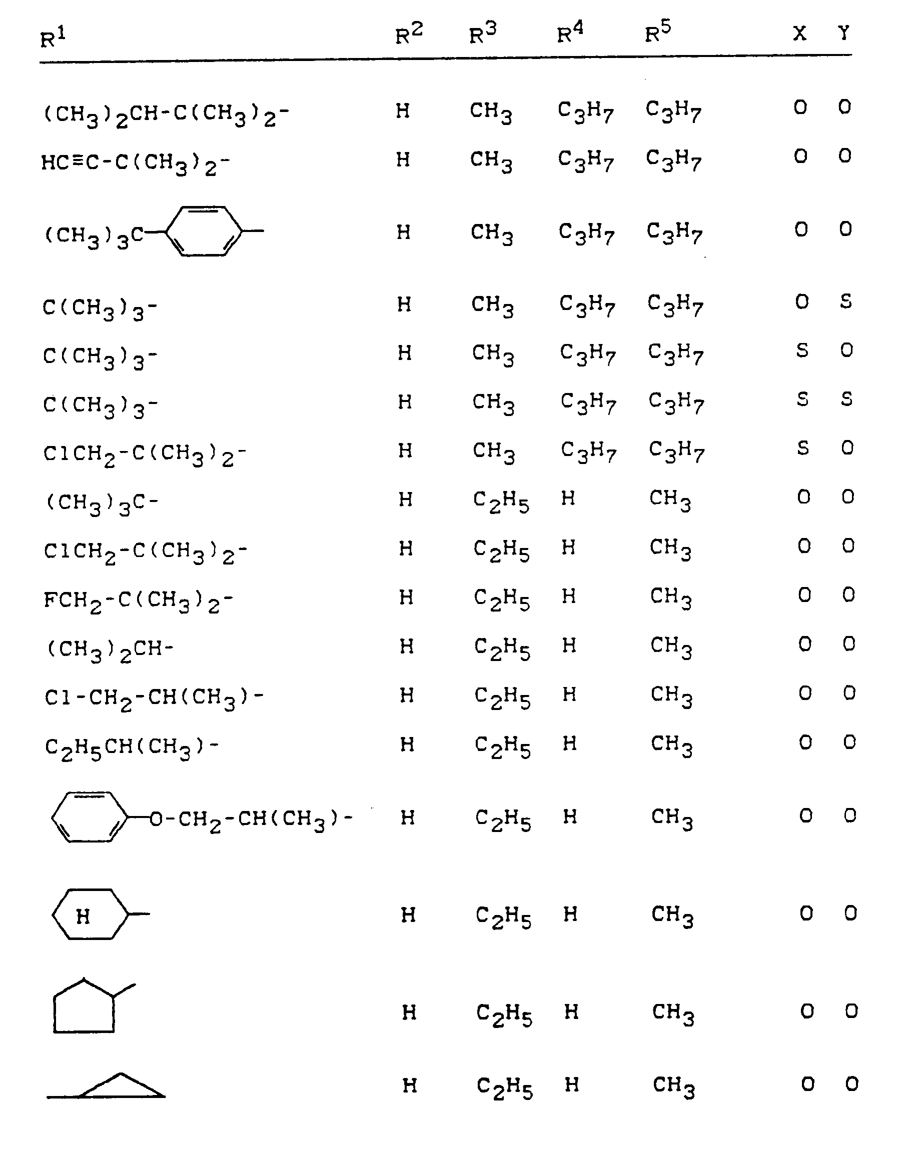

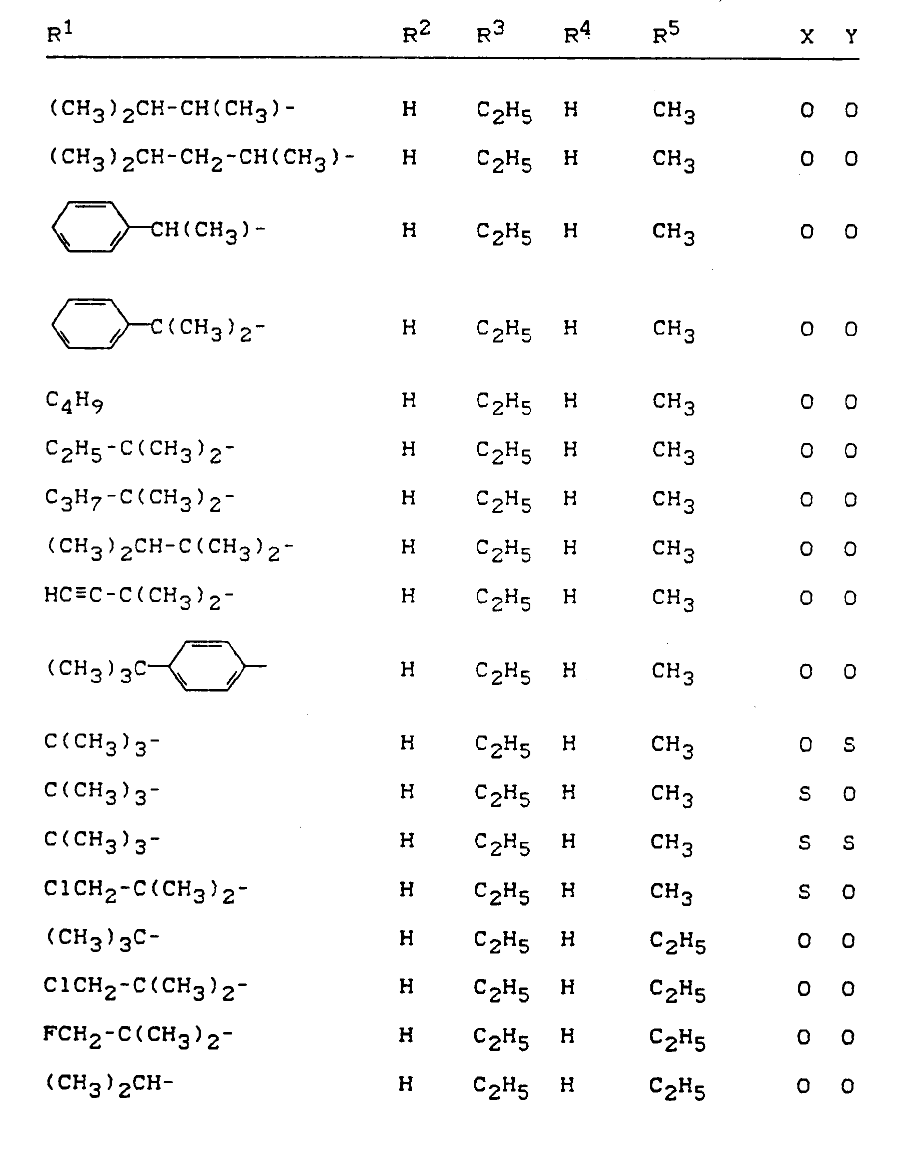

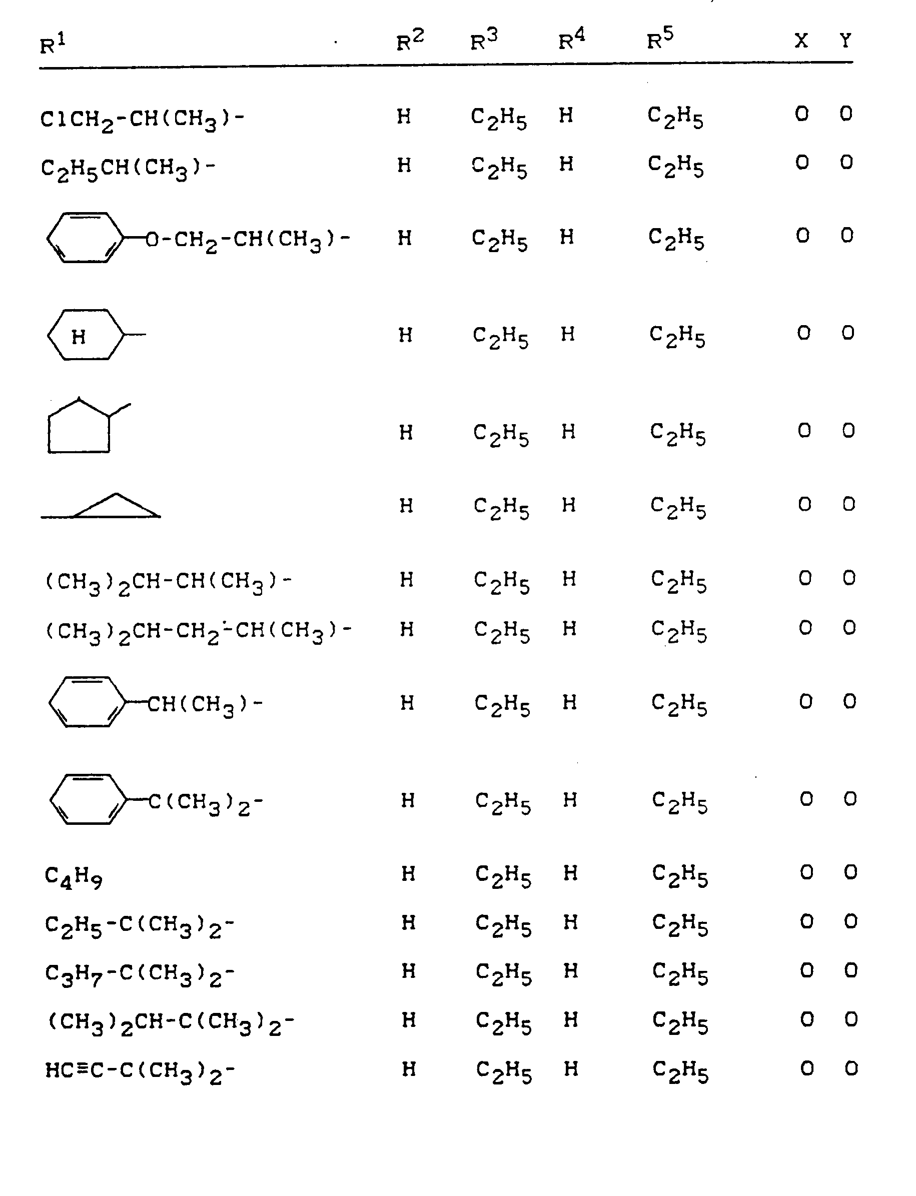

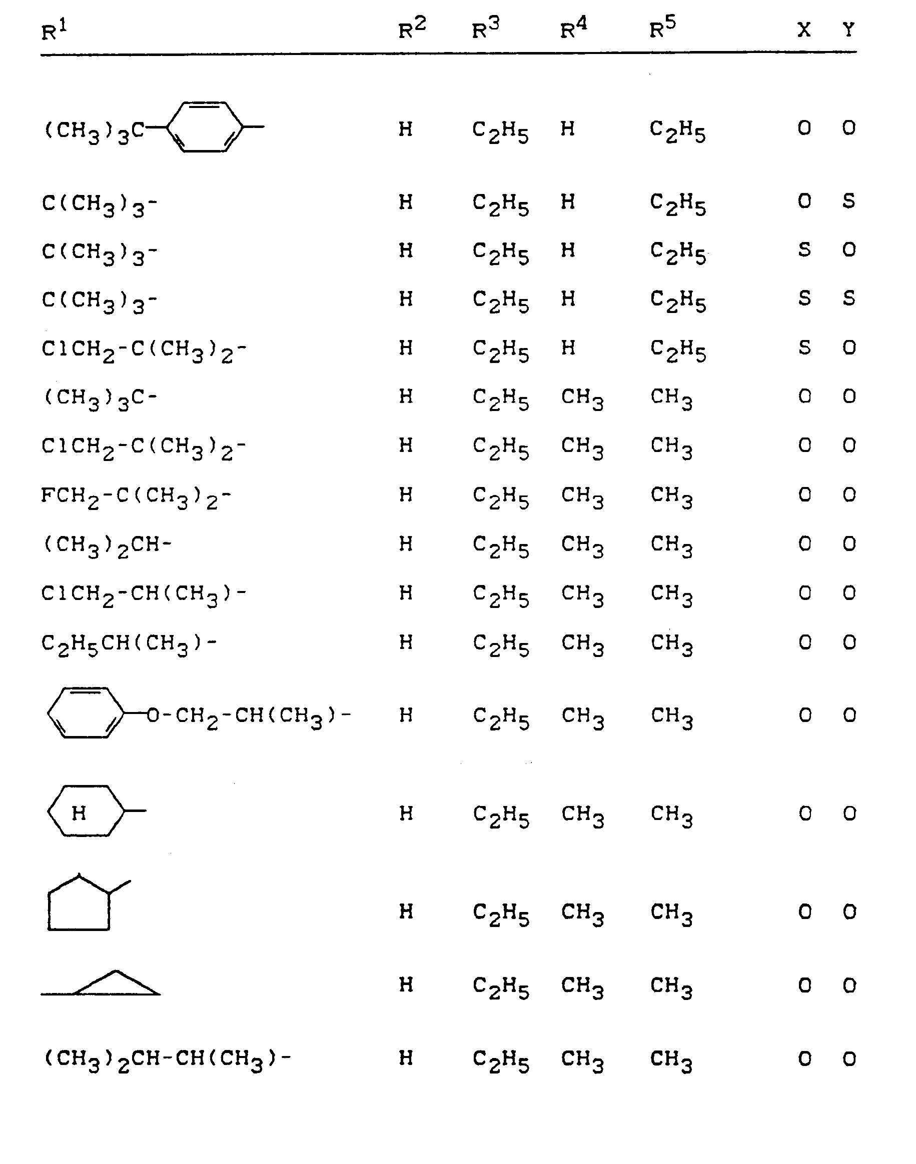

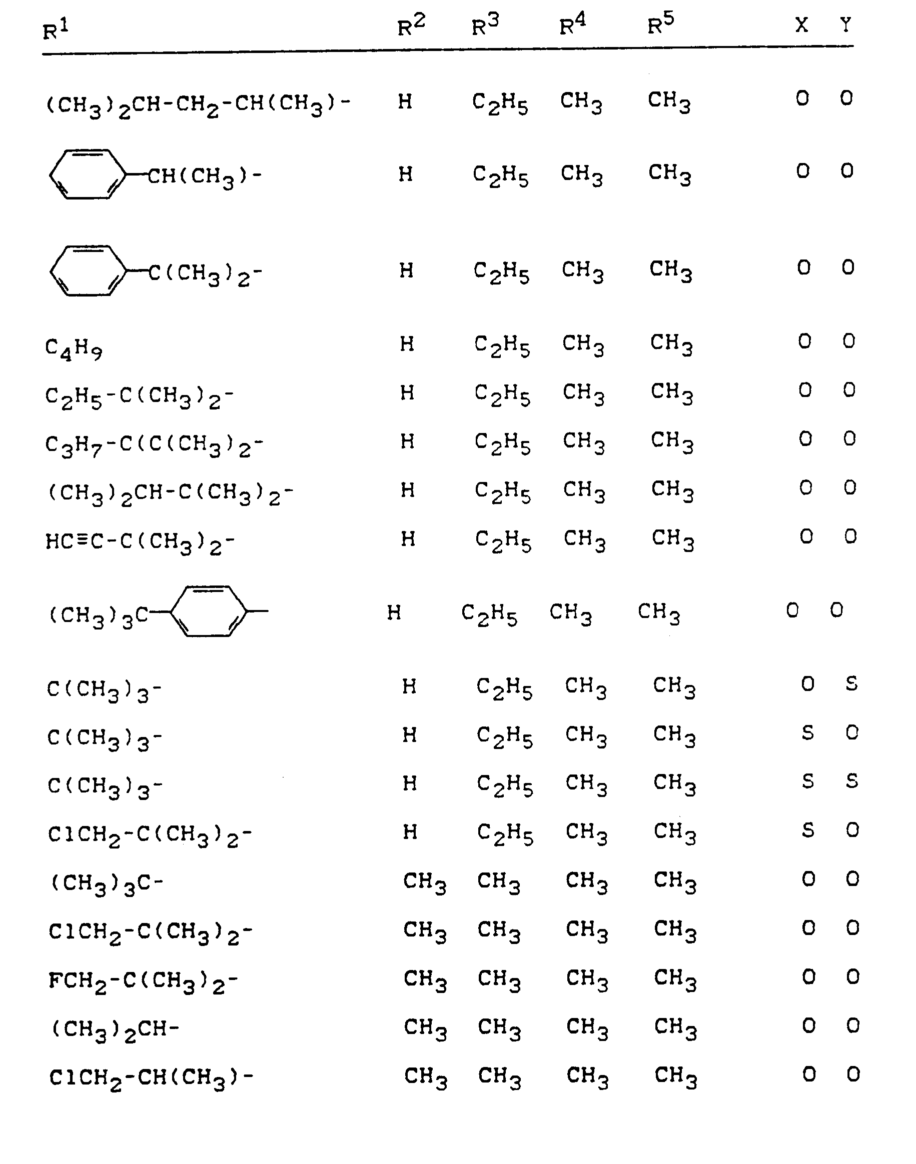

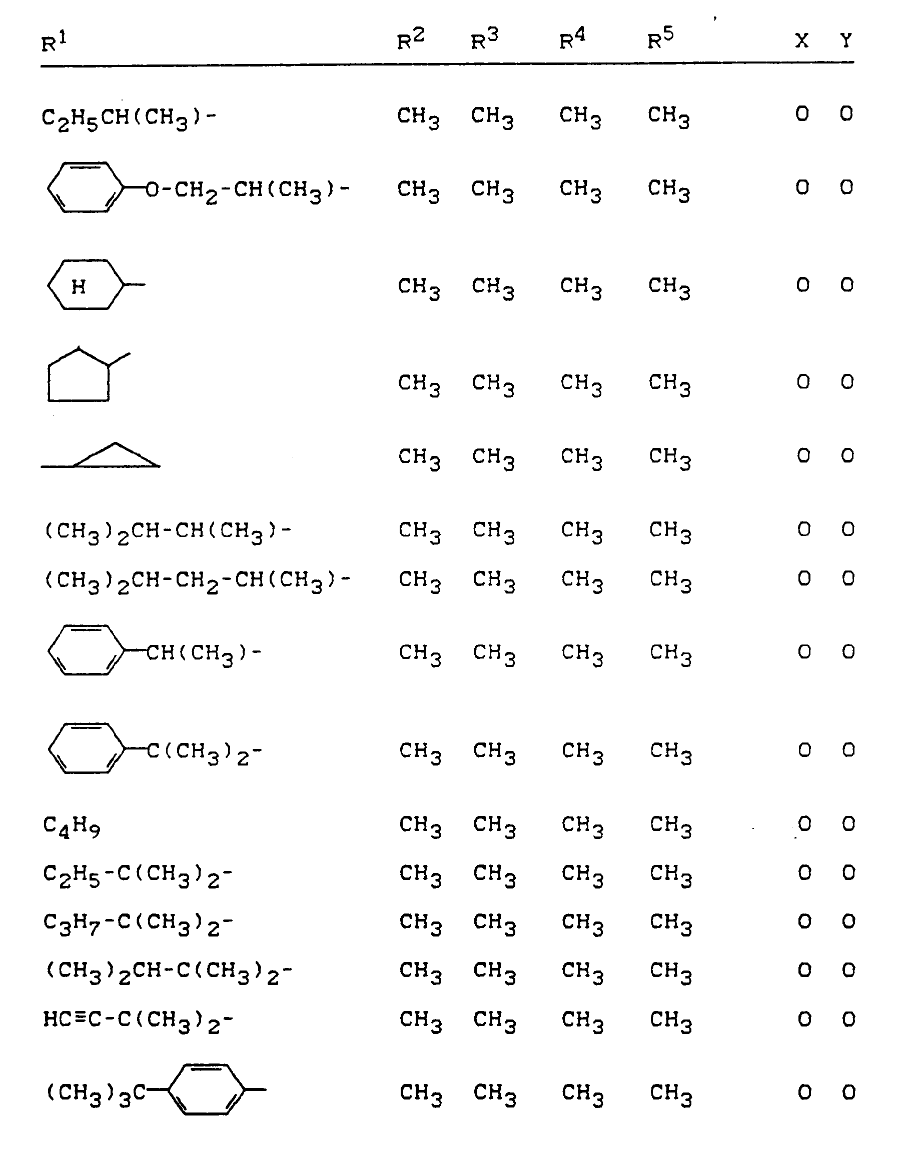

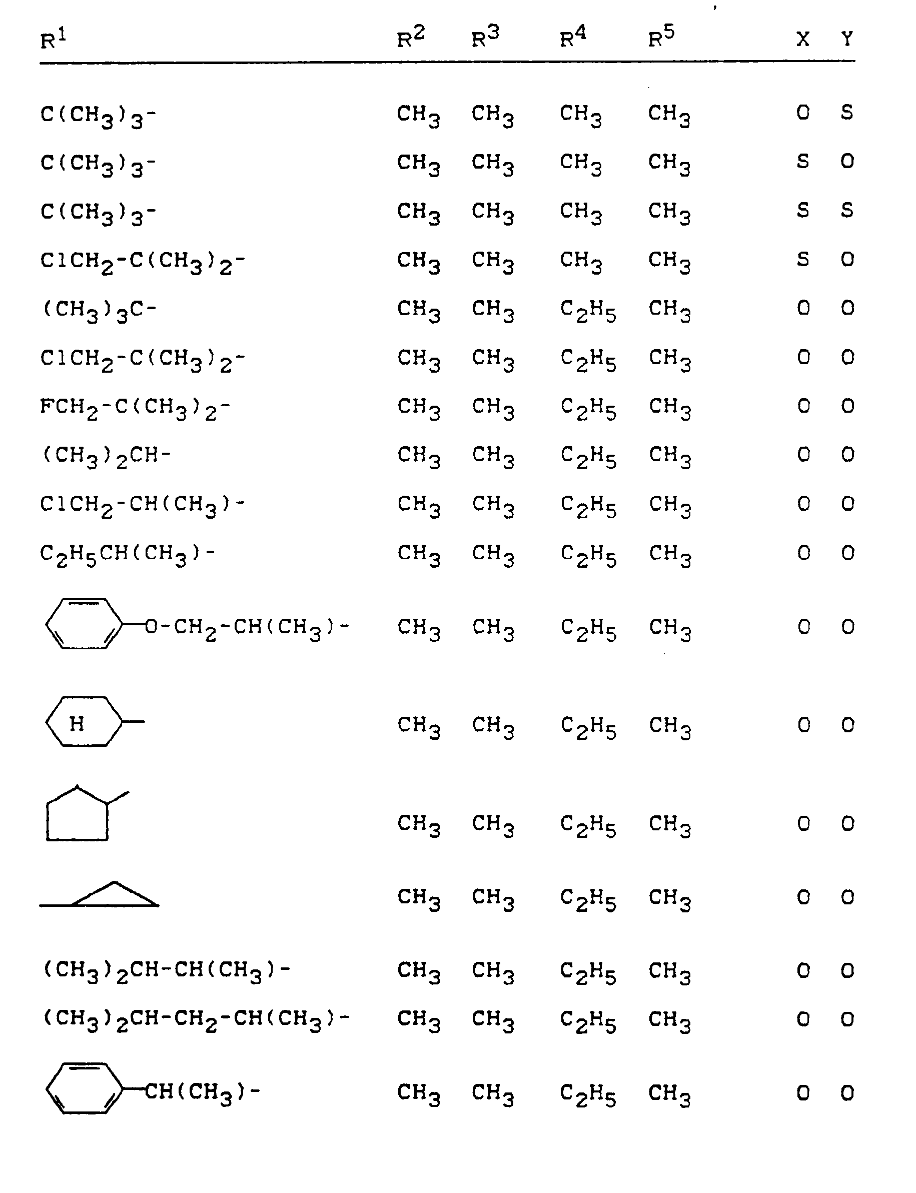

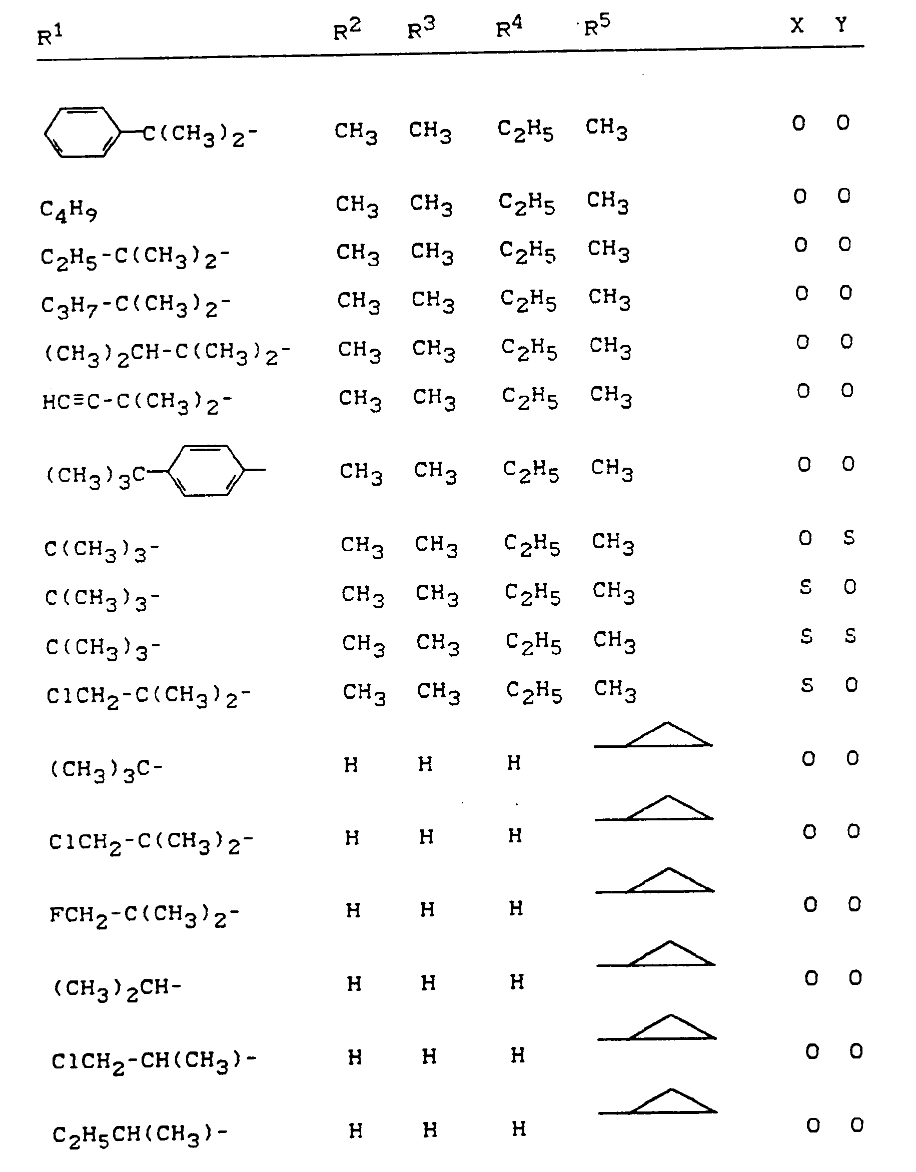

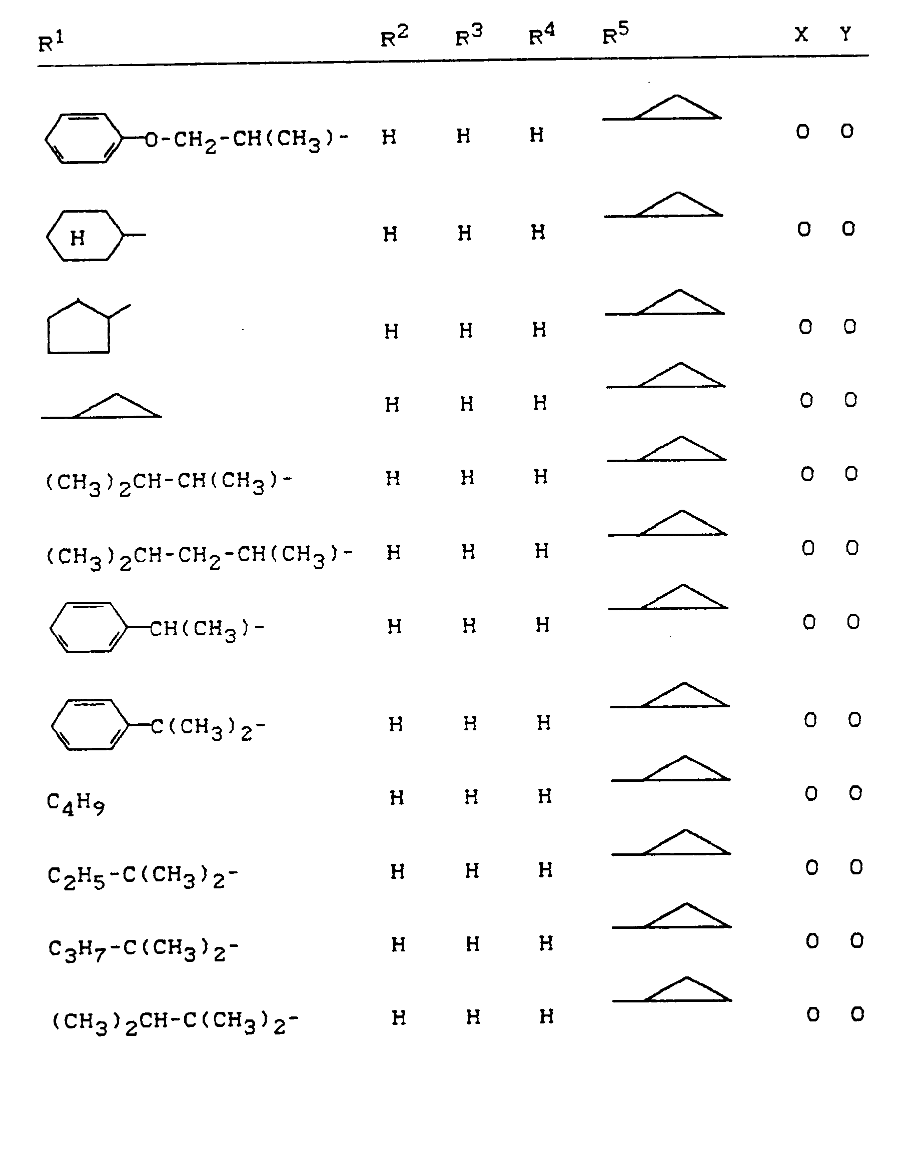

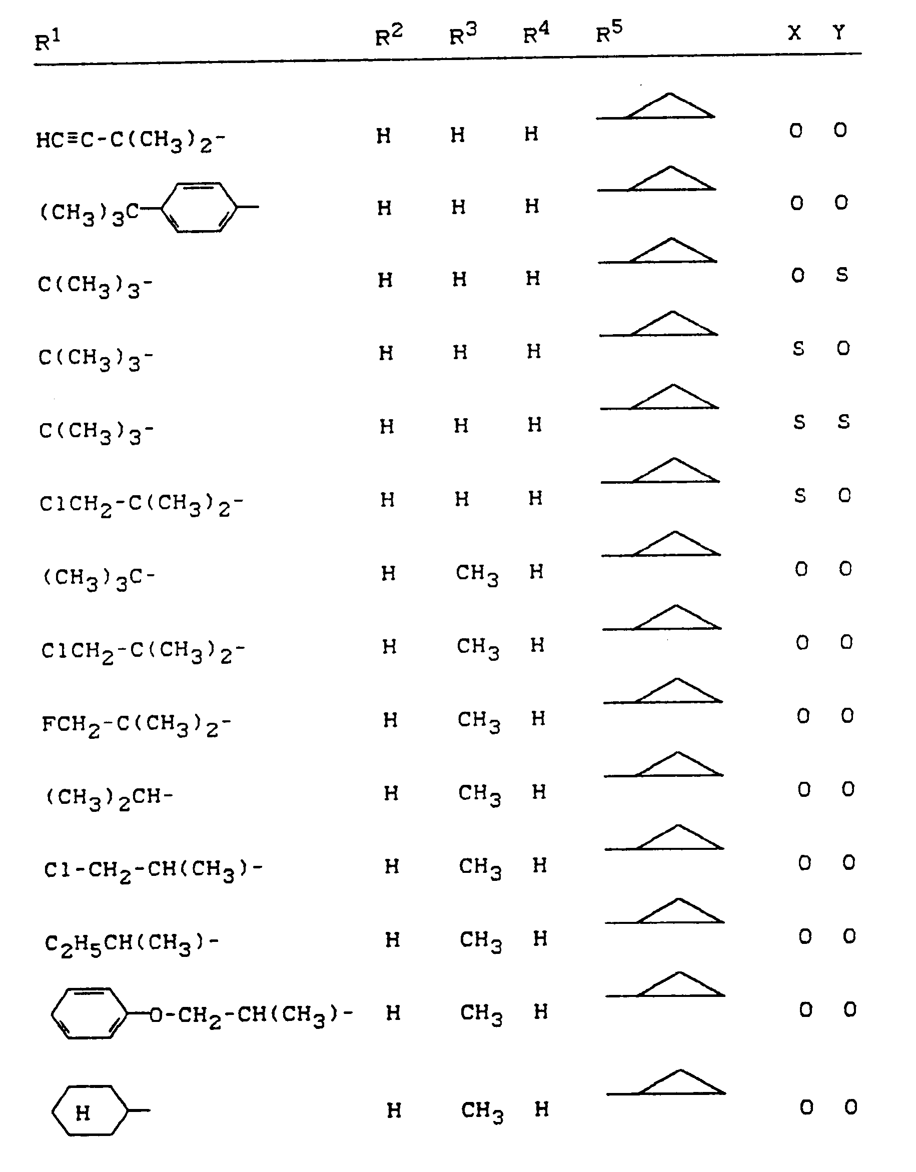

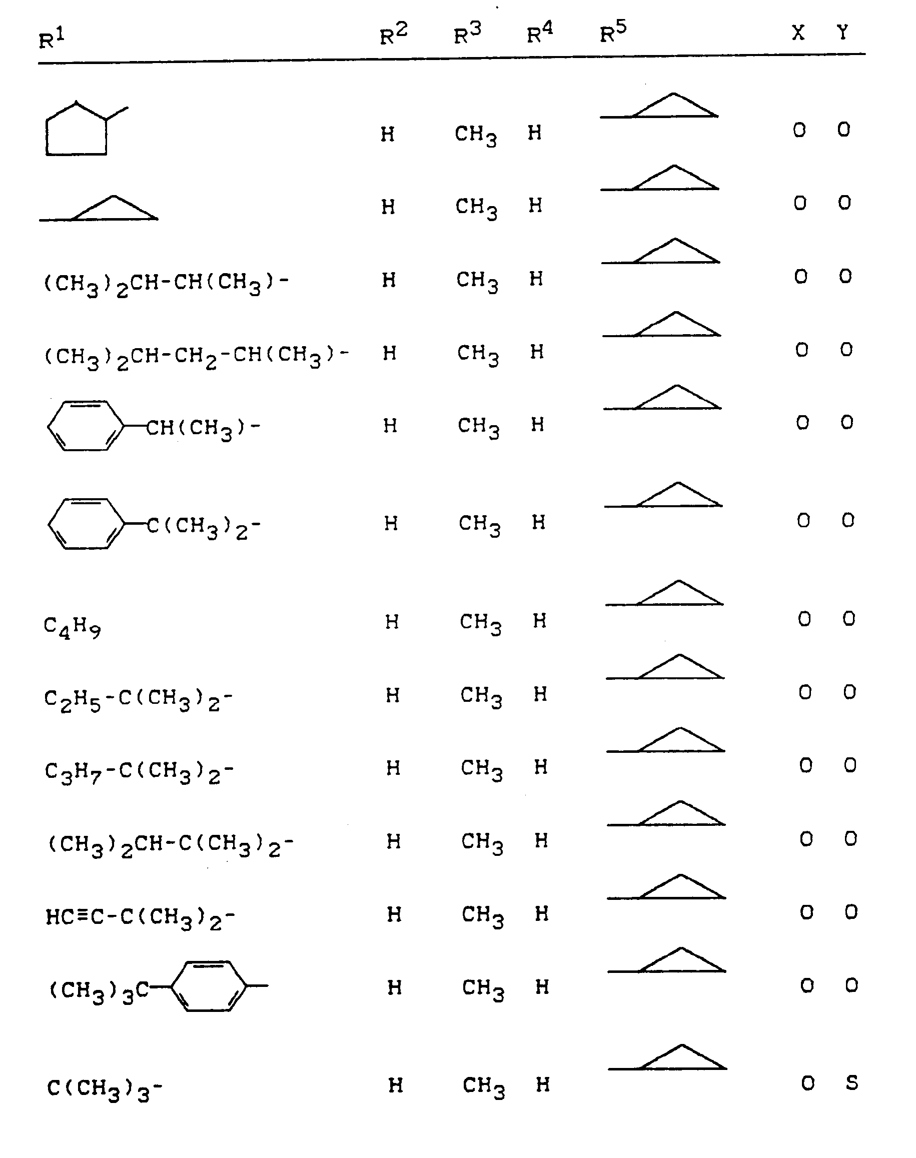

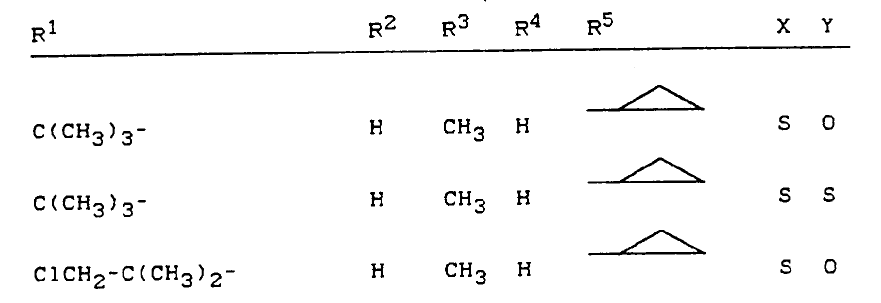

Verwendet man beispielsweise 4-Methylamino-5-dimethylamino-2,4-dihydro-3H-1,2,4-triazol-3-on und sec-Butylisocyanat als Ausgangsstoffe, so läßt sich der Reaktionsablauf beim erfindungsgemäßen Verfahren (a) durch das folgende Formelschema darstellen:

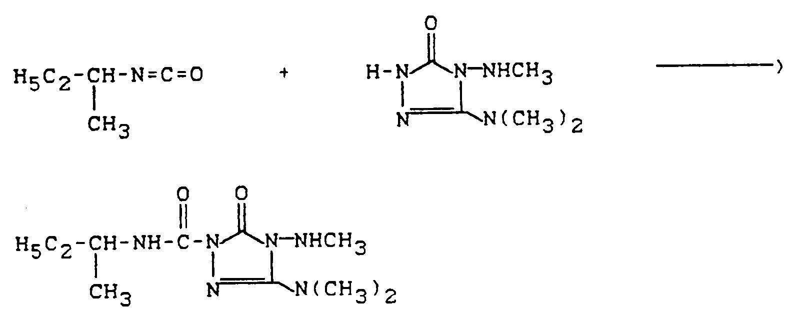

Verwendet man beispielsweise 2-Cyclohexylaminocarbonyl-4-isopropylidenamino-5-dipropylamino-2,4-dihydro-3H-1,2,4-triazol-3-on als Ausgangsstoff, so läßt sich der Reaktionsablauf beim erfindungsgemäßen Verfahren (b) durch das folgende Formelschema darstellen:

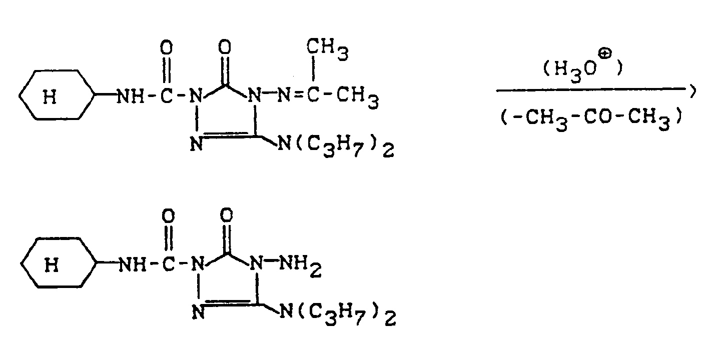

Verwendet man beispielsweise 2-Phenoxycarbonyl-4-methylamino-5-(N-methyl-propylamino)-2,4-dihydro-3H-1,2,4-triazol-3-on und Pentylamin als Ausgangsstoffe, so läßt sich der Reaktionsablauf beim erfindungsgemäßen Verfahren (c) durch das folgende Formelschema darstellen:

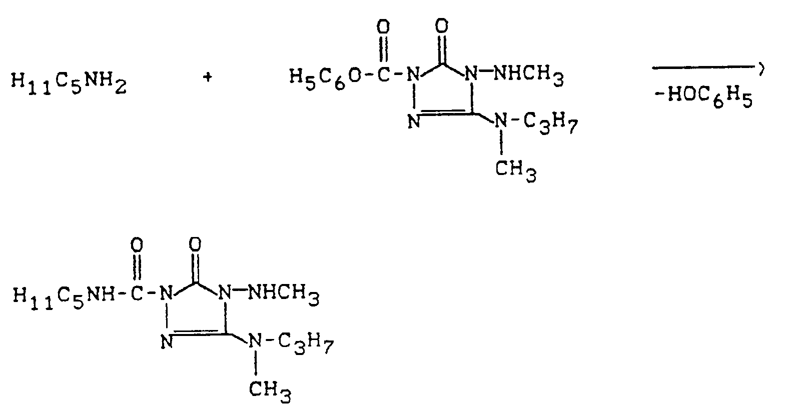

Verwendet man beispielsweise 4-Dimethylamino-5-diethylamino-2,4-dihydro-3H-1,2,4-triazol-3-on und N-Benzyl-O-phenyl-urethan als Ausgangsstoffe, so läßt sich der Reaktionsablauf beim erfindungsgemäßen Verfahren (d) durch das folgende Formelschema darstellen:

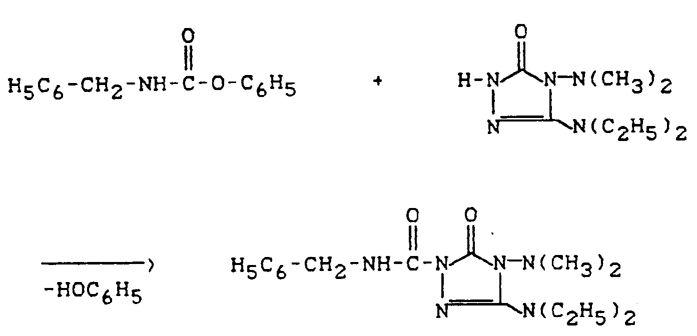

Verwendet man beispielsweise 2-(1-Methyl-1-phenyl-ethylamino-carbonyl)-4-ethoxymethylenamino-5-(pyrrolidin-1-yl)-2,4-dihydro- 3H-1,2,4-triazol-3-on und Natriumboranat als Ausgangsstoffe, so läßt sich der Reaktionsablauf beim erfindungsgemäßen Verfahren (e) durch das folgende Formelschema darstellen:

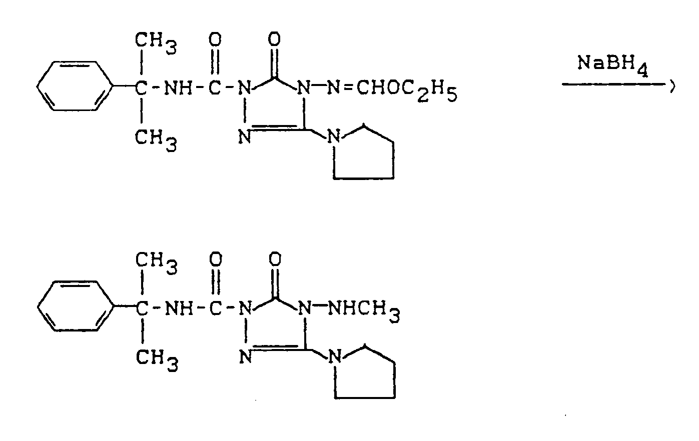

Verwendet man beispielsweise 2-(1-Methyl-1-trifluormethyl-ethyl-amino-carbonyl)-4-isobutylidenamino-5-(N-methyl-propylamino)-2,4-dihydro- 3H-1,2,4-triazol-3-on und Natrium-cyanoborhydrid als Ausgangsstoffe, so läßt sich der Reaktionsablauf beim erfindungsgemäßen Verfahren (f) durch das folgende Formelschema darstellen:

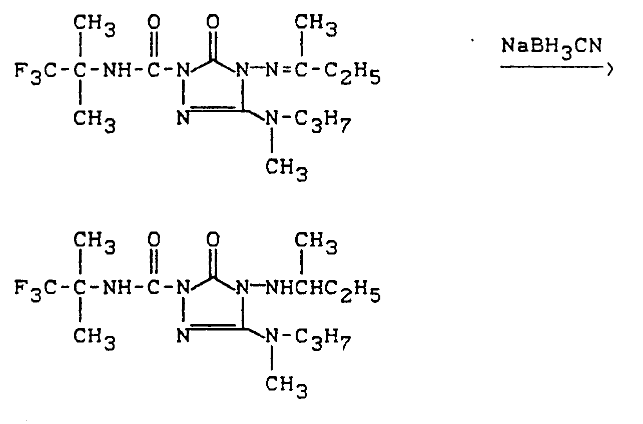

Die bei den erfindungsgemäßen Verfahren (a) und (d) zur Herstellung von Verbindungen der Formel (I) als Ausgangsstoffe zu verwendenden 4,5-Diamino-1,2,4-triazol-3-one sind durch die Formel (11) allgemein definiert.

In Formel (II) haben R², R³, R⁴, R⁵ und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R², R³, R⁴, R⁵ und Y angegeben wurden.

Die 4,5-Diamino-1,2,4-triazol-3-(thi)one der Formel (II) sind bekannt und/oder können nach an sich bekannten Verfahren hergestellt werden (vgl. Advan. Heterocycl. Chem. 5 (1965), 119-204; Chem. Ber. 99 (1966), 81-84; J. Chem. Soc. 1952, 4817; J. Heterocycl. Chem. 2 (1965), 302-304; Eur. J. Med. Chem. - Chim. Ther. 21 (1986), 235-244; J. Chem, Soc, C 1968, 2099-2107; J, Chem, Soc, C 1970, 2634; Liebigs Ann, Chem, 702 (1967), 101-111; Liebigs Ann, Chem, 703 (1967), 116-130).

Neu sind die 4,5-Diamino-1,2,4-triazol-3-one der allgemeinen Formel (Ila) 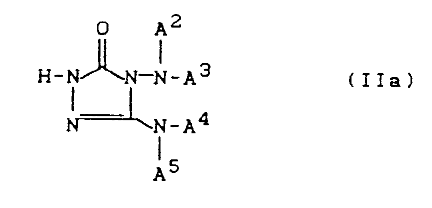 in welcher
A², A³, A⁴ und A⁵ gleich oder verschieden sind und für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw, 13 gleichen oder verschiedenen Halogenatomen, Cyanalkyl mit 1 bis 8 Kohlenstoffatomen, Alkoxyalkyl oder Alkylthioalkyl mit jeweils bis zu 4 Kohlenstoffatomen in den einzelnen Alkylteilen, für jeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen, in welcher weiter auch jeweils zwei dieser Reste -A² und A³ oderA⁴ und A^{5 -} zusammen für geradkettiges oder verzweigtes Alkandiyl oder Oxaalkandiyl mit jeweils 2 bis 6 Kohlenstoffatomen stehen können, und A⁵ auch für geradkettiges oder verzweigtes Alkoxy mit 1 bis 8 Kohlenstoffatomen stehen kann, mit der Maßgabe, daß wenigstens einer der Reste A², A³, A⁴ oder A⁵ von Wasserstoff verschieden ist.

Bevorzugt sind die neuen Verbindungen der Formel (Ila), in welcher
A² für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl oder Cyclohexyl steht,
A³ für Wasserstoff, Methyl, Ethyl, Propyl, Butyl, Allyl, Propargyl, Cyanoethyl oder Methoxyethyl steht, oder zusammen mit A² für Butan-1,4-diyl oder Pentan-1,5-diyl steht,
A⁴ für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl oder Cyclohexyl steht, und
A⁵ für Methyl, Ethyl, Propyl, Butyl, Allyl, Propargyl, Cyanoethyl, Methoxyethyl oder Methoxy steht, oder zusammen mit A⁴ für Butan-1,4-diyl oder Pentan-1,5-diyl oder 3-Oxa-pentan-1,5-diyl steht.

Man erhält die neuen Verbindungen der Formel (Ila), wenn man
(a) Amino- bzw. Imino-verbindungen der allgemeinen Formeln (X), (XI) oder (XII) 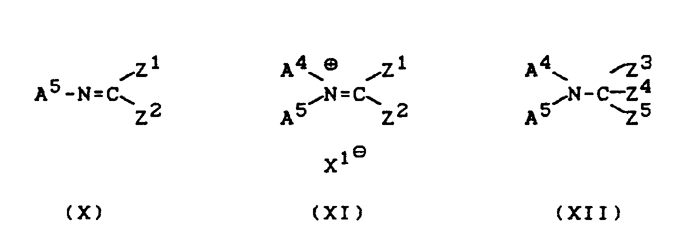 in welchen
   A⁴ und A⁵ die oben angegebenen Bedeutungen haben,
   X¹ für Halogen steht und
   Z¹, Z², Z³, Z⁴ und Z5 für in der Kohlensäurechemie übliche Abgangsgruppen stehen,
      mit Carbodihydrazid-Derivaten der allgemeinen Formeln (Xllla) oder (Xlllb) 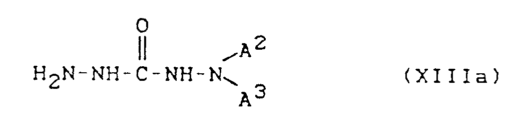 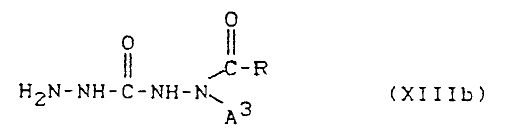 in welchen
   A² und A³ die oben angegebenen Bedeutungen haben und
   R für C₁-C₄ Alkyl, C₁-C₄ Alkoxy oder Phenyl steht,
      gegebenenfalls in Gegenart eines Verdünnungsmittels, wie z.B. Phenol und/oder Chlorbenzol, gegebenenfalls in Gegenwart eines Katalysators, wie z.B. Dibutylzinnoxid, und gegebenenfalls in Gegenwart eines Säureakzeptors, wie z.B. Natriumcarbonat, bei Temperaturen zwischen 20°C und 200°C umsetzt, gegebenenfalls die Gruppierung -CO-R durch Umsetzung mit einer wäßrigen Lauge, wie z.B. Natronlauge, anschließend bei Temperaturen zwischen 20°C und 120°C abspaltet und nach üblichen Methoden aufarbeitet (vgl. die Herstellungsbeispiele), oder wenn man
(ß) Diaminoguanidin-Derivate der allgemeinen Formel (XIV) 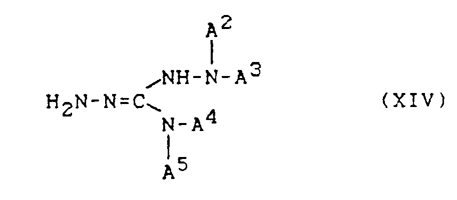 in welcher
   A², A³, A⁴ und A⁵ die oben angegebenen Bedeutungen haben,
      oder Säureaddukte von Verbindungen der Formel (XIV) oder Tautomere von Verbindungen der Formel (XIV)
      mit Kohlensäurederivaten der allgemeinen Formel (XV) 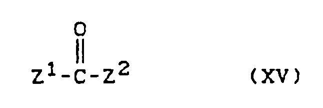 in welcher
   Z¹ und Z² die oben angegebenen Bedeutungen haben,
      gegebenenfalls in Gegenwart eines Verdünnungsmittels, wie z.B. Phenol, und gegebenenfalls in Gegenwart eines Säureakzeptors, wie z.B. Natriumcarbonat, bei Temperaturen zwischen 20°C und 200°C umsetzt und nach üblichen Methoden aufarbeitet (vgl. die Herstellungsbeispiele).

In den Formeln (X), (XI) und (XII) haben A⁴ und A⁵ vorzugsweise bzw. insbesondere diejenigen Bedeutungen, die bereits oben bei der Beschreibung der Verbindungen der Formel (Ila) als bevorzugt bzw. als besonders bevorzugt für A⁴ und A⁵ angegeben wurden und
Z¹, Z², Z³, Z⁴ und Z5 sind gleich oder verschieden und stehen vorzugsweise für Halogen, C₁-C₄-Alkoxy, C₁-C₄-Alkylthio, C₁-C₄-Alkylamino, Di-(Cᵢ-C₂-alkyl)-amino, Phenoxy oder Phenylthio, insbesondere für Chlor, Methoxy, Ethoxy, Methylthio, Ethylthio, Methylamino, Ethylamino, Dimethylamino oder Phenoxy.

Die Abgangsgruppen aus der Reihe Z¹ bis Z5 können gegebenenfalls auch verknüpft sein. Vorzugsweise stehen dann Z¹ und Z² oder Z³ und Z⁴ zusammen für C₂-C₄-Alkandioxy, insbesondere für Ethan-1,2-dioxy (-OCH₂ CH₂0-).

Die Verbindungen der Formeln (X), (XI) und (XII) sind bekannt und/oder können nach an sich bekannten Verfahren hergestellt werden (vgl. Synthesis 1977, 73-90; loc. cit. 1988, 460-466; J. Chem. Soc. 1951, 2492-2494; Chem. Ber. 120 (1987), 339-344; Tetrahedron Lett. 1982, 3539-3542; Houben-Weyl, Methoden der Organischen Chemie, 4. Auflage, Band E4 (1983), 522-624 und 652-722).

In den Formeln (Xllla) und (Xlllb) haben A² und A³ vorzugsweise bzw, insbesondere diejenigen Bedeutungen, die bereits oben bei der Beschreibung der Verbindungen der Formel (Ila) als bevorzugt bzw, als besonders bevorzugt für A², A³ und R angegeben wurden und R vorzugsweise für Methyl, Ethyl, Methoxy oder Ethoxy.

Die Carbodihydrazid-Derivate der Formel (Xllla) sind mit Ausnahme von Carbodihydrazid (A²=A³=H) noch nicht aus der Literatur bekannt.

Man erhält die neuen Verbindungen der Formel (Xllla), wenn man Kohlensäurederivate der Formel (XV) 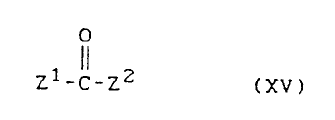 in welcher
Z¹ und Z² die oben angegebenen Bedeutungen haben,
   nacheinander mit etwa einem Moläquivalent eines Hydrazinderivates der Formel (XVI) 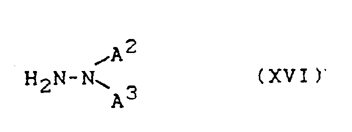 in welcher
A² und A³ die oben angegebenen Bedeutungen haben
   und etwa einem Moläquivalent Hydrazin oder Hydrazinhydrat bei Temperaturen zwischen 0°C und 100°C umsetzt. Die Aufarbeitung kann anschließend nach üblichen Methoden erfolgen. Vorzugsweise werden jedoch die Verbindungen der Formel (Xllla) nicht in reiner Form isoliert, sondern direkt weiter umgesetzt.

Die Carbodihydrazid-Derivate der Formel (Xlllb) sind noch nicht aus der Literatur bekannt. Man erhält die neuen Verbindungen der Formel (Xlllb), wenn man Hydrazinderivate der Formel (XVla) 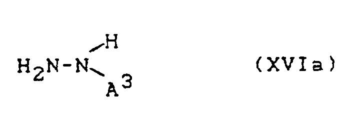 in welcher
A³ die oben angegebene Bedeutung hat,
   mit Acylierungsmitteln der Formel (XVII) 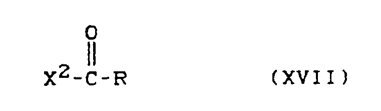 in welcher
R die oben angegebene Bedeutung hat und
X² für Halogen oder die Gruppierung -O-CO-R steht,
   gegebenenfalls in Gegenwart eines Verdünnungsmittels, wie z.B. Methanol, Methylenchlorid oder Toluol, und gegebenenfalls in Gegenwart eines Säureakzeptors, wie z.B. Kaliumcarbonat oder Pyridin, bei Temperaturen zwischen -80°C und +80_{°}C umsetzt, die hierbei erhaltenen acylierten Hydrazine der allgemeinen Formel (XVIII) 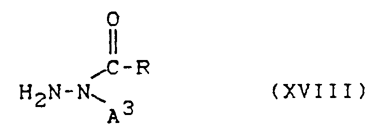 in welcher
A³ und R die oben angegebenen Bedeutungen haben,
   mit Kohlensäurederivaten der allgemeinen Formel (XV) 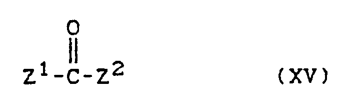 in welcher
Z¹ und Z² die oben angegebenen Bedeutungen haben,
   gegebenenfalls in Gegenwart eines Verdünnungsmittels, wie z.B. Methylenchlorid oder Ethylenchlorid, und gegebenenfalls in Gegenwart eines Säureakzeptors, wie z.B. Natriumcarbonat, bei Temperaturen zwischen -20°C und +80_{°}C umsetzt und die hierbei erhaltenen zweifach acylierten Hydrazine der Formel (XIX) 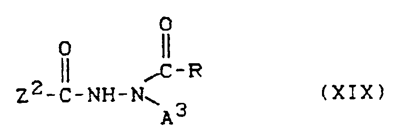 in welcher
A³, R und Z² die oben angegebenen Bedeutungen haben,
   mit Hydrazin oder Hydrazinhydrat gegebenenfalls in Gegenwart eines Verdünnungsmittels, wie z.B. Chlorbenzol, bei Temperaturen zwischen 0°C und 120°C umsetzt (vgl. die Herstellungsbeispiele).

Die als Vorprodukte benötigten Verbindungen der Formeln (XV), (XVI), (XVla) und (XVII) sind bekannte organische Synthesechemikalien.

In der Formel (XIV) haben A², A³, A⁴ und A⁵ vorzugsweise bzw. insbesondere diejenigen Bedeutungen, die bereits oben bei der Beschreibung der Verbindungen der Formel (Ila) als bevorzugt bzw. als besonders bevorzugt für A², A³, A⁴ und A⁵ angegeben wurden.

Die Verbindungen der Formel (XIV) sind bekannt und/oder können nach an sich bekannten Verfahren hergestellt werden (vgl. EP-A 150677).

In der Formel (XV) sind Z¹ und Z² gleich oder verschieden und stehen jeweils vorzugsweise für Halogen, C₁-C₄-Alkoxy, C₁-C₄-Alkylthio, C₁-C₄-Alkylamino, Di-(C₁-C₂-alkyl)-amino, Phenoxy oder Phenylthio, insbesondere für Chlor, Methoxy, Ethoxy, Phenoxy, Methylthio oder Dimethylamino. Z¹ und Z² können auch cyclisch verknüpft sein. Vorzugsweise stehen dann Z¹ und Z² zusammen für C₂-C₄-Alkandioxy, insbesondere für Ethan-1,2-dioxy (-OCH₂CH₂0-). Die Verbindungen der Formel (XV) sind bekannte organische Synthesechemikalien.

Die beim erfindungsgemäßen Verfahren (a) weiter als Ausgangsstoffe zu verwendenden Iso(thio)cyanate sind durch die Formel (III) allgemein definiert.

In Formel (III) haben R¹ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und X angegeben wurden.

Die Iso(thio)cyanate der Formel (III) sind bekannte organische Synthesechemikalien.

Die bei den erfindungsgemäßen Verfahren (b) und (f) zur Herstellung von Verbindungen der Formel (I) als Ausgangsstoffe zu verwendenden 4-Alkylidenamino-5-amino-1,2,4-triazol-3-(thi)one sind durch die Formel (IV) allgemein definiert.

In Formel (IV) haben R^{l}, R⁴, R⁵, X und Y vorzugsweise bzw, insbesondere diejenigen Bedeutungen, die bereits oben im Zusammenhang mit der Beschriebung der erfindungsgemäßen Verbindungen der Formel (I) vorzugsweise bzw. als insbesondere bevorzugt für R¹, R⁴, R⁵, X und Y angegeben wurden und R⁶ und R⁷ die oben angegebenen Bedeutungen haben.

Die 4-Alkylidenamino-5-amino-1,2,4-triazol-3-(thi)one der Formel (IV) sind noch nicht aus der Literatur bekannt.

Man erhält die neuen Verbindungen der Formel (IV), wenn man die oben beschriebenen 4,5-Diamino-1,2,4-triazol-3-(thi)one der allgemeinen Formel (II) 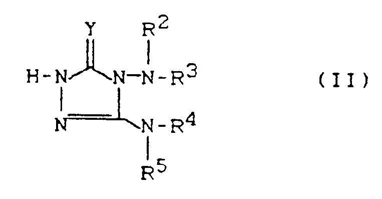 in welcher
R², R³, R⁴, R⁵ und Y die oben angegebenen Bedeutungen haben mit der Maßgabe, daß R² und R³ für Wasserstoff stehen,
   mit Aldehyden oder Ketonen der Formel (XX) 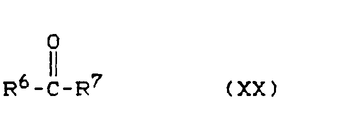 in welcher
R⁶ und R⁷ die oben angegebenen Bedeutungen haben,
   gegebenenfalls in Gegenwart eines Verdünnungsmittels, wie z.B. Methylenchlorid oder Toluol, und gegebenenfalls in Gegenwart eines Katalysators, wie z.B. p-Toluolsulfonsäure, bei Temperaturen zwischen 20°C und 120°C umsetzt und die so erhaltenen 4-Alkylidenamino-5-amino-1, 2,4-triazol-3-(thi)one der Formel (XXI) 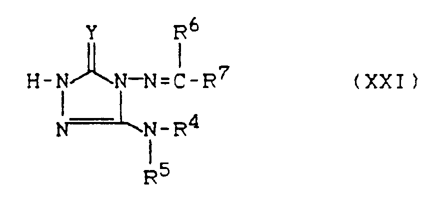 in welcher
R⁴, R⁵, R⁶, R⁷ und Y die oben angegebenen Bedeutungen haben,
   entweder in einer anschließenden zweiten Stufe mit Iso(thio)cyanaten der Formel (III) 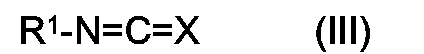 in welcher
R¹ und X die oben angegebenen Bedeutungen haben,
   gegebenenfalls in Gegenwart eines Verdünnungsmittels, wie z.B. Methylenchlorid oder Dioxan, und gegebenenfalls in Gegenwart eines Reaktionshilfsmittels, wie z.B. Triethylamin, bei Temperaturen zwischen 20°C und 150°C umsetzt,
   oder wenn man alternativ in einer zweiten Stufe die Verbindungen der Formel (XXI) mit Chlor(thio)ameisensäureestern der Formel (XXII) 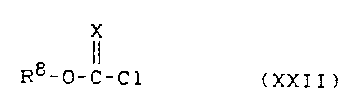 in welcher
X die oben angegebene Bedeutung hat und
R⁸ für Methyl, Ethyl, Propyl, Isopropyl, Butyl, Isobutyl, sec-Butyl, tert.-Butyl, Phenyl oder Benzyl steht, gegebenenfalls in Gegenwart eines Verdünnungsmittels, wie z.B. Tetrahydrofuran, und gegebenenfalls in Gegenwart eines Reaktionshilfsmittels, wie z.B. Natriumhydrid oder Kalium-tert-butylat, bei Temperaturen zwischen20° C und +40_{°} C umsetzt und die so erhaltenen 2-Oxy(thio)-carbonyl-4-alkylidenamino-5-amino-1,2,4-triazol-3-(thi)one der Formel (XXIII) 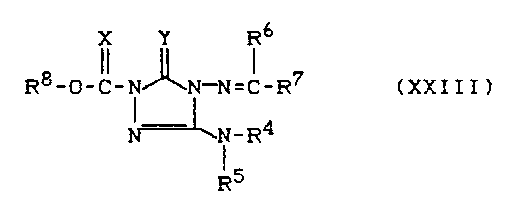 in welcher
R4, R⁵, R⁶, R⁷, R⁸, X und Y die oben angegebenen Bedeutungen haben,
   in einer anschließenden dritten Stufe mit Aminen der Formel (VI) 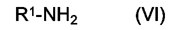 in welcher
R¹ die oben angegebene Bedeutung hat,
   gegebenenfalls in Gegenwart eines Verdünnungsmittels, wie z.B. Tetrahydrofuran oder Dioxan, und gegebenenfalls in Gegenwart einer Base, wie z.B. Natriumhydroxid oder Kaliumhydroxid, bei Temperaturen zwischen 20°C und 100°C umsetzt.

Dabei ist es möglich und gegebenenfalls von Vorteil, die Umsetzung der Verbindungen der Formel (XXI) mit Chlor(thio)ameisensäureestern und die anschließende Umsetzung mit Aminen in sogenannten Eintopfverfahren durchzuführen.

Die Aldehyde bzw, Ketone der Formel (XX), die Iso(thio)cyanate der Formel (111), die Chlor(thio)ameisensäureesterder Formel (XXII) und die Amine der Formel (VI) sind bekannte organische Synthesechemikalien.

Die 4-Alkylidenamino-5-amino-1,2,4-triazol-3-(thi)one der Formel (XXI) - mit der Maßgabe, daß R⁴ und/ oder R⁵ von Wasserstoff verschieden sind - und die 2-Oxy(thio)carbonyl-4-alkylidenamino-5-amino-1,2,4-triazol-3-(thi)one der Formel (XXIII) sind noch nicht aus der Literatur bekannt und Gegenstand der vorliegenden Patentanmeldung.

In den Formeln (XXI) und (XXIII) haben R⁴, R⁵, R⁶, R⁷ und Y vorzugsweise bzw, insbesondere diejenigen Bedeutungen, die oben bei der Beschreibung der erfindingsgemäßen Verbindungen der Formel (IV) vorzugsweise bzw. als besonders bevorzugt für R⁴, R⁵, R⁶, R⁸ und Y angegeben wurden.

Die beim erfindungsgemäßen Verfahren (c) zur Herstellung von Verbindungen der Formel (I) als Ausgangsstoffe zu verwendenden substituierten 1,2,4-Triazol-3-(thi)one sind durch die Formel (V) allgemein definiert.

In Formel (V) haben R², R³, R⁴, R⁵, X und Y vorzugsweise bzw, insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R², R³, R⁴, R⁵, X und Y angegeben wurden, und
R⁸ die oben angegebenen Bedeutungen hat.

Die substituierten 1,2,4-Triazol-3-(thi)one der Formel (V) sind noch nicht aus der Literatur bekannt. Man erhält die neuen Verbindungen der Formel (V), wenn man 4,5-Diamino-1,2,4-triazol-3-(thi)one der Formel (11) 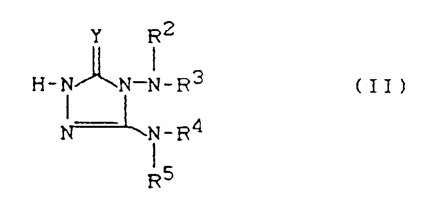 in welcher
R², R³, R⁴, R⁵ und Y die oben angegebenen Bedeutungen haben,
   mit Chlor(thio)ameisensäureestern der Formel (XXII) 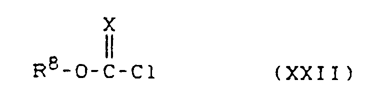 in welcher
R⁸ und X die oben angegebenen Bedeutungen haben,
   gegebenenfalls in Gegenwart eines Verdünnungsmittels, wie z.B. Tetrahydrofuran, und gegebenenfalls in Gegenwart eines Säureakzeptors, wie z.B. Kalium-tert-butylat, bei Temperaturen zwischen -20°C und +100_{°}C umsetzt.

Die beim erfindungsgemäßen Verfahren (c) weiter als Ausgangsstoffe zu verwendenden Aminoverbindungen sind durch die Formel (VI) allgemein definiert.

In Formel (VI) hat R¹ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R¹ angegeben wurde.

Die beim erfindungsgemäßen Verfahren (d) zur Herstellung von Verbindungen der Formel (I) als Ausgangsstoffe zu verwendenden (Thio)Urethane sind durch die Formel (VII) allgemein definiert.

In Formel (VII) haben R¹ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R⁹ und X angegeben wurden.

Die Ausgangsstoffe der Formel (VII) sind bekannte organische Synthesechemikalien.

Die beim erfindungsgemäßen Verfahren (e) zur Herstellung von Verbindungen der Formel (I) als Ausgangsstoffe zu verwendenden 4-Oxyalkylidenamino-5-amino-1,2,4-triazol-(thi)one sind durch die Formel (VIII) allgemein definiert.

In Formel (VIII) haben R¹, R⁴, R⁵, X und Y vorzugsweise bzw, insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R⁴, R⁵, R¹⁰, R¹¹, X und Y angegeben wurden.

Vorzugsweise stehen R¹⁰ für Wasserstoff, Methyl, Ethyl, Propyl, Phenyl oder Benzyl und R¹¹ für Methyl, Ethyl, Propyl oder Benzyl.

Die 4-Oxyalkylidenamino-5-amino-1,2,4-triazol-3-(thi)one der Formel (VIII) sind noch nicht aus der Literatur bekannt.

Man erhält die neuen Verbindungen der Formel (VIII), wenn man 4,5-Diamino-1,2,4-triazol-3-(thi)one der allgemeinen Formel (II) 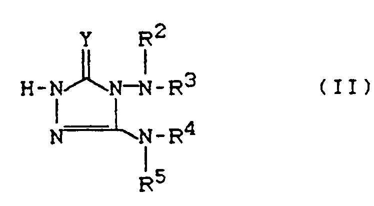 in welcher
R² und R³ für Wasserstoff stehen und
Y, R⁴ und R⁵ die oben angegebenen Bedeutungen haben,
   mit Orthocarbonsäureestern der allgemeinen Formel (XXIV) 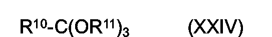 in welcher
R¹⁰ und R¹¹ die oben angegebenen Bedeutungen haben,
   gegebenenfalls in Gegenwart eines Verdünnungsmittels, wie z.B. Chloroform, Toluol oder Chlorbenzol, und gegebenenfalls in Gegenwart eines Katalysators, wie z.B. p-Toluolsulfonsäure, bei Temperaturen zwischen 50°C und 200°C umsetzt und die so erhaltenen Oxyalkylidenverbindungen der Formel (XXV) 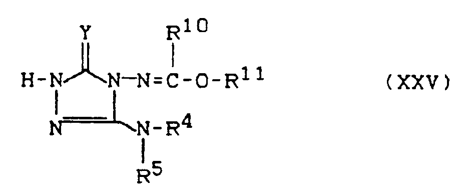 in welcher
R⁴, R⁵, R¹⁰, R¹¹ und Y die oben angegebenen Bedeutungen haben,
   in einer anschließenden zweiten Stufe mit Iso(thio)cyanaten der Formel (III) 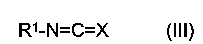 in welcher
R¹ und X die oben angegebenen Bedeutungen haben,
   gegebenenfalls in Gegenwart eines Verdünnungsmittels, wie z.B. Methylenchlorid oder Dioxan, und gegebenenfalls in Gegenwart eines Reaktionshilfsmittels, wie z.B. Triethylamin, bei Temperaturen zwischen 20°C und 150°C umsetzt.

Alternativ erhält man die Verbindungen der Formel (VIII) auch, wenn man Verbindungen der Formel (I) 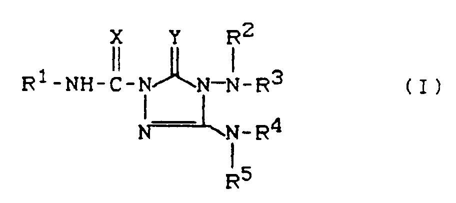 in welcher
R² und R³ für Wasserstoff stehen und
R¹, R⁴, R⁵, X und Y die oben angegebenen Bedeutungen haben,

mit Orthocarbonsäureestern der Formel (XXIV) 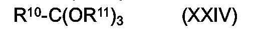 in welcher
R¹⁰ und R¹¹ die oben angegebenen Bedeutungen haben,

gegebenenfalls in Gegenwart eines Verdünnungsmittels, wie z.B. Chloroform, Toluol oder Chlorbenzol, und gegebenenfalls in Gegenwart eines Katalysators, wie z.B. p-Toluolsulfonsäure, bei Temperaturen zwischen 50°C und 200°C umsetzt.

Die Orthocarbonsäureester der Formel (XXIV) sind bekannte organische Synthesechemikalien.

Die Oxyalkylidenverbindungen der Formel (XXV) sind noch nicht aus der Literatur bekannt und Gegenstand der vorliegenden Patentanmeldung.

In der Formel (XXV) haben R⁴, R⁵ und Y vorzugsweise bzw. insbesondere diejenigen Bedeutungen, die oben bei der Beschreibung der erfindungsgemäßen Verbindungen der Formel (I) als bevorzugt bzw. als besonders bevorzugt für R⁴, R⁵ und Y angegeben wurden und R¹⁰ und R¹¹ stehen für C₁-C₆-Alkyl, C₂-C₆-Alkenyl, C₂-C₆-Alkinyl, C₃-Cₛ-Cycloalkyl, Phenyl oder Benzyl; R¹⁰ auch für Wasserstoff; vorzugsweise steht R¹⁰ fürWasserstoff, Methyl, Ethyl, Propyl, Phenyl oder Benzyl und R¹¹ für Methyl, Ethyl, Propyl oder Benzyl.

Die bei Verfahren (e) weiter als Ausgangsstoffe benötigten Hydridkomplexe der Formel (IX) sind bekannte Synthesechemikalien.

Das erfindungsgemäße Verfahren (a) wird vorzugsweise in Gegenwart eines Verdünnungsmittels durchgeführt.

Als Verdünnungsmittel zur Durchführung des erfindungsgemäßen Verfahrens (a) kommen insbesondere inerte organische Lösungsmittel infrage. Hierzu gehören beispielsweise aliphatische, alicyclische oder aromatische, gegebenenfalls halogenierte Kohlenwasserstoffe, wie Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (a) wird gegebenenfalls in Gegenwart eines geeigneten Reaktionshilfsmittels durchgeführt. Als solche kommen alle üblichen anorganischen oder organischen Basen in Frage. Hierzu gehören beispielsweise tertiäre Amine, wie Triethylamin, N,N-Dimethylanilin, N,N-Diethylbenzylamin, N,N-Dimethylcyclohexylamin oder Dibutylzinndilaureat, Pyridin, N,N-Di-methylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens (a) setzt man auf 1 Mol 4,5-Diamino-1,2,4-triazol-3-(thi)on der Formel (11) im allgemeinen 1.0 bis 2.0 Mol, vorzugsweise 1.0 bis 1.5 Mol an Iso(thio)cyanat der Formel (111) und gegebenenfalls 0.001 bis 2.0 Mol, vorzugsweise 0.001 bis 1.0 Mol an Reaktionshilfsmittel ein. Die Reaktionsdurchführung, Aufarbeitung und Isolierung der Reaktionsprodukte erfolgt nach allgemein üblichen Methoden.

Als Säuren zur Durchführung des erfindungsgemäßen Verfahrens (b) kommen alle üblicherweise für Hydrazonspaltungen verwendbaren anorganischen und organischen Säuren infrage. Vorzugsweise verwendet man anorganische Mineralsäuren wie Chlorwasserstoffsäure, Schwefelsäure oder Phosphorsäure.

Als Verdünnungsmittel zur Durchführung des erfindungsgemäßen Verfahrens (b) kommen alle üblichen organischen oder anorganischen Lösungsmittel infrage. Vorzugsweise verwendet man polare mit Wasser mischbare organische Lösungsmittel, insbesondere Alkohole, wie Methanol, Ethanol, Propanol oder Butanol, deren Gemische mit Wasser oder reines Wasser als Verdünnungsmittel.

Die Reaktionstemperaturen können bei der Durchführung des erfindungsgemäßen Verfahrens (b) in einem größeren Bereich variiert werden. Im allgemeinen arbeitet man bei Temperaturen zwischen 20°C und 150°C, vorzugsweise bei Temperaturen zwischen 50°C und 120°C.

Das erfindungsgemäße Verfahren (b) wird üblicherweise bei Normaldruck oder unter vermindertem Druck durchgeführt. Arbeitet man unter vermindertem Druck, so kommen Druckbereiche zwischen 20 und 400 mbar, vorzugsweise zwischen 100 und 200 mbar infrage.

Zur Durchführung des erfindungsgemäßen Verfahrens (b) setzt man auf 1 Mol 4-AIkyIidenamino-5-amino-1,2,4-triazol-3-(thi)on der Formel (IV) im allgemeinen 0.01 bis 50 Mol, vorzugsweise 0.1 bis 20 Mol einer Säure ein.

Im allgemeinen löst man die Verbindung der Formel (IV) in einem geeigneten Verdünnungsmittel, gibt dann die erforderliche Säuremenge dazu und engt die Mischung unter vermindertem Druck über mehrere Stunden langsam ein.

In einer besonderen Durchführungsform ist es auch möglich, das erfindungsgemäße Verfahren (b) und die Herstellung der dafür erforderlichen Vorprodukte der Formel (IV) in einem Reaktionsschritt in einem sogenannten Eintopfverfahren durchzuführen.

Dabei gibt es die Möglichkeit, als Ausgangsstoffe die Verbindungen der Formel (XXIII) zu wählen und diese nacheinander im Eintopfverfahren mit Aminen der Formel (VI) und anschließend mit Säure gemäß dem erfindungsgemäßen Verfahren (b) umzusetzen oder alternativ, als Ausgangsstoffe die Verbindungen der Formel (XXI) zu wählen und diese nacheinander im Eintopfverfahren mit (Thio)Chlorameisensäureestern der Formel (XXII), dann mit Aminen der Formel (VI) und anschließend mit Säure gemäß dem erfindungsgemäßen Verfahren (b) umzusetzen.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder-diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester, oder Sulfoxide, wie Dimethylsulfoxid.

Das erfindungsgemäße Verfahren (c) kann gegebenenfalls in Gegenwart eines geeigneten Reaktionshilfsmittels durchgeführt werden. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 120 °C, vorzugsweise bei Temperaturen zwischen 20 °C und 50 °C.

Zur Durchführung des erfindungsgemäßen Verfahrens (c) setzt man auf 1 Mol substituiertes 1,2,4-Triazol-3-(thi)on der Formel (V) im allgemeinen 1 bis 5 Mol, vorzugsweise 1.0 bis 2.5 Mol, Aminoverbindung der Formel (VI) und gegebenenfalls 0.1 bis 2 Mol, vorzugsweise 1.0 bis 1.2 Mol, Reaktionshilfsmittel ein.

Die Reaktionsdurchführung, Aufarbeitung und Isolierung der Reaktionsprodukte erfolgt nach allgemein üblichen Methoden.

In einer besonderen Durchführungsform ist es auch möglich, das erfindungsgemäße Verfahren (c) und die Herstellung der dafür erforderlichen Vorprodukte der Formel (V) in einem Reaktionsschritt in einem sogenannten Eintopfverfahren durchzuführen.

Man geht dabei aus von Verbindungen der Formel (11) und setzt diese nacheinander im Eintopfverfahren zunächst mit (Thio)Chlorameisensäureestern der Formel (XXII) und anschließend mit Aminen der Formel (VI) gemäß dem erfindungsgemäßen Verfahren (c) um.

Das erfindungsgemäße Verfahren (d) wird vorzugsweise in Gegenwart eines Verdünnungsmittels durchgeführt. Es können hierbei die gleichen Lösungsmittel verwendet werden, die oben für das erfindungsgemäße Verfahren (a) angegeben sind.

Verfahren (d) wird vorzugsweise in Gegenwart eines Reaktionshilfsmittels durchgeführt. Es können hierbei die gleichen Reaktionshilfsmittel eingesetzt werden, die oben für das erfindungsgemäße Verfahren (c) angegeben sind.

Die Reaktionstemperaturen können bei dem erfindungsgemäßen Verfahren (d) in einem größeren Bereich variiert werden. Im allgemeinen arbeitet man bei Temperaturen zwischen 0 °C und 150 °C, vorzugsweise bei Temperaturen zwischen 10 °C und 120 °C.

Zur Durchführung des erfindungsgemäßen Verfahrens (d) setzt man auf 1 Mol 4,5-Diamino-1,2,4-triazol-3-(thi)on der Formel (11) im allgemeinen 1 bis 2 Mol, vorzugsweise 1.0 bis 1.5 Mol, (Thio)Urethan der Formel (VII) ein.

Die Reaktionsdurchführung Aufarbeitung und Isolierung der Produkte der Formel (I) erfolgt nach allgemein üblichen Methoden.

Das erfindungsgemäße Verfahren (e) wird vorzugsweise in Gegenwart eines polaren Lösungsmittels durchgeführt. Als solche kommen vorzugsweise Wasser, Alkohole wie Methanol, Ethanol, Propanol, Isopropanol, Butanol, Isobutanol, sec-Butanol und tert-Butanol, Etheralkohole wie Methoxyethanol und Ethoxyethanol, oder Ether wie Diethylether, Dipropylether, Diisopropylether, Tetrahydrofuran und Dioxan, in Betracht.

Die Reaktionstemperaturen können bei der zweiten Stufe von Verfahren (e) in einem größeren Bereich variiert werden. Im allgemeinen arbeitet man bei Temperaturen zwischen -20°C und +100_{°}C, vorzugsweise zwischen 0°C und +30_{°}C.

Zur Durchführung des erfindungsgemäßen Verfahrens (e) setzt man auf 1 Mol 4-Oxyalkylidenamino-5-amino-1,2,4-triazol-3-(thi)on der Formel (VIII) im allgemeinen 0.5 bis 5 Mol, vorzugsweise 1 bis 3 Mol, Hydridkomplex der Formel (IX) ein.

Die Reaktionsdurchführung, Aufarbeitung und Isolierung der Reaktionsprodukte erfolgt nach allgemein üblichen Methoden.

Das erfindungsgemäße Verfahren (f) wird unter Verwendung eines Reduktionsmittels und gegebenenfalls eines Katalysators durchgeführt. Geeignete Systeme aus Reduktionsmitteln und Katalysatoren sind beispielsweise Wasserstoff in Kombination mit üblichen Hydrierungskatalysatoren, wie z.B. Raney-Nickel, Palladium oder Platin, ferner auch gegebenenfalls komplexe Metallhydride, wie z.B. Lithiumalanat, Natriumboranat und Natriumcyanoborhydrid, gegebenenfalls in Kombination mit sauren Katalysatoren, wie z.B. Salzsäure oder Essigsäure.

Verfahren (f) wird vorzugsweise in Gegenwart eines Verdünnungsmittels durchgeführt. Es können hierbei die gleichen Lösungsmittel verwendet werden, die oben für das erfindungsgemäße Verfahren (e) angegeben sind.

Die Reaktionstemperaturen können bei dem erfindungsgemäßen Verfahren (f) in einem größeren Bereich variiert werden. Im allgemeinen arbeitet man bei Temperaturen zwischen -20 °C und +100 °C, vorzugsweise bei Temperaturen zwischen 0 °C und +30 °C.

Die Reaktionsdurchführung, Aufarbeitung und Isolierung der Reaktionsprodukte erfolgt nach üblichen Methoden.

Die erfindungsgemäßen Wirkstoffe können als Defoliants, Desiccants, Krautabtötungsmittel und insbesondere als Unkrautvernichtungsmittel verwendetwerden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der an gewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und dikotylen Unkräutern, insbesondere in monokotylen Kulturen, sowohl im Vorauflauf- als auch im Nachauflauf-Verfahren. Mais zeigt insbesondere bei der Nachauflauf-Anwendung eine sehr gute Verträglichkeit für die erfindungsgemäßen Wirkstoffe.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oderfesten Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendetwerden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (META-BENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BEN-TAZON); Methyl-5-(2,4-dichlorphenoxy)-2-nitrobenzoat (BIFENOX); 3,5-Dibrom-4-hydroxy-benzonitril (BRO-MOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-me thylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphen- oxy)-propionsäure (MCPP); N-Methyl-2-(1,3-benzthiazol-2-yloxy)-acetanilid (MEFENACET); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); O-(6-Chlor-3-phenylpyridazin-4-yl)-S-octyl- thiocarbonat (PYRIDATE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

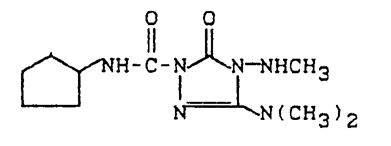

### (Verfahren (a))

2,3 g (20.7 mMol) Cyclopentylisocyanat werden zu einer Mischung aus 3,2 g (20 mMol) 4-Methylamino-5-dimethylamino-2,4-dihydro-3H-1,2,4-triazol-3-on, 80 ml Acetonitril und 100 mg Diazabicycloundecen (DBU) gegeben und das Reaktionsgemisch wird zwei Tage bei 20°C gerührt. Dann wird eingeengt, der Rückstand in 100 ml Methylenchlorid aufgenommen, mit Wasser gewaschen, mit Natriumsulfat getrocknet und filtriert. Das Filtrat wird eingeengt, der Rückstand mit Petrolether verrieben und das kristallin angefallene Produkt durch Absaugen isoliert.

Man erhält 4,6 g (85% der Theorie) 2-Cyclopentylaminocarbonyl-4-methylamino-5-dimethylamino-2,4-dihydro-3H-1,2,4-triazol-3-on vom Schmelzpunkt 137°C.

### Beispiel 2

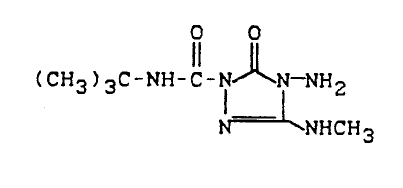

### (Verfahren (b))

4,6 g (15 mMol) 2-tert-Butylaminocarbonyl-4-(4-methyl-2-pentyliden-amino)-5-methylamino-2,4-dihydro-3H-1,2,4-triazol-3-on werden in einer Mischung aus 50 ml Wasser und 50 ml Ethanol aufgenommen und mit 2 ml konz. Salzsäure versetzt. Das Reaktionsgemisch wird im Wasserstrahlvakuum langsam eingeengt, der Rückstand in 50 ml Wasser/50 ml Ethanol aufgenommen und nach Zugabe von 2 ml konz. Salzsäure erneut eingeengt. Dann wird der Rückstand in 150 ml Methylenchlorid aufgenommen, mit 10%iger Sodalösung gewaschen, mit Natriumsulfat getrocknet und filtriert. Das Filtrat wird eingeengt, der Rückstand mit Petrolether verrieben und das kristallin angefallene Produkt durch Absaugen isoliert.

Man erhält 1,0 g (29% der Theorie) 2-tert-Butylaminocarbonyl-4-amino-5-methylamino-2,4-dihydro-3H-1,2,4-triazol-3-on vom Schmelzpunkt 174°C.

Analog zu den Beispielen 1 und 2 und entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

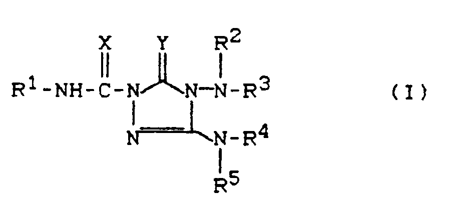

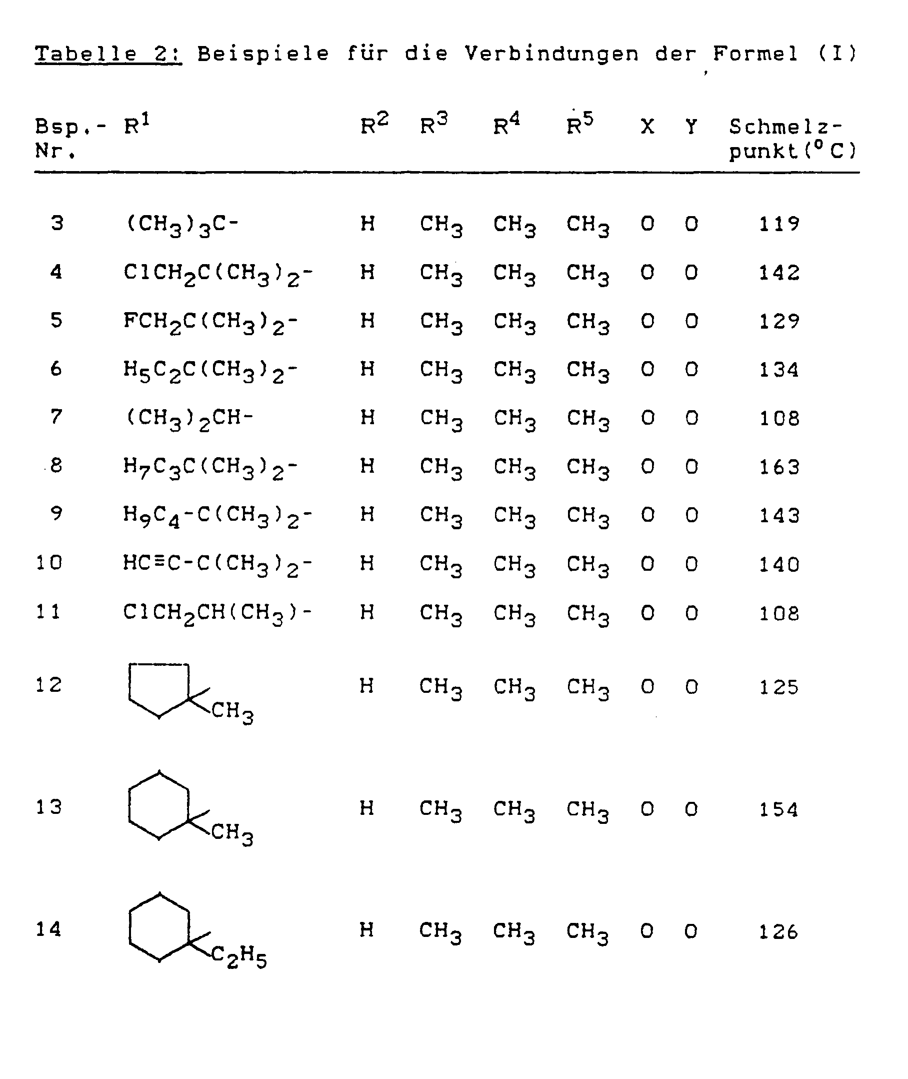

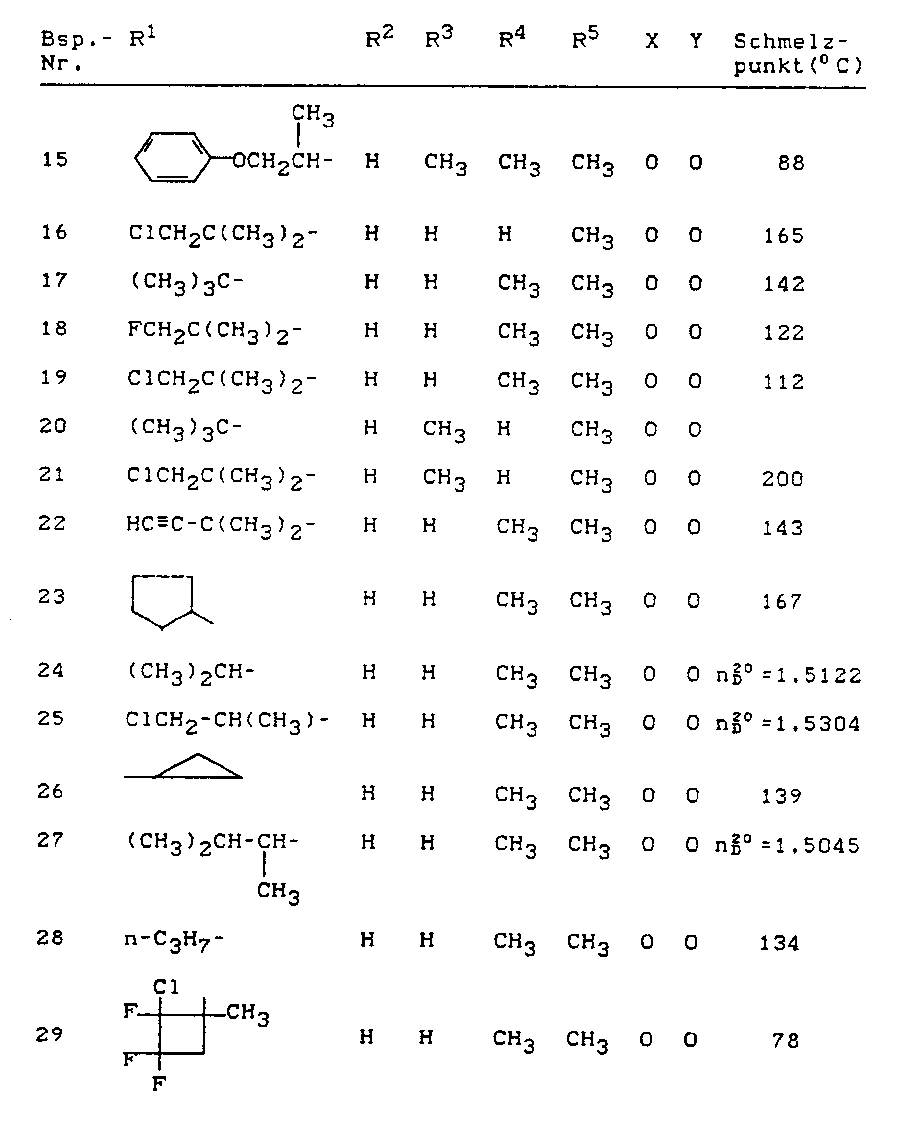

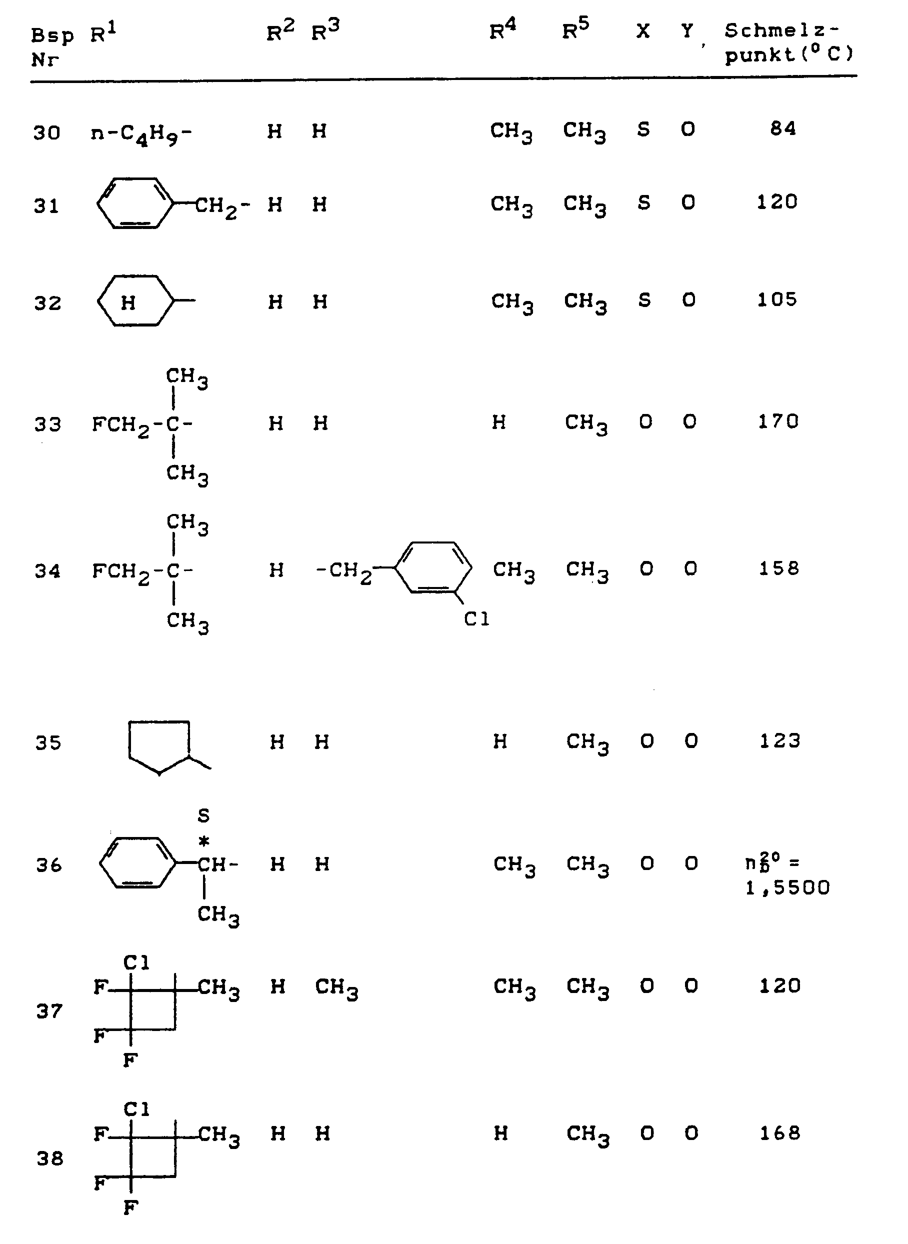

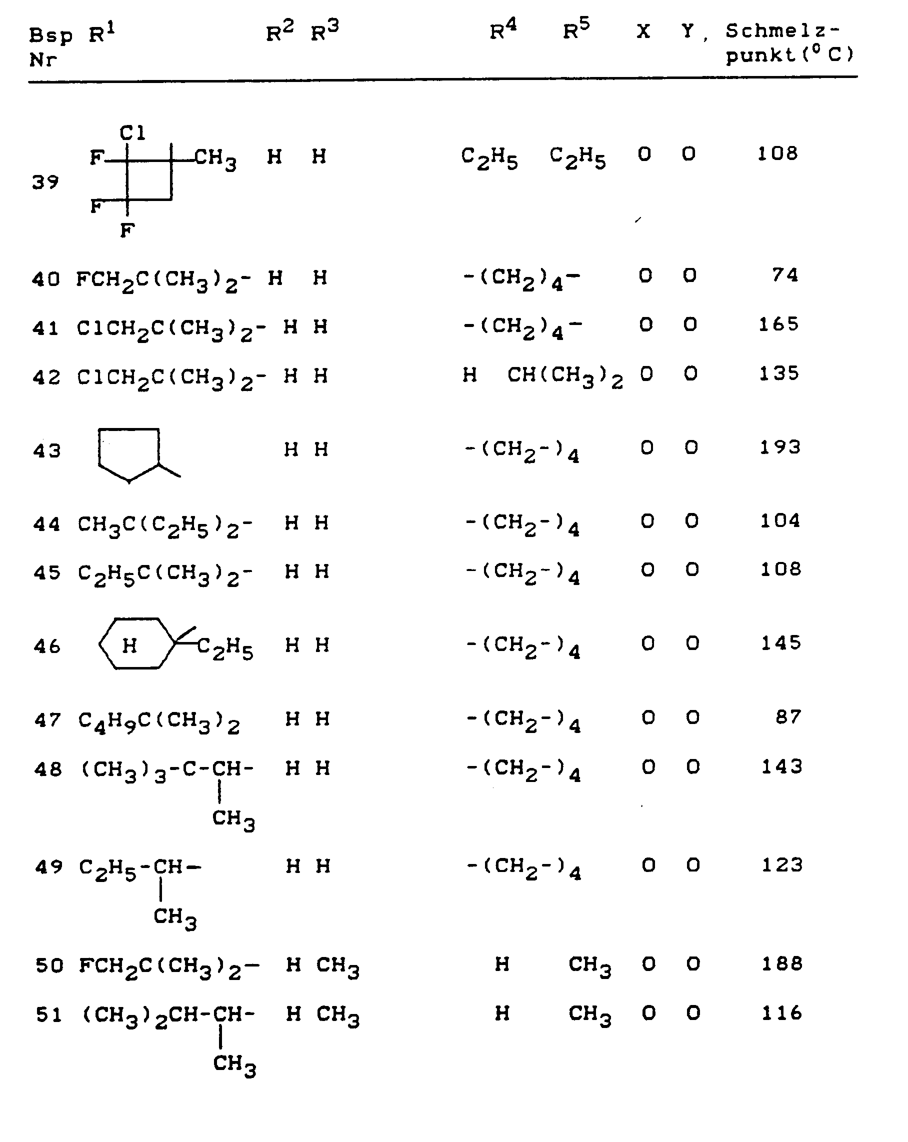

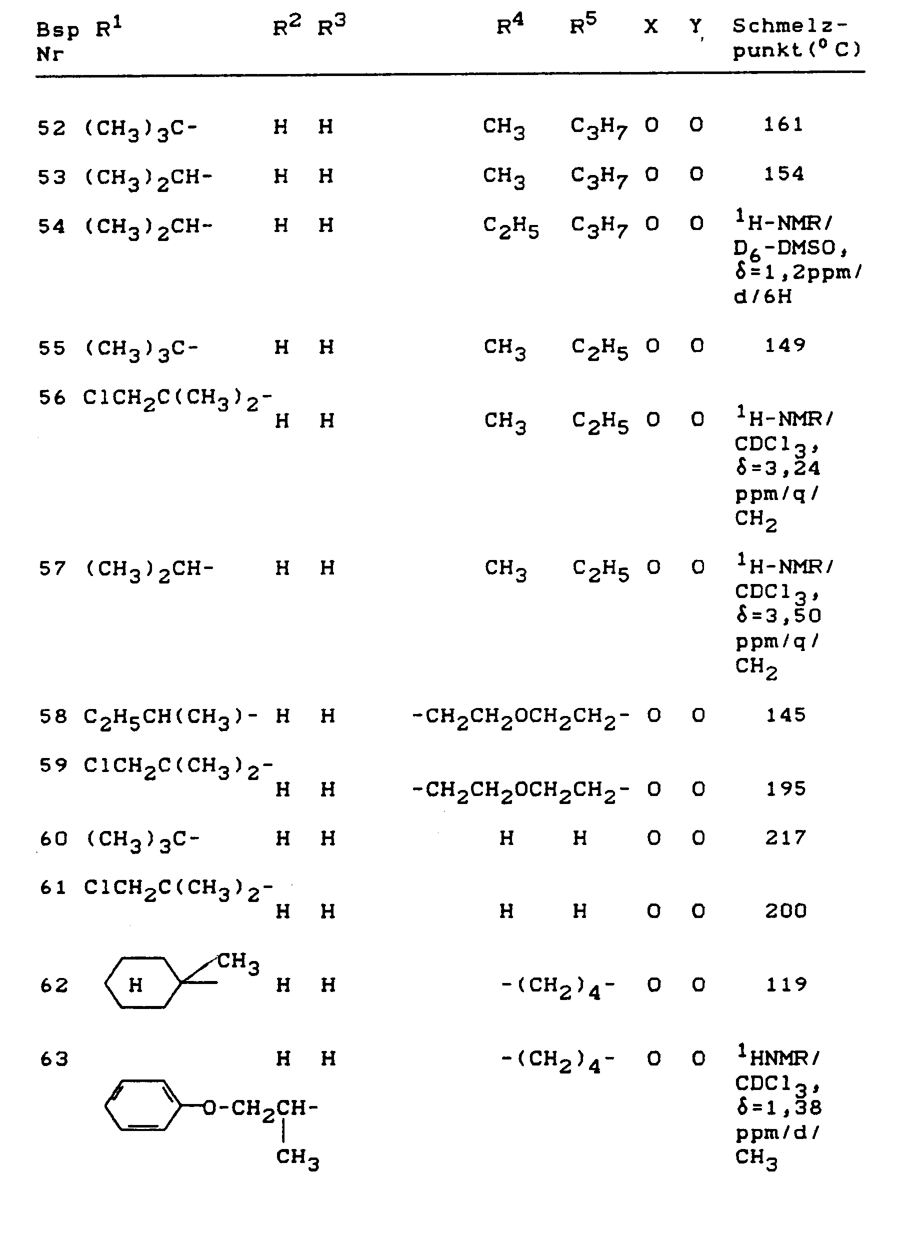

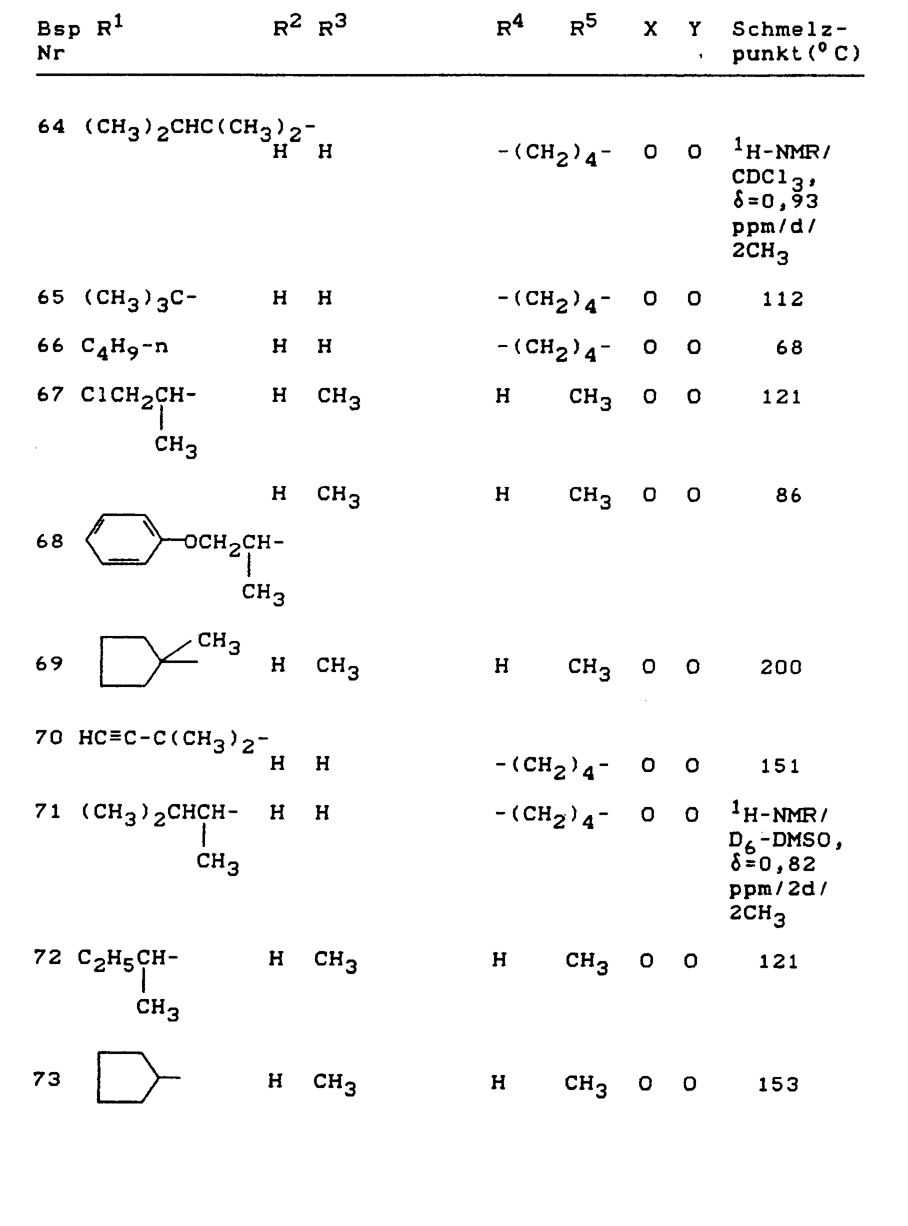

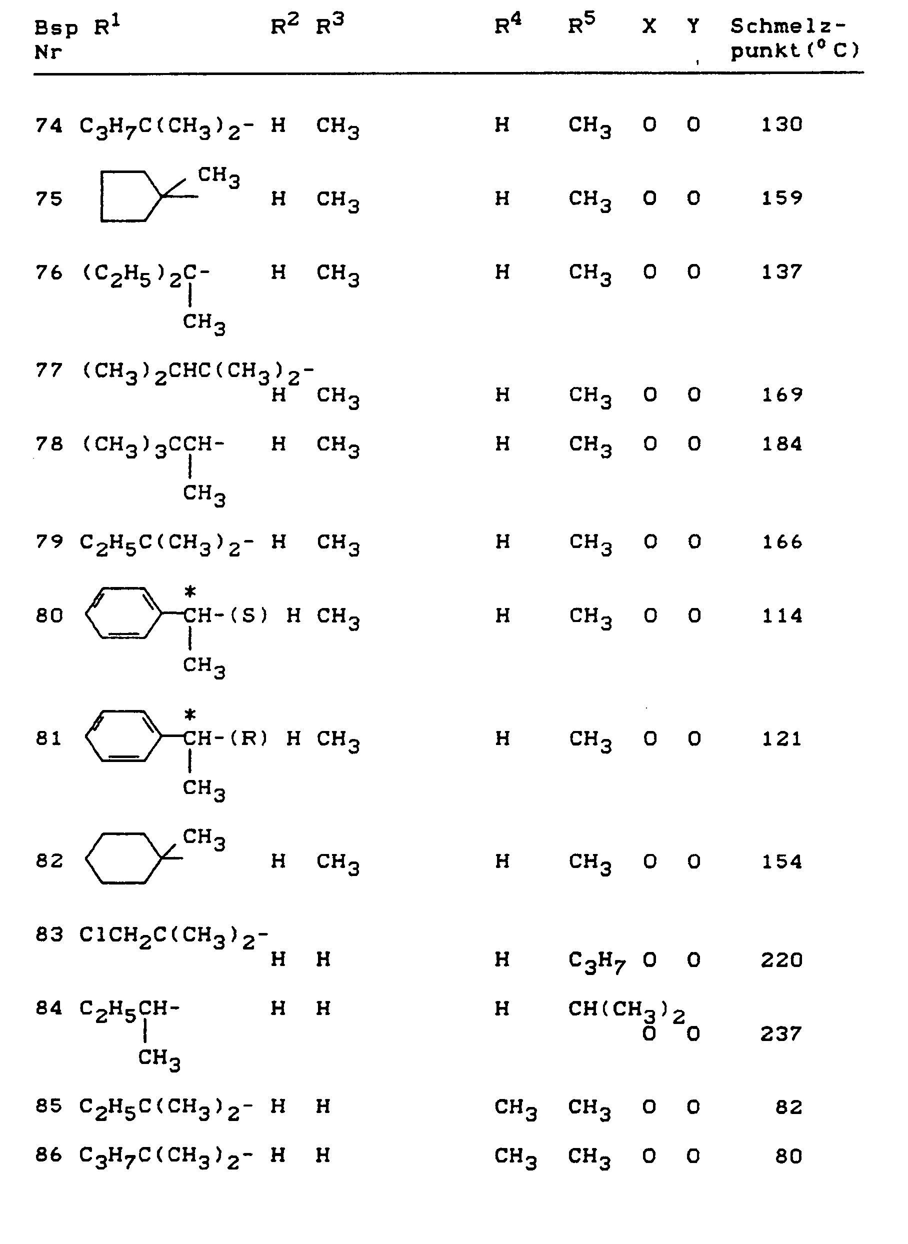

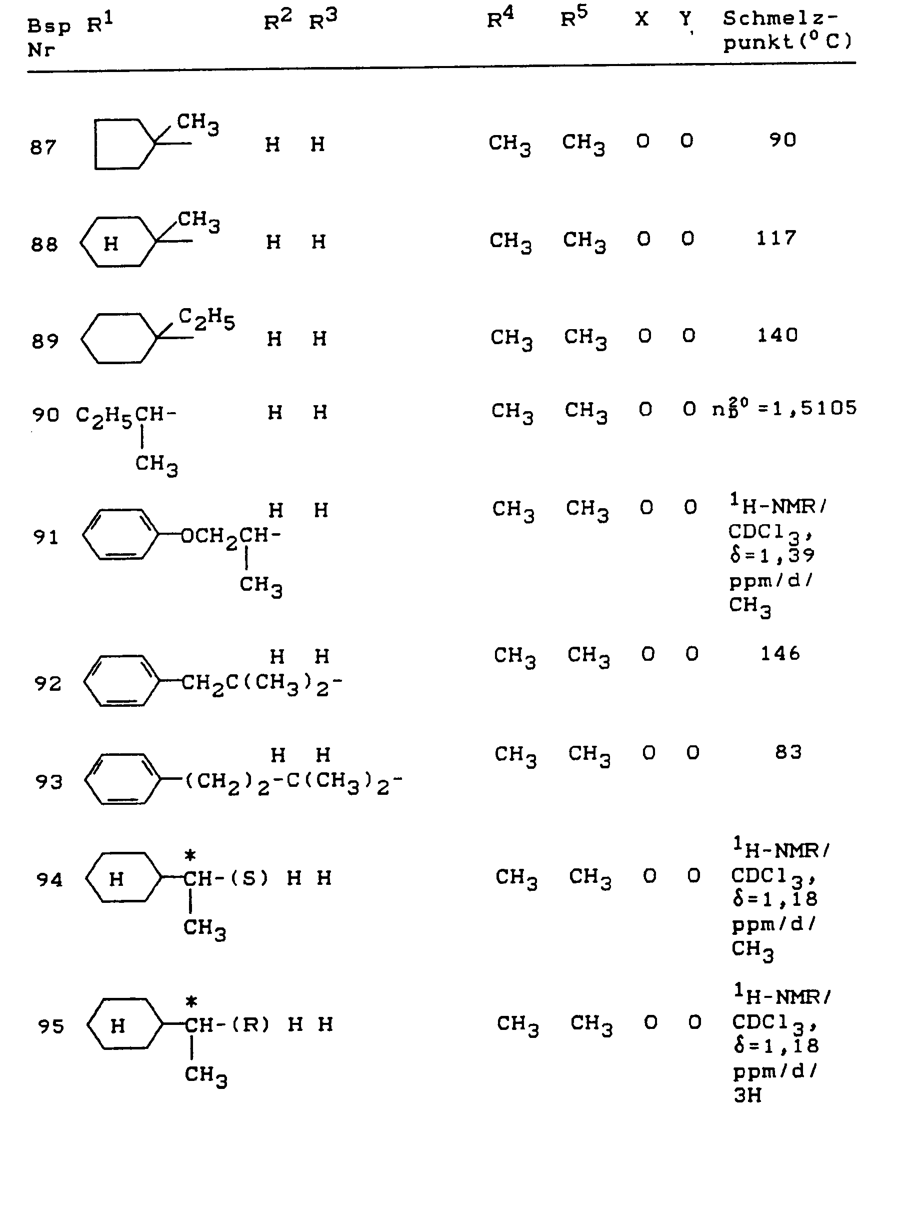

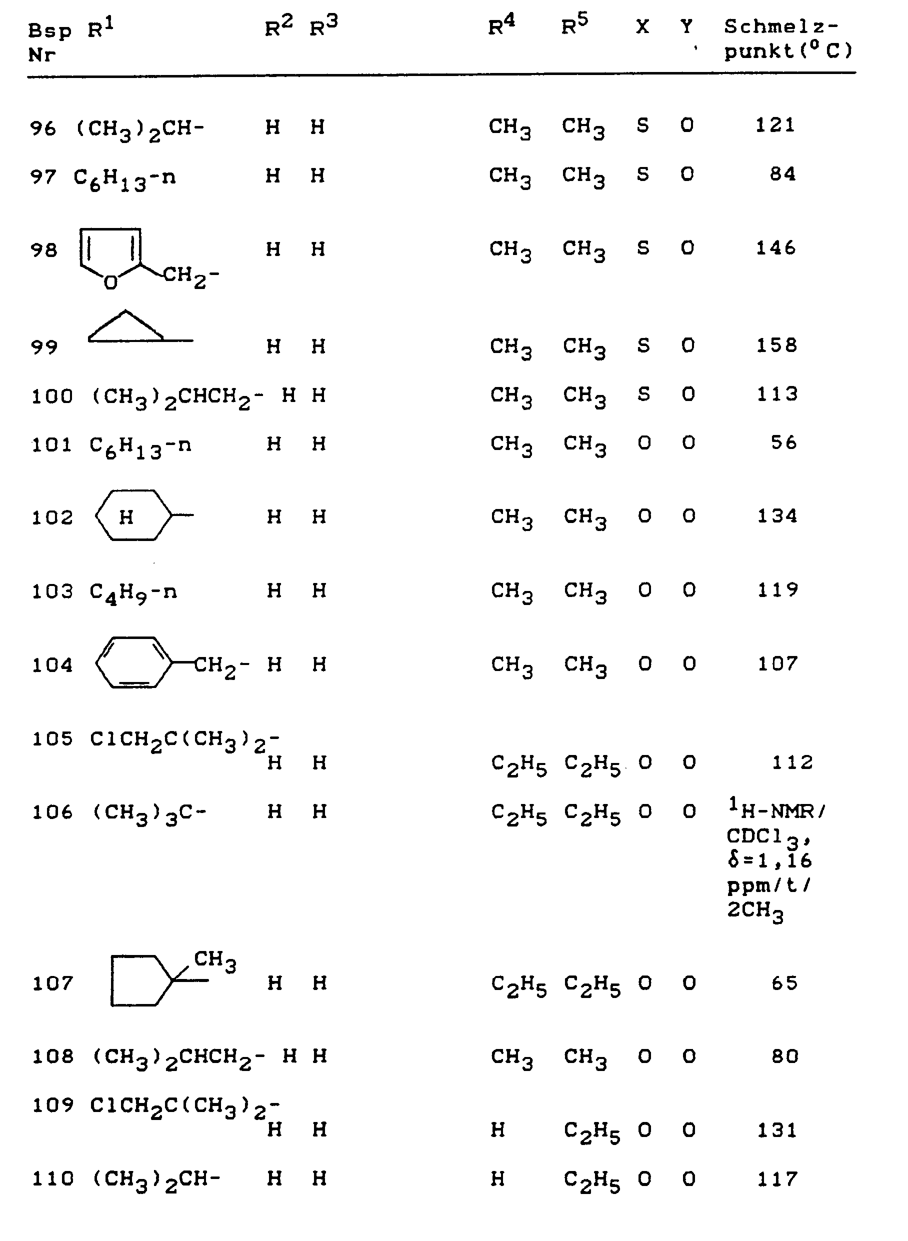

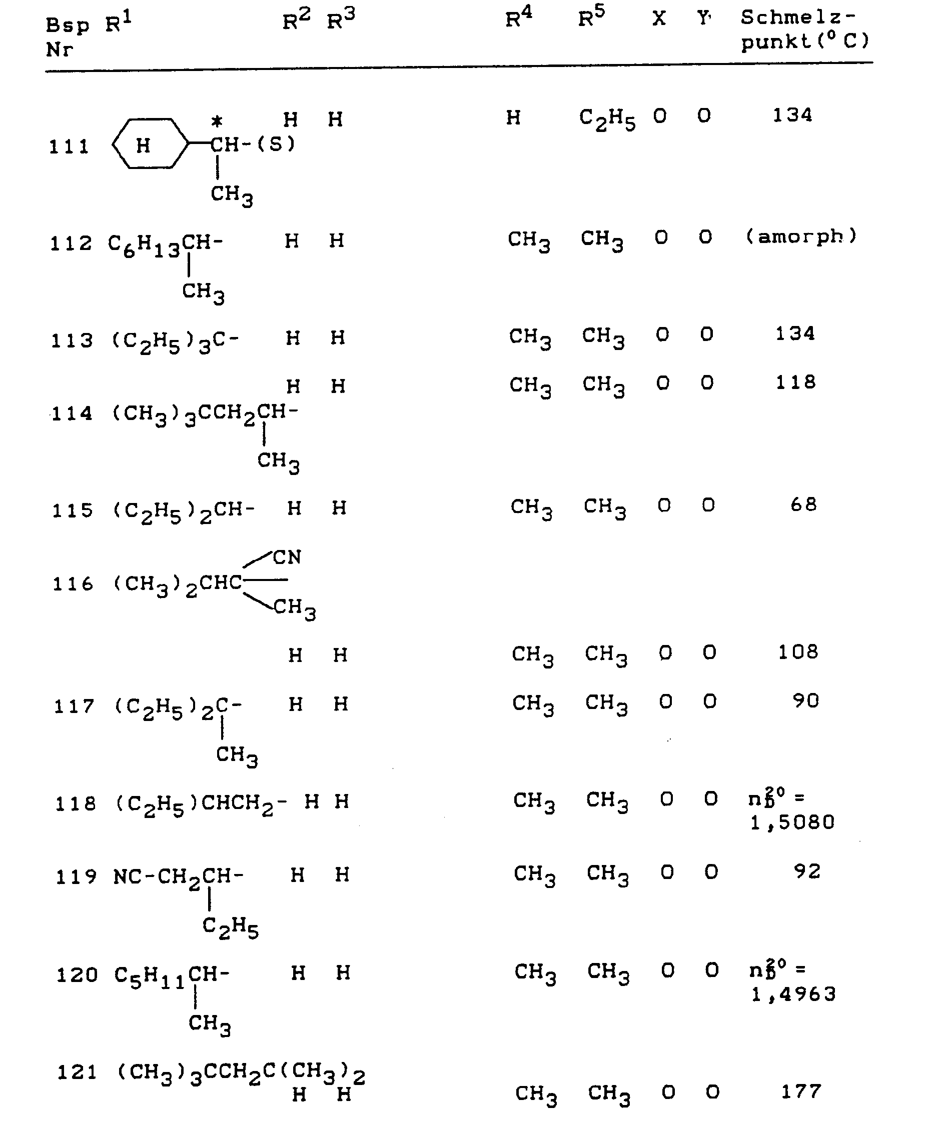

Die Herstellung der in Tabelle 2 als Beispiel Nr. 34 aufgeführten Verbindung ist im Folgenden ausführlich beschrieben:

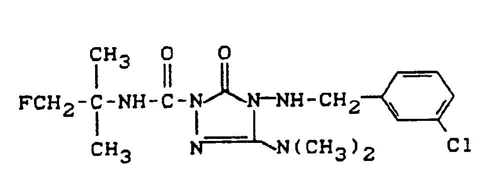

### (Verfahren (f))

2 g (32 mMol) Natriumcyanoborhydrid werden bei 0°C zu einer Mischung aus 9,6 g (25 mMol) 4-(3-Chlor- benzylidenamino)-5-dimethylamino-2-(1,1-dimethyl-2-fluor-ethylamino-carbonyl)-2,4-dihydro- 3H-1,2,4-triazol-3-on und 50 ml Methanol gegeben und die Mischung wird bis zum Umschlag eines Methylorange-Indikators mit gesättigter ethanolischer Hydrogenchlorid-Lösung versetzt. Im Verlauf von 6 Stunden werden weitere 2 g Natriumcyanoborhydrid portionsweise dazugegeben und das Gemisch wird dann noch 12 Stunden bei 20°C gerührt. Anschließend wird eingeengt, der Rückstand in Methylenchlorid aufgenommen, mit Wasser gewaschen, mit Natriumsulfat getrocknet und filtriert. Das Filtrat wird eingeengt, der Rückstand mit Petrolether zur Kristallisation gebracht und das Produkt durch Absaugen isoliert.

Man erhält 7,9 g (82% der Theorie) 4-(3-Chlor-benzylamino)-5-dimethylamino-2-(1,1-dimethyl-2-fluor- ethylamino-carbonyl)-2,4-dihydro- 3H-1,2,4-triazol-3-on vom Schmelzpunkt 158°C.

### Ausgangsstoffe der Formeln (ll)/(lla):

### Beispiel (11-1)

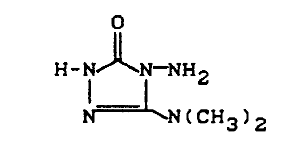

130 g (1.44 Mol) Carbodihydrazid werden in einer Mischung aus 470 g Phenol und 200 ml Chlorbenzol suspendiert, mit 153 g (1.44 Mol) Natriumcarbonat und 3.0 g (12 mMol) Dibutylzinnoxid versetzt und im Wasserstrahlvakuum auf 50°C bis 60°C erwärmt. Im Verlauf von 20 Minuten wird eine Lösung von 247 g (1.44 Mol) Tetramethylchlorformamidiniumchlorid in 400 g Phenol zugetropft, wobei Wasser abdestilliert. Dann wird bis zum Erreichen des Phenol-Siedepunktes Chlorbenzol im Wasserstrahlvakuum abdestilliert. Anschließend wird die Reaktionsmischung auf 160°C bis 180°C unter Normaldruck erhitzt, wobei Dimethylamin abgespalten wird. Nach 3 Stunden wird bei 190°C eine Stunde lang Phenol abdestilliert. Der verbleibende feste Rückstand wird im Soxleth-Extraktor mit 1.5 Liter Isopropanol extrahiert; die Isopropanol-Lösung wird eingedampft.

Man erhält 47 g (23% der Theorie) 4-Amino-5-dimethylamino-2,4-dihydro-3H-1,2,4-triazol-3-on als kristallinen Rückstand vom Schmelzpunkt 205°C.

### Beispiel (11-2)

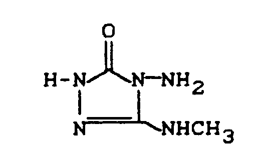

Eine Mischung aus 360 g (2.0 Mol) Carbodihydrazid, 212 g (2.0 Mol) Natriumcarbonat und 580 g Phenol wird auf 50°C bis 60°C erwärmt. Im Verlauf von 30 Minuten wird dann im Wasserstrahlvakuum eine Lösung von 314 g (2.0 Mol) Trimethyl-chlorformamidin-hydrochlorid in 314 g Phenol zugetropft. Dann wird das Reaktionsgemisch 60 Minuten bei 50°C bis 60°C gerührt und anschließend auf 190°C erwärmt, wobei Dimethylamin abgespaltet wird. Nach Ende der Gasentwicklung wird das Phenol im Wasserstrahlvakuum abdestilliert und der Rückstand aus Wasser umkristallisiert.

Man erhält 60 g (26% der Theorie) 4-Amino-5-methylamino-2,4-dihydro-3H-1,2,4-triazol-3-on vom Schmelzpunkt 220°C.

### Beispiel (11-3)

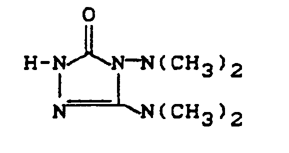

### Stufe 1:

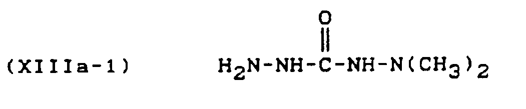

856 g (4.0 Mol) Kohlensäure-diphenylester werden unter Wasserkühlung vorgelegt und tropfenweise mit 245 g (4.0 Mol) N,N-Dimethylhydrazin versetzt. Dann wird langsam (innerhalb von 4 Stunden) auf 60°C erwärmt. Anschließend werden nach Abkühlen auf 20°C 200 g (4.0 Mol) Hydrazinhydrat dazu gegeben und die Reaktionsmischung wird 12 Stunden bei 20°C gerührt. Nach einstündigem Erwärmen auf 70°C bis 80°C werden flüchtige Komponenten im Wasserstrahlvakuum bis zu einer Sumpftemperaturvon 100°C abdestilliert. Der verbleibende Rückstand enthält im wesentlichen eine Lösung von 1,1-Dimethyl-carbodihydrazid in Phenol, welche direkt für die nächste Stufe eingesetzt wird.

### Stufe 2:

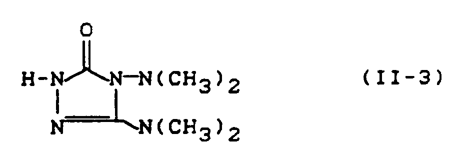

251 g der obigen Lösung aus 1,1-Dimethyl-carbodihydrazid in Phenol (ca 0.82 Mol) werden mit 100 g Phenol verdünnt, mit 87 g (0.82 Mol) Natriumcarbonat versetzt und auf 50°C bis 60°C erwärmt. Im Ölpumpenvakuum wird dann eine Lösung von 140 g (0.82 Mol) Tetramethyl-chlorformamidiniumchlorid in 250 g Phenol im Verlauf von 75 Minuten zugetropft. Das Reaktionsgemisch wird dann unter Normaldruck 6 Stunden auf Rückflußtemperatur erhitzt (ca. 190°C bis 195°C), wobei Dimethylamin abgespalten wird. Dann wird im Ölpumpenvakuum destilliert und das Destillat erneut destilliert. Das nun erhaltene Destillat (40 g) wird in Xylol aufgenommen und auf -78°C abgekühlt. Das hierbei kristallin angefallene Produkt wird durch Absaugen isoliert.

Man erhält 22.8 g (16% der Theorie) 4.5-Bis-dimethylamino-2,4-dihydro-3H-1,2,4-triazol-3-on vom Schmelzpunkt 93°C.

### Beispiel (11-4)

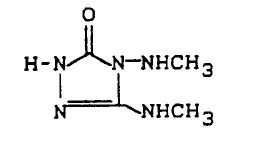

### Stufe 1:

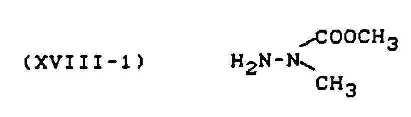

Eine Lösung von 94 g (2.0 Mol) Methylhydrazin in 100 ml Methanol wird auf 0°C bis -20°C abgekühlt und 268 g (2.0 Mol) Pyrokohlensäure-dimethylester werden kontinuierlich so zugetropft, daß die Temperatur von 0°C nicht überstiegen wird. Dann wird die Reaktionsmischung praktisch bis zum Ende der Gasentwicklung bei 80°C gerührt und anschließend im Wasserstrahlvakuum destilliert.

Man erhält 181 g (87% der Theorie) N-Methyl-N-methoxycarbonyl-hydrazin vom Siedepunkt 65°C/15 Torr.

### Stufe 2:

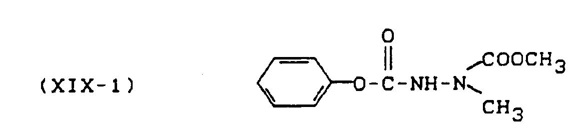

624 g (6.0 Mol) 1-Methyl-1-methoxycarbonyl-hydrazin und 334 g (3.15 Mol) Natriumcarbonat werden in 2 Liter Ethylenchlorid vorgelegt und unter Rühren werden 939 g (6.0 Mol) Chlorameisensäure-phenylester so zugetropft, daß die Temperatur von 20°C nicht überschritten wird. Dann wird das Gemisch noch 60 Minuten bei 60°C gerührt und das freigesetzte Natriumchlorid durch Absaugen abgetrennt. Vom Filtrat wird das Lösungsmittel im Wasserstrahlvakuum bei einer Sumpftemperatur von maximal 110°C abdestilliert.

Man erhält 1285 g (96% der Theorie) 1-Methyl-1-methoxycarbonyl-2-phenoxycarbonyl-hydrazin als öligen Rückstand, welcher allmählich kristallisiert.
Schmelzpunkt: 86°C.

### Stufe 3:

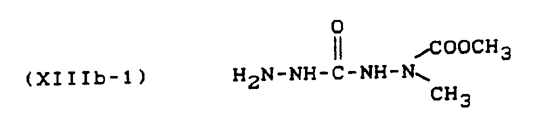

206 g (4.12 Mol) Hydrazinhydrat werden bei 20°C vorgelegt und eine auf 40°C erwärmte Lösung von 922 g (4.12 Mol) 1-Methyl-1-methoxycarbonyl-2-phenoxycarbonylhydrazin in 600 g Chlorbenzol wird in einem Guß dazu gegeben, worauf sich die Reaktionsmischung auf ca. 60°C erwärmt. Das Gemisch wird dann 4 Stunden bei 80°C gerührt und anschließend im Wasserstrahlvakuum bis zu einer Sumpftemperatur von ca. 100°C eingeengt. Der Rückstand, welcher im wesentlichen 1-Methyl-1-methoxycarbonyl-carbodihydrazid enthält wird direkt für die nächste Stufe eingesetzt.

### Stufe 4:

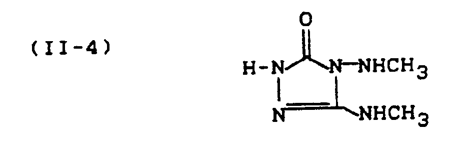

Eine Mischung aus 160 g (0.626 Mol) 1-Methyl-1-methoxycarbonyl-carbodihydrazid und 160 g Phenol wird auf40°C erwärmt und mit 142 g (0.626 Mol) Methyliminokohlensäure-diphenylester versetzt. Nach Abklingen der exothermen Reaktion wird das Gemisch unter Wasserstrahlvakuum langsam auf 150°C erhitzt, wobei ca. 150 g Phenol abdestillieren. Nach Erkalten wird der Rückstand mit 100 ml Wasser und 120 g 50%iger Natronlauge verrührt und 60 Minuten unter Rückfluß erhitzt. Dann werden 300 ml 18%ige Salzsäure dazu gegeben und der Ansatz wird eingedampft. Der Rückstand wird im Ölpumpenvakuum destilliert und das Rohdestillat erneut destilliert.

Man erhält 45 g (50% der Theorie) 4,5-Bis-methylamino-2,4-dihydro-3H-1,2,4-triazol-3-on als öliges Produkt, welches in der Vorlage wachartig erstarrt. Nach Verrühren mit Essigester werden 40 g (44%) weißkristallines Produkt vom Schmelzpunkt 135-137°C erhalten.

### Beispiel (11-5)

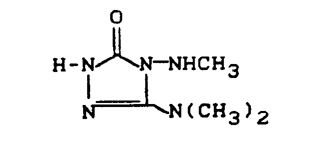

Wie unter Beispiel (11-4) - Stufe 3 - beschrieben, wird eine phenolische Lösung von 1-Methyl-1-methox- ycarbonylcarbodihydrazid erzeugt. 384 g dieser Lösung (1.5 Mol) werden in 200 ml Chlorbenzol aufgenommen, mit 239 g (2.25 Mol) Natriumcarbonat versetzt und im Wasserstrahlvakuum auf 50°C erwärmt. Dabei wird eine Lösung von 244 g (1.5 Mol) Dichlormethylen-dimethylimmoniumchlorid in 566 g Phenol zugetropft, wobei Wasser azeotrop abdestilliert. Anschließend wird das Reaktionsgemisch noch 60 Minuten bei 120°C unter Normaldruck gerührt und dann heiß filtriert und mit Ethanol und Aceton gewaschen. Die organische Lösung wird eingengt, der Rückstand mit 240 ml 50%iger Natronlauge unter Rückfluß erhitzt und nach Erkalten mit konz. Salzsäure neutralisiert. Nach Einengen im Wasserstrahlvakuum wird der Rückstand im Ölpumpenvakuum destilliert und das Rohdestillat erneut destilliert.

Man erhält 72 g öliges Produkt (Siedepunkt 165°C/1 mbar), das mit 300 ml Toluol in ein reines kristallines Produkt überführt wird.

Ausbeute: 60.0 g (25.5% der Theorie) 4-Methylamino-5-dimethylamino-2,4-dihydro-3H-1,2,4-triazol-3-on; Schmelzpunkt: 129°C.

### Beispiel (11-6)

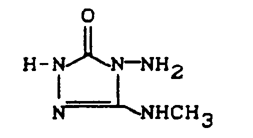

Eine Mischung aus 58 g (0.25 Mol) 1,3-Diamino-2-methylguanidin-hydroiodid, 53.5 g (0.25 Mol) Kohlensäure-diphenylester und 20 g Phenol wird unter Rühren auf 160°C erhitzt, bis die Mischung nahezu homogen geworden ist. Dann werden 34.5 g (0.25 Mol) Kaliumcarbonat portionsweise dazu gegeben, wobei Kohlendioxid freigesetzt wird. Nach 60 Minuten bei 160°C wird etwas abgekühlt und das Phenol im Wasserstrahlvakuum abdestilliert. Der Rückstand wird in 200 ml Wasser aufgenommen, mit Salzsäure neutralisiert und langsam mit Ethanol versetzt, wobei das Reaktionsgemisch kristallin anfällt.

Man erhält 24 g (74% der Theorie) 4-Amino-5-methylamino-2,4-dihydro-3H-1,2,4-triazol-3-on vom Schmelzpunkt 214°C.

Analog zu den Beispielen (11-1) bis (11-6) und entsprechend der allgemeinen Beschreibung dererfindungsgemäßen Herstellungsverfahren können auch die in der nachstehenden Tabelle 3 aufgeführten Verbindungen der Formel (11) hergestellt werden:

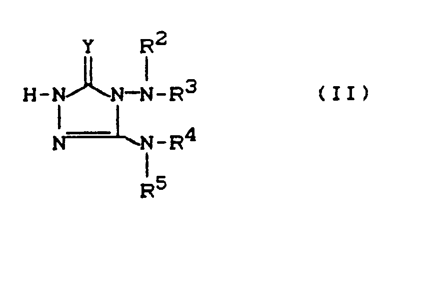

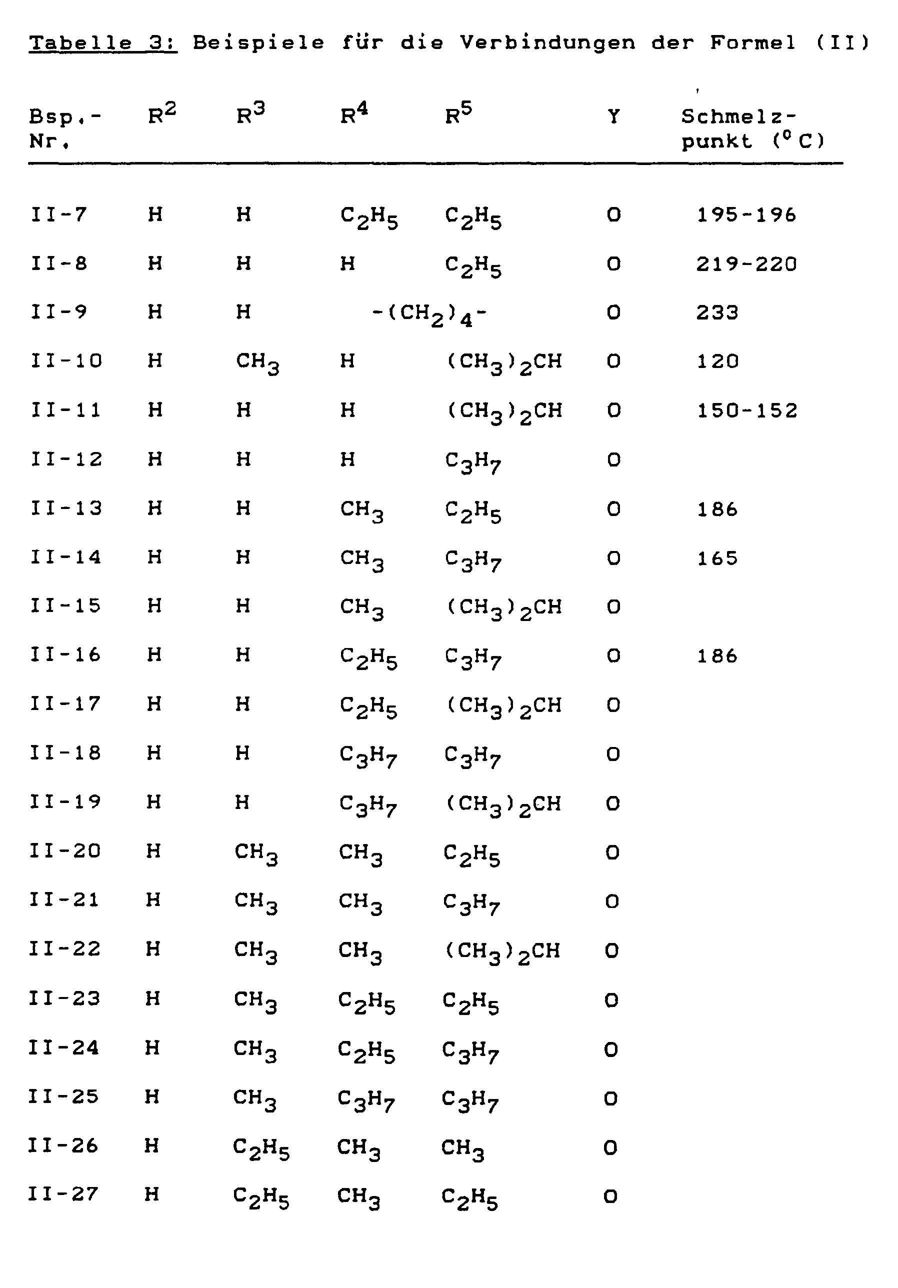

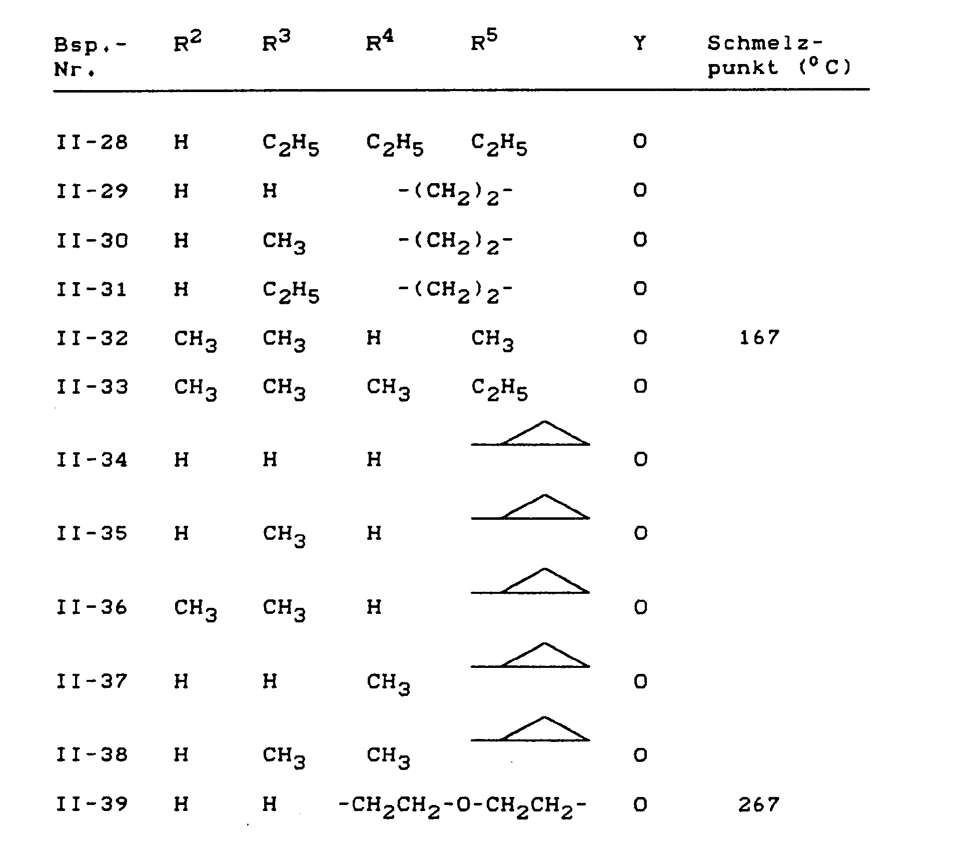

Die in Tabelle 3 als Beispiel (11-11) aufgeführte Verbindung kann beispielsweise wie folgt hergestellt werden:

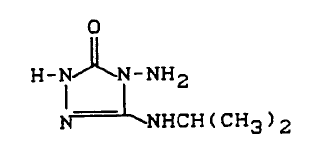

Eine Mischung aus 448 g (2 Mol) 1-Ethoxycarbonyl-2-phenoxycarbonyl-hydrazin (vgl.EP-A 65 366), 350 g Phenol und 100 ml 1,2-Dichlorethan wird bei 20°C vorgelegt und unter Wasserkühlung werden 100 g (2 Mol) Hydrazinhydrat in einem Guß dazugegeben. Das Gemisch wird 15 Stunden bei 40°C gerührt, dann innerhalb von 6 Stunden auf 100°C aufgeheizt. Dann wird im Wasserstrahlvakuum Wasser abdestilliert (Sumpftemperatur maximal 100°C) und das Gemisch anschließend wieder auf 20°C abgekühlt. Nun wird eine Lösung von 510 g (2 Mol) N-Isopropyl-iminokohlendiphenylester in 500 ml Dichlormethan in einem Guß dazugegeben.

Das Gemisch wird eine Stunde bei 20°C, eine weitere Stunde bei 50°C und schließlich 30 Minuten bei 95°C gerührt; dann wird innerhalb etwa einer Stunde unter Abdestillieren von 1,2-Dichlorethan auf 180° erhitzt. Anschließend wird - zunächst im Wasserstrahlvakuum und dann im Ölpumpenvakuum - Phenol (insgesamt 834 g) abdestilliert. Der Rückstand wird mit 150 ml Wasser und 640 g (4 Mol NaOH) 25%iger Natronlauge versetzt und 60 Minuten unter Rückfluß erhitzt; dann wird bei Raumtemperatur mit 811 g (4 Mol HCI) 18%iger Salzsäure neutralisiert und eingedampft. Der nun praktisch wasserfreie Rückstand wird mit 600 ml Dimethylformamid heiß extrahiert und durch Absaugen von Natriumchlorid befreit. Das Filtrat wird eingeengt und der Rückstand in 250 ml Wasser aufgenommen, mit etwa dem gleichen Volumen Essigsäureethylester geschüttelt, die organische Phase abgetrennt, über Magnesiumsulfat getrocknet und filtriert. Vom Filtrat wird das Lösungsmittel im Wasserstrahlvakuum sorgfältig abdestilliert. Man erhält 34 g (11 % der Theorie) 4-Amino-5-isopropylamino-2,4-dihydro-3H-1,2,4-triazol-3-on vom Schmelzpunkt 150°C-152°C.

Die unter Beispiel (11-4) beschriebene Verbindung kann beispielsweise auch wie folgt hergestellt werden:

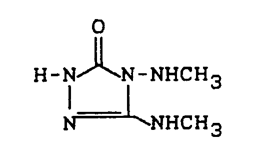

256 g einer phenolischen Lösung von (1 Mol) 1-Methyl-1-methoxycarbonyl-carbodihydrazid - Herstellung vgl. Beispiel (11-4), Stufe 3 - werden mit zusätzlich 150 g Phenol bei 40°C vorgelegt und mit 135 g (1 Mol) N-Methyliminodithiokohlensäure-S,S-dimethylester versetzt. Nun wird im Wasserstrahlvakuum auf 60°C erwärmt, wobei die Umsetzung unter Abspaltung von Methylmercaptan eintritt. Im Verlauf einer Stunde wird dann auf 80°C erwärmt. Dann wird bis maximal 160°C - zuletzt im Ölpumpenvakuum - Phenol abdestilliert. Nach Zugabe von 50 ml Wasser und 160 g (2 Mol NaOH) 50%iger Natronlauge wird eine Stunde unter Rückfluß erhitzt.

Zu der wieder abgekühlten Mischung werden 406 g (2 Mol HCI) 18%ige Salzsäure gegeben und das Wasser wird dann am Rotationsverdampfer abdestilliert. Das durch Destillation des Rückstandes im Ölpumpenvakuum erhaltene Produkt wird mit Essigsäureethylester verrührt und abgesaugt.

Man erhält 60 g (42% der Theorie) 4,5-Bis-methyl-amino-2,4-dihydro-3H-1,2,4-triazol-3-on vom Schmelzpunkt 135°C-137°C.

Die unter Beispiel (11-1) beschriebene Verbindung kann beispielsweise auch nach den folgenden Varianten erhalten werden:

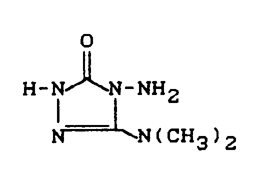

700 g (4,3 Mol) Dichlormethylen-dimethylimmoniumchlorid werden in 3 Liter Methylenchlorid bei ca. 40°C vorgelegt und im Verlauf von 90 Minuten mit 808 g (8,6 Mol) Phenol tropfenweise versetzt. Danach werden weitere 540 g Phenol dazugegeben und das Methylenchlorid wird im Wasserstrahlvakuum abdestilliert. Es verbleibt eine phenolische Lösung von Diphenoxymethylen-dimethylimmoniumchlorid, welche direkt in die nächste Stufe eingesetzt wird.

Eine Lösung von 387 g (4,3 Mol) Carbodihydrazid in 1300 Phenol wird zusammen mit 274 g (2,58 Mol) Natriumcarbonat vorgelegt und die wie oben beschrieben erhaltene Lösung von Diphenoxymethylen-dimethylimmoniumchlorid (1193 g) in 540 g Phenol so zugetropft, daß eine Innentemperatur von 40°C nicht überschritten wird. Die Umsetzung erfolgt im Verlauf von 90 Minuten, wobei im Ölpumpenvakuum Wasser abdestilliert wird. Danach wird weiter im Ölpumpenvakuum auf 130°C langsam aufgeheizt, wobei schließlich das Phenol weitgehend abdestilliert wird. Der Rückstand wird mit 2,4 Liter Ethoxyethanol (heiß) behandelt, das ungelöste Natriumchlorid in der Hitze durch Absaugen abgetrennt und das Filtrat abgekühlt. Das hierbei kristallin angefallene Produkt wird durch Absaugen isoliert.

Man erhält 87 g (14% der Theorie) 4-Amino-5-dimethylamino-2,4-dihydro-3H-1,2,4-triazol-3-on vom Schmelzpunkt 205°C.

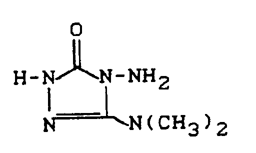

Eine Mischung aus 104 g (1 Mol) Hydrazinoameisensäureethylester, 200 g Phenol, 50 ml Dimethylformamid und 106 g (1 Mol) Natriumcarbonat wird im Wasserstrahlvakuum vorgelegt und eine Lösung von 171 g (1 Mol) Tetramethylchlorformamidiniumchlorid in 200 g Phenol so zugetropft, daß eine Temperatur von 40°C nicht überschritten wird, wobei allmählich Wasser abdestilliert wird. Dann wird langsam aufgeheizt, bei 80°C 55 g (1,1 Mol) Hydrazinhydratdazugegeben und die Mischung unter atmosphärischem Druck im Verlaufvon 3 Stunden bis zum Phenol-Rückfluß (ca. 190°C) erhitzt, wobei Dimethylamin abgespalten wird. Das Phenol wird dann im Wasserstrahlvakuum abdestilliert, der Rückstand mit 750 ml Ethoxyethanol (heiß) behandelt, das Salz durch Filtration in der Hitze abgetrennt und das Filtrat abgekühlt. Das hierbei kristallin angefallene Produkt wird durch Absaugen isoliert.

Man erhält 49 g (34% der Theorie) 4-Amino-5-dimethylamino-2,4-dihydro-3H-1,2,4-triazol-3-on vom Schmelzpunkt 205°C.

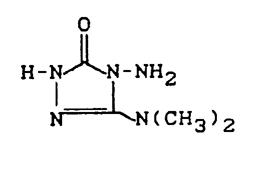

88 g (1 Mol) Ethylenglycolcarbonat werden in 300 ml Ethylenglycol vorgelegt und mit 50 g (1 Mol) Hydrazinhydrat tropfenweise versetzt. Nach Abklingen der exothermen Reaktion wird im Wasserstrahlvakuum Wasserabdestilliert. Dann werden 106 g (1 Mol) Natriumcarbonat dazugegeben und im Ölpumpenvakuum bei 20°C bis 30°C wird im Verlauf einer Stunde eine Lösung von 171 g (1 Mol) Tetramethylchlorformamidiniumchlorid in 300 ml Ethylenglykol zugetropft, wobei Wasser abdestilliert wird. Danach wird innerhalb von 90 Minuten auf 80°C erwärmt und nach Zugabe von 55 g (1,1 Mol) Hydrazinhydrat im Verlauf von 3 Stunden auf 180°C aufgeheizt, wobei Dimethylamin abgespalten wird. Anschließend wird das Ethylenglycol im Wasserstrahlvakuum abdestilliert, der Rückstand mit heißem Dimethylformamid (300 ml) behandelt, vom Salz abfiltriert und das Filtrat eingedampft. Der Rückstand wird aus 240 ml Wasser/lsopropanol (Vol. 1:2) umkristallisiert.

Man erhält 25 g (18% der Theorie) 4-Amino-5-dimethylamino-2,4-dihydro-3H-1,2,4-triazol-3-on vom Schmelzpunkt 205°C.

Die unter Beispiel (11-5) beschriebene Verbindung kann beispielsweise auch wie folgt hergestellt werden:

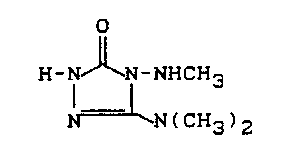

Wie unter Beispiel (11-4) - Stufe 3 - beschrieben, wird eine phenolische Lösung von 1-Methyl-1-methoxycarbonyl-carbodihydrazid erzeugt. 128 g (0,5 Mol) dieser Lösung, werden mit 75 g Phenol, 150 g Chlorbenzol und 52 g (0,5 Mol) Natriumcarbonat vermischt und bei 50°C bis 60°C im Wasserstrahlvakuum werden 85,8 g (0,5 Mol) Tetramethylchlorformamidiniumchlorid (gelöst in 150 g Phenol) im Verlauf einer Stunde zugetropft, wobei Wasser abdestilliert wird.

Die Mischung wird zunächst noch im Wasserstrahlvakuum auf 80°C erwärmt und dann noch eine Stunde unter atmosphärischem Druck bei 120°C gerührt. Anschließend wird im Verlauf von 2 Stunden auf 185°C aufgeheizt, wobei Dimethylamin abgespalten wird. Danach wird das Phenol, zunächst im Wasserstrahlvakuum und dann im Ölpumpenvakuum, abdestilliert und der Rückstand wird mit 200 ml Wasser und 96 g (1,2 Mol Na-OH) 50%iger Natronlauge eine Stunde unter Rückfluß erhitzt. Nach Zugabe vom 243 g (1,2 Mol HCI) 18%iger Salzsäure wird eingedampft, der Rückstand mit 400 ml heißem Chlorbenzol behandelt, das Salz durch Filtration abgetrennt und das Filtrat durch Destillation aufgearbeitet. Die hochsiedende Fraktion (165°C/1 mbar) wird mit 100 ml Toluol zur Kistallisation gebracht. Man erhält 21 g (27% der Theorie) 4-Methylamino-5-dimethylamino-2,4-dihydro-3H-1,2,4-triazol-3-on vom Schmelzpunkt 129°C.

### Ausgangsstoffe der Formel (IV):

### Beispiel (IV-1):

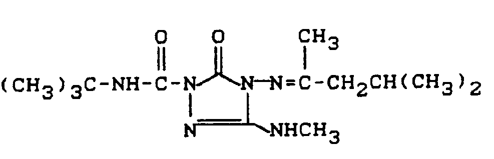

3.2 g (15 mMol) 4-(4-Methyl-2-pentyliden-amino)-5-methylamino-2,4-dihydro-3H-1,2,4-triazol-3-on werden in 80 ml Acetonitril gelöst und nacheinander mit 100 mg Diazabicycloundecen (DBU) und 1.5 g (15 mMol) tert-Butylisocyanatversetzt. Das Reaktionsgemisch wird 2 Tage bei 20°C gerührt und anschließend eingeengt. Der Rückstand wird in Methylenchlorid aufgenommen, mit Wasser gewaschen, mit Natriumsulfat getrocknet und filtriert. Vom Filtrat wird das Lösungsmittel im Wasserstrahlvakuum sorgfältig abdestilliert.

Man erhält 4.0 g (86% der Theorie) 2-tert-Butylaminocarbonyl-4-(4-methyl-2-pentylidenamino)-5-methylamino-2,4-dihydro- 3H-1,2,4-triazol-3-on als öligen Rückstand.

1 H-NMR (CDCI₃, 8, ppm): 1.00 (d, CH₃), 1.43 (s, CH₃), 2.35 (d, CH₂), 2.96 (d, NHCH₃), 4.35 (q, NHCH₃), 7.65 (s, NH).

Analog zu Beispiel (IV-1) und entsprechend der allgemeinen Beschreibung der erfindungsgemäßen Herstellungs verfahren können auch die in der nachstehenden Tabelle 4 aufgeführten Verbindungen der Formel (IV) hergestellt werden.

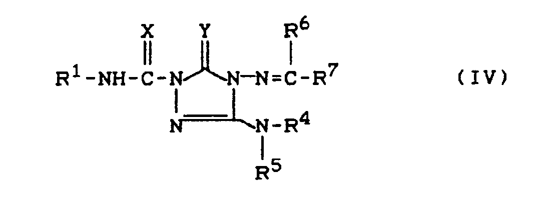

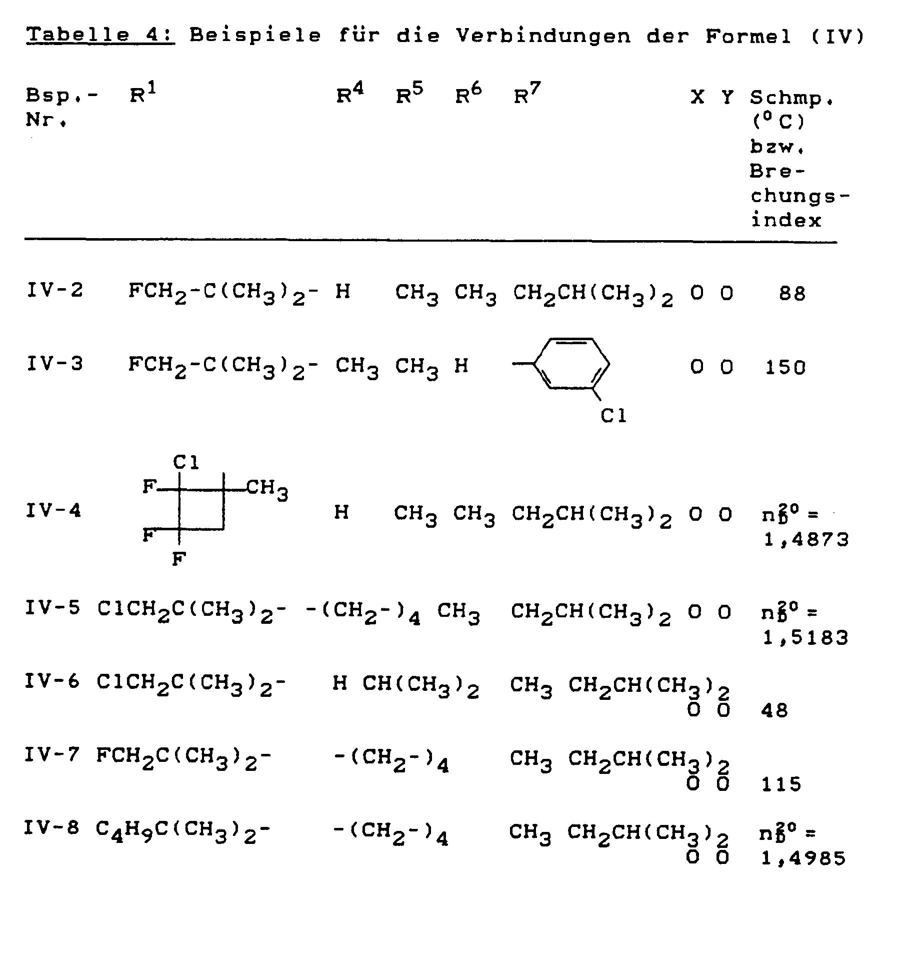

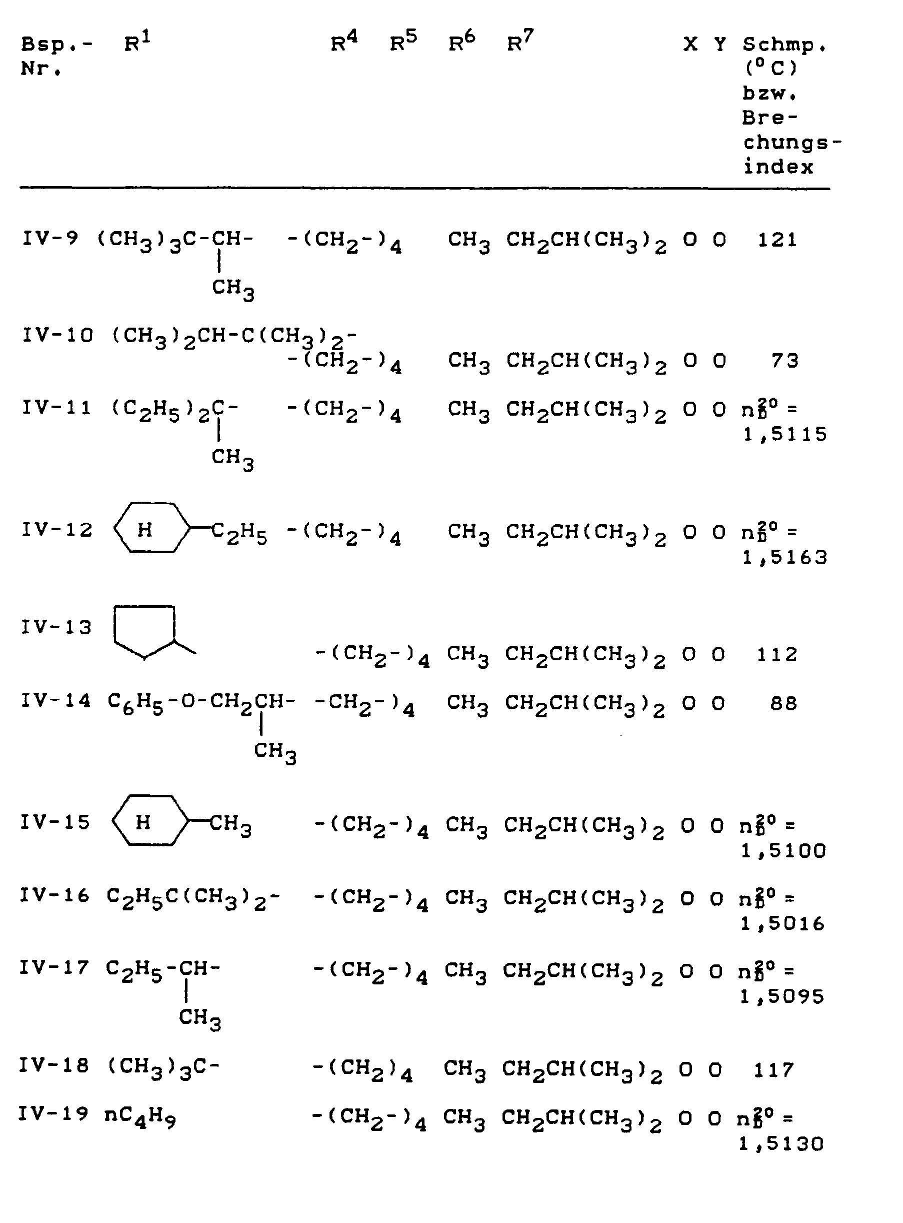

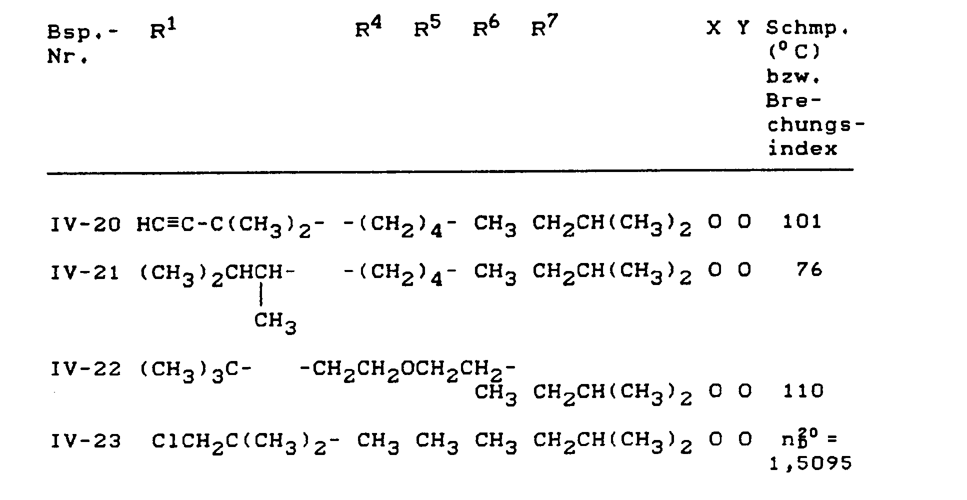

### Ausgangsstoffe der Formel (VIII):

### Beispiel (VIII-1):

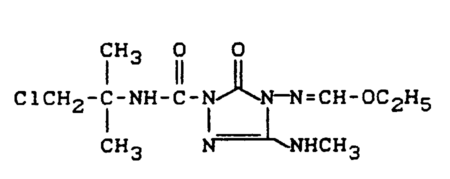

3,7g g (0.02 Mol) 4-Ethoxymethylenamino-5-methylamino-2,4-dihydro-3H-1,2,4-triazol-3-on werden in 100 ml Acetonitril gelöst und nacheinander mit 100 mg Diazabicycloundecen (DBU) und 2.8 g (0.02 Mol) Chlor-tert-butylisocyanat versetzt. Das Gemisch wird 12 Stunden bei 20°C gerührt und dann eingeengt. Der Rückstand wird in Methylenchlorid aufgenommen, mit Wasser gewaschen, mit Natriumsulfat getrocknet und filtriert. Vom Filtrat wird das Lösungsmittel im Wasserstrahlvakuum abdestilliert und der Rückstand durch Verreiben mit Diethylether zur Kristallisation gebracht.

Man erhält 5.2 g (82% der Theorie) 2-Chlor-tert-butylaminocarbonyl-4-ethoxymethylenamino-5-methylamino-2,4-dihydro- 3H-1,2,4-triazol-3-on vom Schmelzpunkt 142°C.

Analog zu Beispiel VIII-1 und entsprechend der allgemeinen Beschreibung der erfindungsgemäßen Herstellungsverfahren können auch die in der nachstehenden Tabelle 5 aufgeführten Verbindungen der Formel (VIII) hergestellt werden.

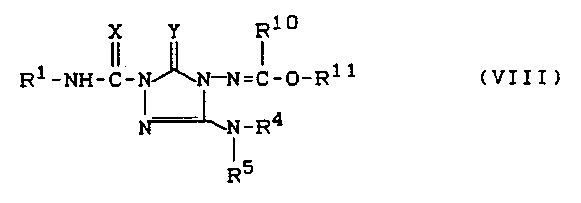

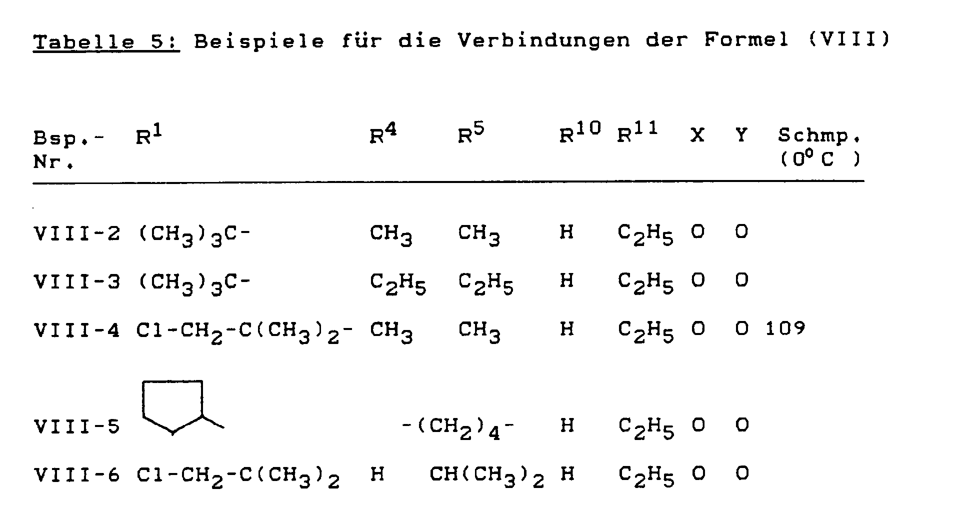

### Zwischenprodukte der Formel (XXI):

### Beispiel (XXI-1)

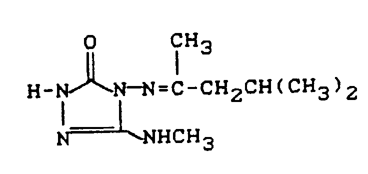

12.9 g (0.1 Mol) 4-Amino-5-methylamino-2,4-dihydro-3H-1,2,4-triazol-3-on werden mit 150 ml Methyl-isobutylketon und 100 mg p-Toluolsulfonsäure unter Rückfluß am Wasserabscheider erhitzt, bis praktisch kein Wasser mehr abgeschieden wird (ca. 2 Stunden). Man filtriert, engt das Filtrat ein und verreibt den Rückstand mit Petrolether.

Man erhält 8.3 g (39% der Theorie) 4-(4-Methyl-2-pentyliden-amino) 5-methylamino-2,4-dihydro-3H-1,2,4-triazol-3-on vom Schmelzpunkt 136°C.

Analog zu dem Beispiel (XXI-1) und entsprechend der allgemeinen Beschreibung der erfindungsgemäßen Herstellungsverfahren können auch die in der nachstehenden Tabelle 6 aufgeführten Verbindungen der Formel (XXI) hergestellt werden:

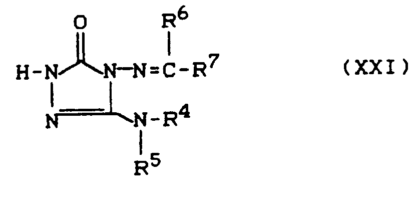

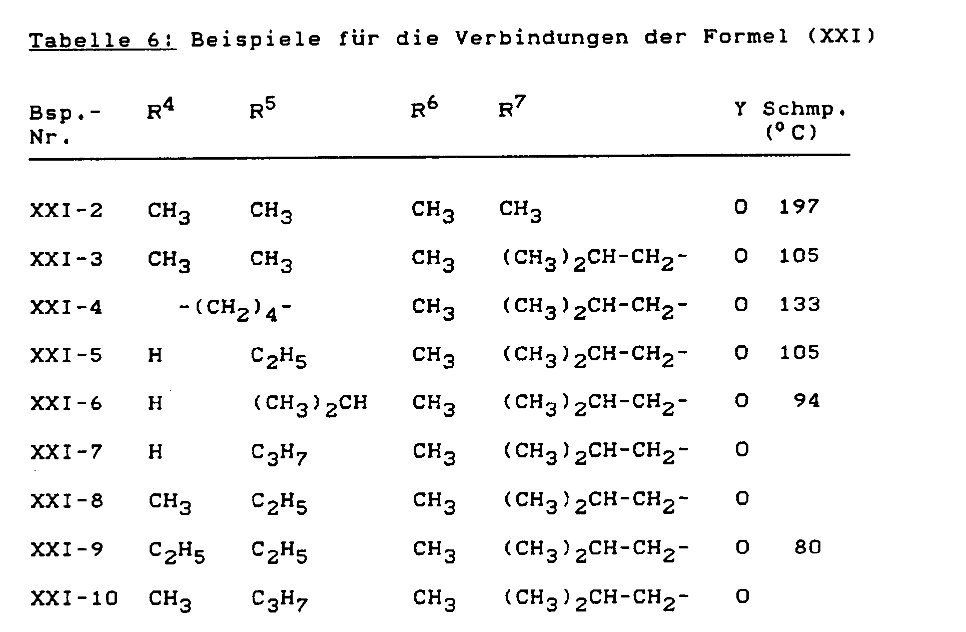

### Zwischenprodukte der Formel (XXV):

### Beispiel (XXV-1)

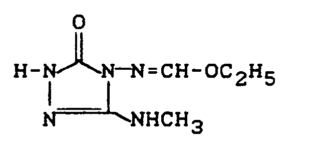

12.9 g (0.1 Mol) 4-Amino-5-methylamino-2,4-dihydro-3H-1,2,4-triazol-3-on werden mit 100ml Orthoameisensäuretriethylester und 100 mg p-Toluolsulfonsäure 4 Stunden unter Rückfluß erhitzt. Dann wird eingeengt und mit Diethylether zur Kristallisation gebracht. Nach Umkristallisation aus Ethanol erhält man 10.0 g (54% der Theorie) 4-Ethoxymethylenamino-5-methylamino-2,4-dihydro-3H-1,2,4-triazol-3-on vom Schmelzpunkt 169°C.

Analog zu Beispiel (XXV-1) und entsprechend derallgemeinen Beschreibung dererfindungsgemäßen Herstellungsverfahren können auch die in der nachstehenden Tabelle 7 aufgeführten Verbindungen der Formel (XXV) hergestellt werden:

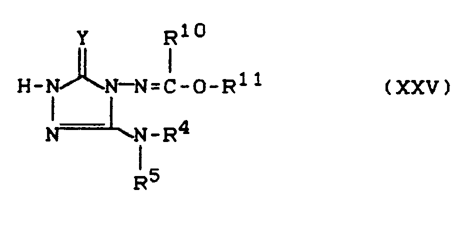

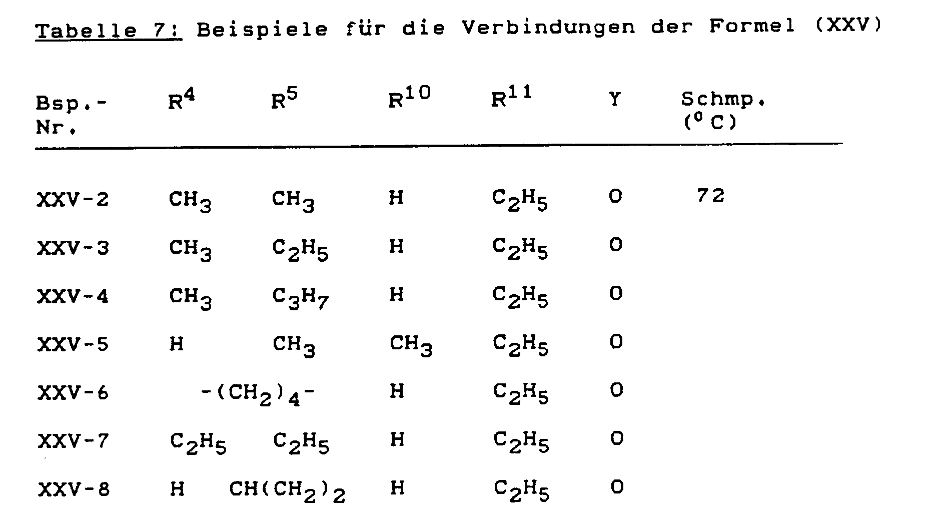

### Anwendungsbeispiele:

In den folgenden Anwendungsbeispielen wurde die nachstehend aufgeführte Verbindung als Vergleichssubstanz eingesetzt: 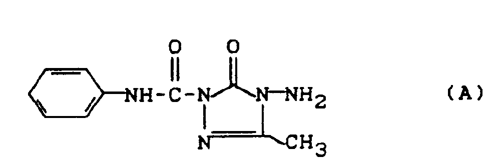 4-Amino-5-methyl-2-phenylaminocarbonyl-2,4-dihydro-3H-1,2,4-triazol-3-on
(bekannt aus EP-A 294666, Beispiel 122).

### Beispiel A

### Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in derZubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrollen Es bedeuten:
0 % = keine Wirkung (wie unbehandelte Kontrolle)
100% = totale Vernichtung

Eine deutliche Überlegenheit in der Wirksamkeit gegenüber der Vergleichsverbindung (A) zeigen in diesem Test z.B. die Verbindungen gemäß den Herstellungsbeispielen 16, 17, 18 und 19.

### Beispiel B

### Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 I Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100% = totale Vernichtung

Eine deutliche Überlegenheit in der Wirksamkeit gegenüber der Vergleichsverbindung (A) zeigen in diesem Test z.B. die Verbindungen gemäß den Herstellungsbeispielen 16, 17, 18 und 19.

## Patentansprüche

1. Substituierte 4,5-Diamino-1,2,4-Triazol-3-(thi)one der allgemeinen Formel (I) 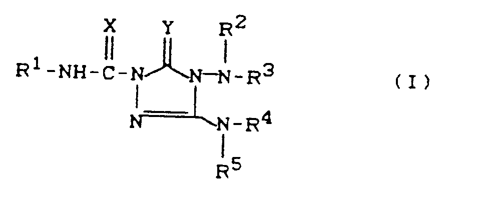 in welcher
R¹ für Wasserstoff, für jeweils geradkettiges oderverzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, für Phenoxyalkyl mit 1 bis 4 Kohlenstoffatomen im Alkylteil, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für jeweils gegebenenfalls einfach bis dreifach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettige: oder verzweigtes Alkyl oder Halogenalkyl mit jeweils 1 bis 4 Kohlenstoffatomen und gegebenenfalls 1 bis 9 gleichen oder verschiedenene Halogenatomen und geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; R¹ außerdem für im Heterocyclylteil gegebenenfalls einfach bis dreifach, gleich oder verschieden substituiertes Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen -insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; R¹ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen oder für jeweils gegebenenfalls einfach bis dreifach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im geradkettigen oder verzweigten Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oderverzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder R¹ für Benzyl mit im Phenylteil ankondensierter -O-CH₂-0- Gruppe steht,
R², R³, R⁴ und R⁵ gleich oder verschieden sind und unabhängig voneinander für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanoalkyl mit 1 bis 8 Kohlenstoffatomen, Alkoxyalkyl oder Alkylthioalkyl mit jeweils bis zu 4 Kohlenstoffatomen in den einzelnen Alkylteilen, für jeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen, die Reste R² bis R⁵ weiter für jeweils gegebenenfalls einfach bis dreifach gleich oder verschieden substituiertes Aryl oder Aralkyl stehen mit jeweils 6 oder 10 Kohlenstoffatomen im Arylteil und gegebenenfalls 1 bis 4 Kohlenstoffatomen im geradkettigen oder verzweigten Alkylteil, wobei als Substituenten infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 Halogenatomen, in welcher weiter auch jeweils zwei dieser Reste - R² und R³ oder R⁴ und R^{5 -} zusammen für geradkettiges oder verzweigtes Alkandiyl oder Oxaalkandiyl mit jeweils 2 bis 6 Kohlenstoffatomen stehen können, und R⁵ auch für geradkettiges oder verzweigtes Alkoxy mit 1 bis 8 Kohlenstoffatomen stehen kann,
X für Sauerstoff oder Schwefel steht und
Y für Sauerstoff oder Schwefel steht.

2. Substituierte 4,5-Diamino-1,2,4-triazol-3-(thi)one der Formel (I) gemäß Anspruch 1, in welcher
R¹ für Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl, Dodecyl, für Allyl, jeweils geradkettiges oder verzweigtes Butenyl, Pentenyl oder Hexenyl, Propargyl, jeweils geradkettiges oder verzweigtes Butinyl, Pentinyl oder Hexinyl, für geradkettiges oder verzweigtes Halogenalkyl mit 1 bis 8 Kohlenstoffatomen und 1 bis 9 gleichen oder verschiedenen Halogenatomen, für jeweils geradkettiges oder verzweigtes Halogenalkenyl oder Halogenalkinyl mit jeweils 3 bis 8 Kohlenstoffatomen und 1 bis 3 Halogenatomen, für jeweils geradkettiges oder verzweigte: Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oderAlkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom, Methyl, Ethyl, n- oder i-Propyl, n-, i-, s- oder t-Butyl, Cyano, Methandiyl, Ethandiyl, Butandiyl oder Butadiendiyl oder Dichlorallyl;
R¹ weiterhin für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen: 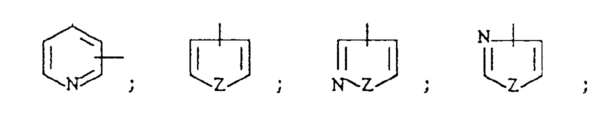 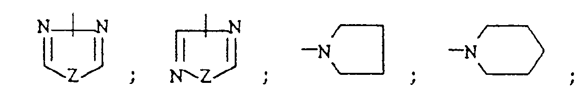 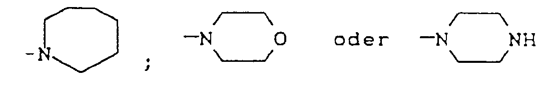 wobei Z jeweils für Sauerstoff oder Schwefel steht und wobei als Substituenten jeweils infrage kommen; Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
R¹ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenyl-, butyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl steht, wobei als Phenylsubstituenten jeweils infrage kommen: Fluor, Chlor, Brom, Hydroxy, Cyano, Nitro, Methyl, Ethyl, n- oder i-Propyl, n-, i-, s-oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy,
R² für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl, Cyclohexyl, Phenyl oder Benzyl steht,
R³ für Wasserstoff, Methyl, Ethyl, Propyl, Butyl, Allyl, Propargyl, Cyanoethyl, Methoxyethyl oder gegebenenfalls durch Chlor substituiertes Benzyl steht, oder zusammen mit R² für Butan-1,4-diyl oder Pentan-1,5-diyl steht,
R⁴ für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl, Cyclohexyl, Phenyl oder Benzyl steht,
R⁵ für Wasserstoff, Methyl, Ethyl, Propyl, Butyl, Allyl, Propargyl, Cyanoethyl, Methoxyethyl, Methoxy oder Benzyl steht, oder zusammen mit R⁴ für Butan-1,4-diyl, Pentan-1,5-diyl oder 3-Oxa-pentan-1,5-diyl steht,
X für Sauerstoff oder Schwefel steht und
Y für Sauerstoff oder Schwefel steht.

3. Verfahren zur Herstellung von substituierten 4,5-Diamino-1,2,4-triazol-3-(thi)onen der allgemeinen Formel (I) 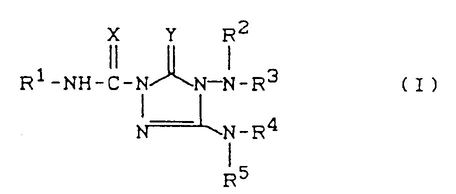 in welcher R¹, R², R³, R⁴, R⁵, X und Y die in Anspruch 1 genannten Bedeutungen haben, dadurch gekennzeichnet, daß man
(a) 4,5-Diamino-1,2,4-triazol-3-(thi)one der allgemeinen Formel (11) 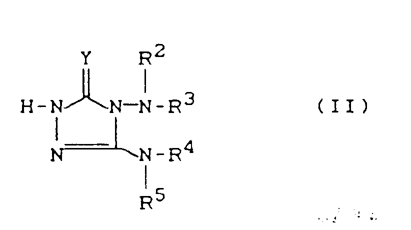 in welcher
R², R³, R⁴, R⁵ und Y die oben angegebenen Bedeutungen haben,
mit Iso(thio)cyanaten der allgemeinen Formel (III) 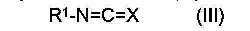 in welcher
R¹ und X die oben angegebenen Bedeutungen haben,
gegebenenfalls in Gegenwart eines Verdünnungsmittels und gegebenenfalls in Gegenwart eines Reaktionshilfsmittels umsetzt,
oderdaß man
(b) 4-Alkylidenamino-5-amino-1,2,4-triazol-3-(thi)one der allgemeinen Formel (IV) 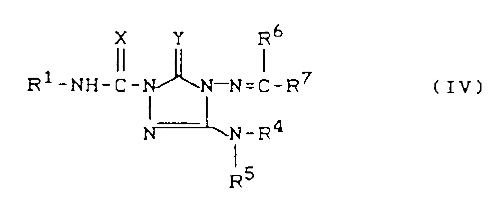 in welcher
R¹, R⁴, R⁵, X und Y die oben angegebenen Bedeutungen haben und
R⁶ und R⁷ gleich oder verschieden sind und unabhängig voneinander für Wasserstoff, geradkettiges oder verzweigtes Alkyl mit 1 bis 4 Kohlenstoffatomen, Phenyl oder Benzyl stehen
mit wäßrigen Säuren, gegebenenfalls in Gegenwart organischer Lösungsmittel, umsetzt,
oder daß man
(c) substituierte 1,2,4-Triazol-3-(thi)one der allgemeinen Formel (V) 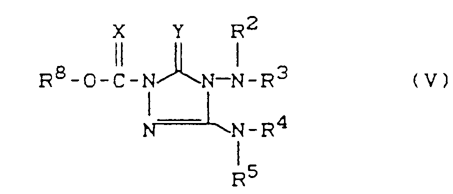 in welcher
R², R³, R⁴, R⁵, X und Y die oben angegebenen Bedeutungen haben und
R⁸ für Methyl, Ethyl, Propyl, Isopropyl, Butyl, Isobutyl, sec.-Butyl, tert.-Butyl, Phenyl oder Benzyl steht
mit Aminoverbindungen der allgemeinen Formel (VI) 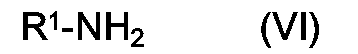 in welcher
R¹ die oben angegebene Bedeutung hat,
gegebenenfalls in Gegenwart eines Verdünnungsmittels und gegebenenfalls in Gegenwart eines Reaktionshilfsmittels umsetzt,
oderdaß man
(d) 4,5-Diamino-1,2,4-triazol-3-(thi)one der allgemeinen Formel (II) 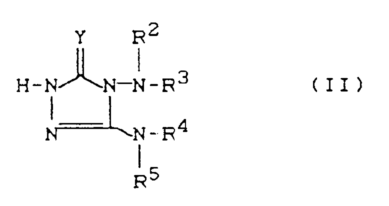 in welcher
R², R³, R⁴, R⁵ und Y die oben angegebenen Bedeutungen haben,
mit (Thio)Urethanen der allgemeinen Formel (VII) 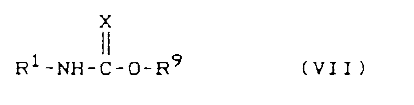 in welcher
R¹ und X die oben angegebenen Bedeutungen haben und
R⁹ für Methyl, Ethyl, Propyl, Isopropyl, Butyl, Isobutyl, sec-Butyl, tert.-Butyl, Phenyl oder Benzyl steht,
gegebenenfalls in Gegenwart eines Verdünnungsmittels und gegebenenfalls in Gegenwart eines Reaktionshilfsmittels umsetzt,
oder daß man
(e) 4-Oxyalkylidenamino-5-amino-1,2,4-triazol-3-(thi)one der allgemeinen Formel (VIII) 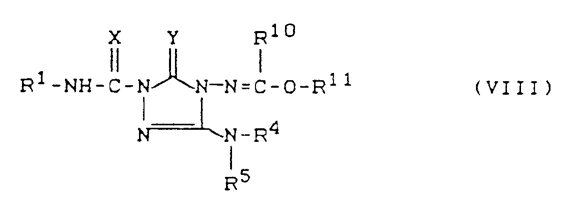 in welcher
R¹, R⁴, R⁵, X und Y die oben angegebenen Bedeutungen haben und
R¹⁰ für Wasserstoff, Methyl, Ethyl, Propyl, Phenyl oder Benzyl und
R¹¹ für Methyl, Ethyl, Propyl oder Benzyl steht,
mit Hydridkomplexen der allgemeinen Formel (IX) 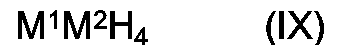 in welcher
M¹ für Lithium, Natrium oder Kalium steht und
M² für Bor oder Aluminium steht,
gegebenenfalls in Gegenwart eines Verdünnungsmittels umsetzt,
oderdaß man
(f) 4-Alkylidenamino-5-amino-1,2,4-triazol-3-(thi)one der allgemeinen Formel (IV) 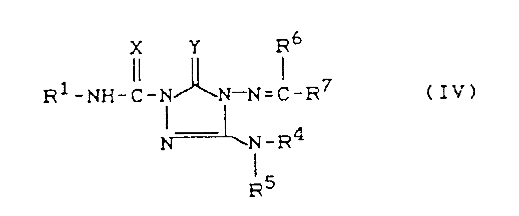 in welcher
R¹, R⁴, R⁵, X und Y die oben angegebenen Bedeutungen haben und
R⁶ und R⁷ gleich oder verschieden sind und unabhängig voneinander die oben angegebenen Bedeutungen haben,
mit Reduktionsmitteln, gegebenenfalls in Gegenwart von Katalysatoren und gegebenenfalls in Gegenwart von Verdünnungsmitteln umsetzt.

4. Herbizide Mittel, gekennzeichnet durch einen Gehalt an mindestens einem substituierten 4,5-Diamino-1,2,4-triazol-3-(thi)on der Formel (I) gemäß Anspruch 1 oder 3.

5. Verfahren zur Bekämpfung von unerwünschten Pflanzen, dadurch gekennzeichnet, daß man substituierte 4,5-Diamino-1,2,4-triazol-3-(thi)one der Formel (I) gemäß Anspruch 1 oder 3 auf die Pflanzen und/oder ihren Lebensraum einwirken läßt.

6. Verwendung von substituierten 4,5-Diamino-1,2,4-triazol-3-(thi)onen der Formel (I) gemäß Anspruch 1 oder 3 zur Bekämpfung von unerwünschten Pflanzen.

7. Verfahren zur Herstellung von herbiziden Mitteln, dadurch gekennzeichnet, daß man substituierte 4,5-Diamino-1,2,4-triazol-3-(thi)one der Formel (I) gemäß Anspruch 1 oder4 mit Streckmitteln und/oderoberflächenaktiven Substanzen vermischt.

8. 4,5-Diamino-1,2,4-triazol-3-one der allgemeinen Formel (Ila) 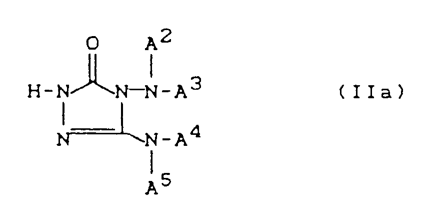 in welcher
A², A³, A⁴ und A⁵ gleich oder verschieden sind und für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Alkoxyalkyl oder Alkylthioalkyl mit jeweils bis zu 4 Kohlenstoffatomen in den einzelnen Alkylteilen, fürjeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oderverschiedenen Halogenatomen, in welcher weiter auch jeweils zwei dieser Reste - A² und A³ oder A⁴ und A^{5 -} zusammen für geradkettiges oder verzweigtes Alkandiyl oder Oxaalkandiyl mit jeweils 2 bis 6 Kohlenstoffatomen stehen können, und A⁵ auch für geradkettiges oder verzweigtes Alkoxy mit 1 bis 8 Kohlenstoffatomen stehen kann, mit der Maßgabe, daß wenigstens einer der Reste A², A³, A⁴ oder A⁵ von Wasserstoff verschieden ist.

9. 4-Alkylidenamino-5-amino-1,2,4-triazol-3-(thi)one der allgemeinen Formel (IV) 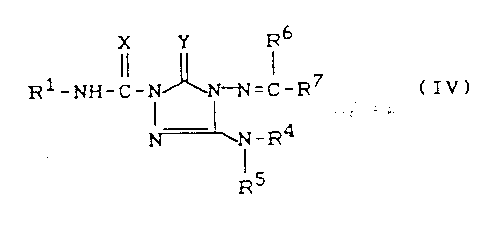 in welcher
R¹ für Wasserstoff, für jeweils geradkettiges oderverzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, für Phenoxyalkyl mit 1 bis 4 Kohlenstoffatomen im Alkylteil, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für jeweils gegebenenfalls einfach bis dreifach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen und geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; R¹ außerdem für im Heterocyclylteil gegebenenfalls einfach bis dreifach, gleich oder verschieden substituiertes Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen -insbesondere Stickstoff, Sauerstoff und/oder Schwefel - im Heterocyclylteil steht, wobei als Substituenten infrage kommen; Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; R¹ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen oder für jeweils gegebenenfalls einfach bis dreifach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im geradkettigen oder verzweigten Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oderverzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder R¹ für Benzyl mit im Phenylteil ankondensierter -O-CH₂-0- Gruppe steht,
R⁴ und R⁵ gleich oder verschieden sind und unabhängig voneinander für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanoalkyl mit 1 bis 8 Kohlenstoffatomen, Alkoxyalkyl oder Alkylthioalkyl mit jeweils bis zu 4 Kohlenstoffatomen in den einzelnen Alkylteilen, für jeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen, die Reste R⁴ und R⁵ weiter für jeweils gegebenenfalls einfach bis dreifach gleich oder verschieden substituiertes Aryl oder Aralkyl stehen mit jeweils 6 oder 10 Kohlenstoffatomen im Arylteil und gegebenenfalls 1 bis 4 Kohlenstoffatomen im geradkettigen oder verzweigten Alkylteil, wobei als Substituenten infrage kommen; Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 Halogenatomen, in welcher weiter auch jeweils zwei dieser Reste - R² und R³ oder R⁴ und R^{5 -} zusammen für geradkettiges oder verzweigtes Alkandiyl oder Oxaalkandiyl mit jeweils 2 bis 6 Kohlenstoffatomen stehen können, und R⁵ auch für geradkettiges oder verzweigtes Alkoxy mit 1 bis 8 Kohlenstoffatomen stehen kann,
X für Sauerstoff oder Schwefel steht und
Y für Sauerstoff oder Schwefel steht,
R⁶ und R⁷ gleich oder verschieden sind und unabhängig voneinander für Wasserstoff, geradkettiges oder verzweigtes Alkyl mit 1 bis 4 Kohlenstoffatomen, Phenyl oder Benzyl stehen.

10. 4-Alkylidenamino-5-amino-1,2,4-triazol-3-(thi)one der Formel (XXI) 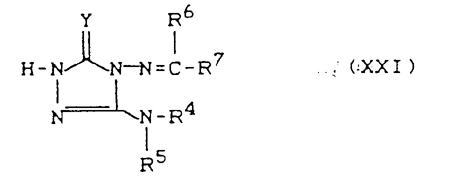 in welcher
R⁴ und R⁵ gleich oder verschieden sind und unabhängig voneinander für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanoalkyl mit 1 bis 8 Kohlenstoffatomen, Alkoxyalkyl oder Alkylthioalkyl mit jeweils bis zu 4 Kohlenstoffatomen in den einzelnen Alkylteilen, für jeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen, die Reste R⁴ und R⁵ weiter für jeweils gegebenenfalls einfach bis dreifach gleich oder verschieden substituiertes Aryl oder Aralkyl stehen mit jeweils 6 oder 10 Kohlenstoffatomen im Arylteil und gegebenenfalls 1 bis 4 Kohlenstoffatomen im geradkettigen oder verzweigten Alkylteil, wobei als Substituenten infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 Halogenatomen, in welcher weiter
R⁴ und R^{5 -} zusammen für geradkettiges oder verzweigtes Alkandiyl oder Oxaalkandiyl mit jeweils 2 bis 6 Kohlenstoffatomen stehen können, und R⁵ auch für geradkettiges oder verzweigtes Alkoxy mit 1 bis 8 Kohlenstoffatomen stehen kann, mit der Maßgabe, daß R⁴ und/oder R⁵ von Wasserstoff verschieden sind,
Y für Sauerstoff oder Schwefel steht,
R⁶ und R⁷ gleich oder verschieden sind und unabhängig voneinander für Wasserstoff, geradkettiges oder verzweigtes Alkyl mit 1 bis 4 Kohlenstoffatomen, Phenyl oder Benzyl stehen.

11. 2-Oxy-(thio)-carbonyl-4-alkylidenamino-5-amino-1,2,4-triazol-3-(thi)one der Formel (XXIII) 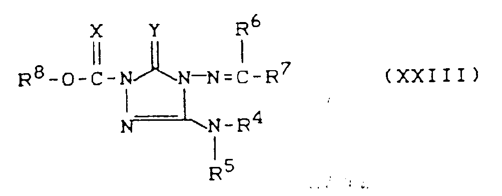 in welcher
R⁴ und R⁵ gleich oder verschieden sind und unabhängig voneinander für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanoalkyl mit 1 bis 8 Kohlenstoffatomen, Alkoxyalkyl oder Alkylthioalkyl mit jeweils bis zu 4 Kohlenstoffatomen in den einzelnen Alkylteilen, für jeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen, die Reste R⁴ und R⁵ weiter für jeweils gegebenenfalls einfach bis dreifach gleich oder verschieden substituiertes Aryl oder Aralkyl stehen mit jeweils 6 oder 10 Kohlenstoffatomen im Arylteil und gegebenenfalls 1 bis 4 Kohlenstoffatomen im geradkettigen oder verzweigten Alkylteil, wobei als Substituenten infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 Halogenatomen, in welcher weiter
R⁴ und R^{5 -} zusammen für geradkettiges oder verzweigtes Alkandiyl oder Oxaalkandiyl mit jeweils 2 bis 6 Kohlenstoffatomen stehen können, und R⁵ auch für geradkettiges oder verzweigtes Alkoxy mit 1 bis 8 Kohlenstoffatomen stehen kann,
X für Sauerstoff oder Schwefel steht,
Y für Sauerstoff oder Schwefel steht,
R⁶ und R⁷ gleich oder verschieden sind und unabhängig voneinander für Wasserstoff, geradkettiges oder verzweigtes Alkyl mit 1 bis 4 Kohlenstoffatomen, Phenyl oder Benzyl stehen, und
R⁸ für Methyl, Ethyl, Propyl, Isopropyl, Butyl, Isobutyl, sec-Butyl, tert.-Butyl, Phenyl oder Benzyl steht.

12. Oxyalkylidenverbindungen der Formel (XXV) 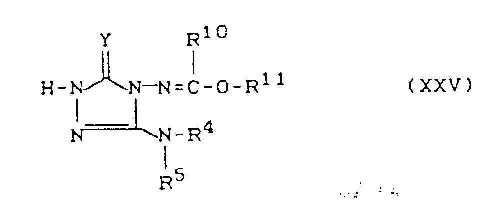 in welcher
R⁴ und R⁵ gleich oder verschieden sind und unabhängig voneinander für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanoalkyl mit 1 bis 8 Kohlenstoffatomen, Alkoxyalkyl oder Alkylthioalkyl mit jeweils bis zu 4 Kohlenstoffatomen in den einzelnen Alkylteilen, für jeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen, die Reste R⁴ und R⁵ weiter für jeweils gegebenenfalls einfach bis dreifach gleich oder verschieden substituiertes Aryl oder Aralkyl stehen mit jeweils 6 oder 10 Kohlenstoffatomen im Arylteil und gegebenenfalls 1 bis 4 Kohlenstoffatomen im geradkettigen oder verzweigten Alkylteil, wobei als Substituenten infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 Halogenatomen, in welcher weiter
R⁴ und R^{5 -} zusammen für geradkettiges oder verzweigtes Alkandiyl oder Oxaalkandiyl mit jeweils 2 bis 6 Kohlenstoffatomen stehen können, und R⁵ auch für geradkettiges oder verzweigtes Alkoxy mit 1 bis 8 Kohlenstoffatomen stehen kann,
Y für Sauerstoff oder Schwefel steht.
R¹⁰ und R11 für Wasserstoff, C₁-Cₛ-Alkyl, C₂-C₆-Alkenyl C₂-C₆-Alkinyl, C₃-Cₛ-Cycloalkyl, Phenyl oder Benzyl stehen.

## Claims

1. Substituted 4,5-diamino-1,2,4-triazol(e)-3-(thi)ones of the general formula (I) 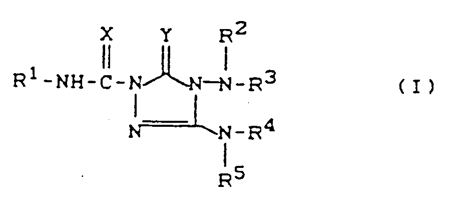 in which
R¹ represents hydrogen, or represents in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, in each case having 2 to 8 carbon atoms and 1 to 15, or 13, identical or different halogen atoms, cyanoalkyl having 1 to 8 carbon atoms or hydroxyalkyl having 1 to 8 carbon atoms and 1 to 6 hydroxyl groups, or represents phenoxyalkyl having 1 to 4 carbon atoms in the alkyl moiety, alkoxyalkyl, alkoxycarbonylalkyl or alkoxycarbonylalkenyl, in each case having up to 6 carbon atoms in the individual alkyl or alkenyl moieties, or alkylaminoalkyl or dialkylaminoalkyl, in each case having 1 to 6 carbon atoms in the individual alkyl moieties, or represents cycloalkyl, cycloalkylalkyl, cycloalkenyl or cycloalkenylalkyl, each of which has 3 to 8 carbon atoms in the cycloalkyl or cycloalkenyl moiety and, where appropriate, 1 to 6 carbon atoms in the alkyl moiety and each of which is optionally monosubstituted to trisubstituted by identical or different substituents, suitable substituents in each case being: halogen, cyano as well as in each case straight-chain or branched alkyl or halogenoalkyl, in each case having 1 to 4 carbon atoms and, where appropriate, 1 to 9 identical ordifferent halogen atoms, and straight-chain or branched halogenoalkenyl having up to 4 carbon atoms and 1 to 5 identical or different halogen atoms, or in each case double- linked alkanediyl or alkenediyl, in each case having up to 4 carbon atoms; R¹ furthermore represents heterocyclylalkyl which has 1 to 6 carbon atoms in the straight-chain or branched alkyl moiety and 2 to 9 carbon atoms as well as 1 to 3 heteroatoms-in particular nitrogen, oxygen and/orsulphur- in the heterocyclyl moiety and which is optionally monosubstituted to trisubstituted in the heterocyclyl moiety by identical or different substituents, suitable substituents being: halogen, cyano, nitro, as well as in each case straight-chain or branched alkyl, alkoxy, alkylthio, halogenoalkyl, halogenoalkoxy, halogenoalkylthio or alkoxycarbonyl, in each case having 1 to 5 carbon atoms and, where appropriate, 1 to 9 identical or different halogen atoms; R¹ furthermore represents in each case straight-chain or branched alkoxy having 1 to 8 carbon atoms, alkenyloxy having 2 to 8 carbon atoms or alkinyloxy having 2 to 8 carbon atoms, or represents aralkyl, aroyl, aryl, aralkyloxy or aryloxy, each of which has 6 to 10 carbon atoms in the aryl moiety and, where appropriate, 1 to 8 carbon atoms in the straight-chain or branched alkyl moiety and each of which is optionally monosubstituted to trisubstitued by identical or different substituents, suitable alkyl substituents, where appropriate, being halogen and cyano and suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, in each case having 1 to 6 carbon atoms in the alkyl moiety and, where appropriate, 1 to 9 identical or different halogen atoms, or cycloalkyl having 3 to 6 carbon atoms, and phenoxy; or R¹ represents benzyl with an -0-CH₂-O-group fused to the phenyl moiety,
R², R³, R⁴ and R⁵ are identical or different and independently of one another represent hydrogen, or represent in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, in each case having 2 to 8 carbon atoms and 1 to 15, or 13, identical or different halogen atoms, cyanoalkyl having 1 to 8 carbon atoms, or alkoxyalkyl or alkylthioalkyl, in each case having up to 4 carbon atoms in the individual alkyl moieties, or represent cycloalkyl orcycloalkylalkyl, each of which has 3 to 8 carbon atoms in the cycloalkyl moiety and, where appropriate, 1 to 6 carbon atoms in the alkyl moiety and each of which is optionally monosubstituted to trisubstituted by identical or different substituents, suitable substituents in each case being: halogen, cyano as well as in each case straight-chain or branched alkyl or halogenoalkyl, in each case having 1 to 4 carbon atoms and, where appropriate, 1 to 9 identical or different halogen atoms, the radicals R² to R⁵ furthermore represent aryl or aralkyl, each of which has 6 or 10 carbon atoms in the aryl moiety and, where appropriate, 1 to 4 carbon atoms in the straight-chain or branched alkyl moiety and each of which is optionally monosubstituted to trisubstituted by identical or different substituents, suitable substituents being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl,halogenoalkylsulphinyl,halogenoalkylsulphonyl, alkanoyl or alkoxycarbonyl, in each case having 1 to 6 carbon atoms in the alkyl moiety and, where appropriate, 1 to 9 halogen atoms, in which formula it is furthermore also possible for in each case two of these radicals together- R² and R³ or R⁴ and R^{5 -} to represent straight-chain or branched alkanediyl or oxaalkanediyl, in each case having 2 to 6 carbon atoms, and R⁵ can also represent straight-chain or branched alkoxy having 1 to 8 carbon atoms,
X represents oxygen or sulphur and
Y represents oxygen or sulphur.

2. Substituted 4,5-diamino-1,2,4-triazol(e)-3-(thi)ones of the formula (I) according to Claim 1, in which
R¹ represents hydrogen, methyl, ethyl, n- or i- propyl, n-, i-, s- or t-butyl, in each case straight-chain or branched pentyl, hexyl, heptyl, octyl, nonyl, decyl or dodecyl, or represents allyl, in each case straight-chain or branched butenyl, pentenyl or hexenyl, propargyl, in each case straight-chain or branched butinyl, pentinyl or hexinyl, or represents straight-chain or branched halogenoalkyl having 1 to 8 carbon atoms and 1 to 9 identical or different halogen atoms, or represents in each case straight-chain or branched halogenoalkenyl or halogenoalkinyl, in each case having 3 to 8 carbon atoms and 1 to 3 halogen atoms, or represents in each case straight-chain or branched cyanoalkyl having 1 to 6 carbon atoms in the alkyl moiety, hydroxyalkyl having 1 to 6 carbon atoms and 1 to 3 hydroxyl groups, alkoxyalkyl, alkoxycarbonylalkyl or alkoxycarbonylalkenyl, alkylaminoalkyl or dialkylaminoalkyl, in each case having up to 4 carbon atoms in the individual alkyl or alkenyl moieties, or represents cyclopropyl, cyclopropylmethyl, cyclopropylethyl, cyclopentyl, cyclohexyl, cycloheptyl, cyclooctyl, cyclohexylmethyl, cyclohexylethyl, cyclohexenyl or cyclohexenylmethyl, each of which is optionally monosubstituted to trisubstituted by identical or different substituents, suitable substituents in each case being: fluorine, chlorine, bromine, methyl, ethyl, nor i-propyl, n-, i-, s- or t-butyl, cyano, methanediyl, ethanediyl, butanediyl or butadienediyl or dichloroallyl;
R¹ furthermore represents heterocyclylmethyl, heterocyclylpropyl or heterocyclylethyl, each of which is optionally monosubstituted to trisubstituted in the heterocyclyl moiety by identical or different substituents, suitable heterocycles in each case being: 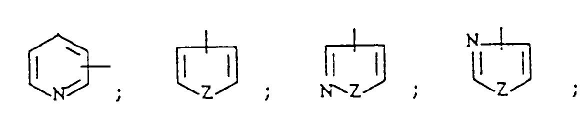 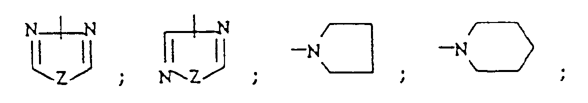 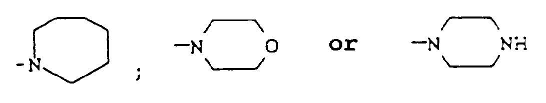 Z in each case representing oxygen or sulphur and suitable substituents in each case being: fluorine, chlorine, bromine, cyano, nitro, methyl, ethyl, n- or i-propyl, n-, i-, s- ort-butyl, methoxy, ethoxy, methylthio, trifluoromethyl, trifluoromethoxy or trifluoromethylthio;
R¹ furthermore represents in each case straight-chain or branched alkoxy having 1 to 6 carbon atoms, alkenyloxy having 3 to 6 carbon atoms or alkinyloxy having 3 to 6 carbon atoms, or represents benzyl, phenylethyl, phenylpropyl, phenylbutyl, phenylpentyl, phenylhexyl, phenylheptyl, phenylcyanomethyl, phenylcyanoethyl, phenylcyanopropyl, benzyloxy, phenylethyloxy, phenoxy, benzoyl, phenyl or naphthyl, where appropriate straight-chain or branched, each of which is optionally monosubstituted to trisubstituted by identical or different substituents, suitable phenyl substituents in each case being: fluorine, chlorine, bromine, hydroxyl, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy,
R² represents hydrogen, methyl, ethyl, propyl, isopropyl, butyl, isobutyl, sec-butyl, allyl, propargyl, cyanoethyl, methoxyethyl,cyclopropyl, cyclopentyl, cyclohexyl, phenyl or benzyl,
R³ represents hydrogen, methyl, ethyl, propyl, butyl, allyl, propargyl, cyanoethyl, methoxyethyl or optionally chlorine-substituted benzyl, or together with R² represents butane-1,4-diyl or pentane-1,5-diyl,
R⁴ represents hydrogen, methyl, ethyl, propyl, isopropyl, butyl, isobutyl, sec-butyl, allyl, propargyl, cyanoethyl, methoxyethyl,cyclopropyl, cyclopentyl, cyclohexyl, phenyl or benzyl,
R⁵ represents hydrogen, methyl, ethyl, propyl, butyl, allyl, propargyl, cyanoethyl, methoxyethyl, methoxy or benzyl, ortogetherwith R⁴represents butane-1,4-diyl, pentane-1,5-diyi or3-oxa-pentane-1,5-diyl,
X represents oxygen or sulphur and
Y represents oxygen or sulphur.

3. Process for the preparation of substituted 4,5-diamino-1,2,4-triazol(e)-3-(thi)ones of the general formula (I) 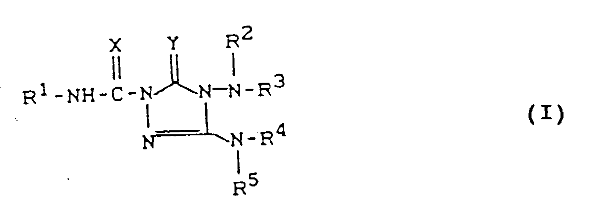 in which R¹, R², R³, R⁴, R⁵, X and Y have the meanings given in Claim 1,
characterized in that
(a) 4,5-diamino-1,2,4-triazol(e)-3-(thi)ones of the general formula (II) 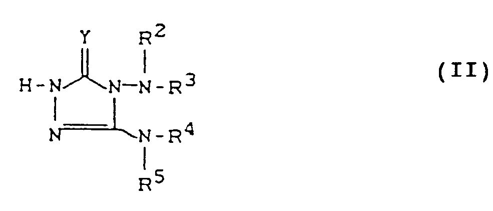 in which
R², R³, R⁴, R⁵ and Y have the abovementioned meanings,
are reacted with iso(thio)cyanates of the general formula (III) 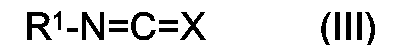 in which
R¹ and X have the abovementioned meanings,
if appropriate in the presence of a diluent and if appropriate in the presence of a reaction auxiliary,
or in that
(b) 4-alkylideneamino-5-amino-1,2,4-triazol(e)-3-(thi)ones of the general formula (IV) 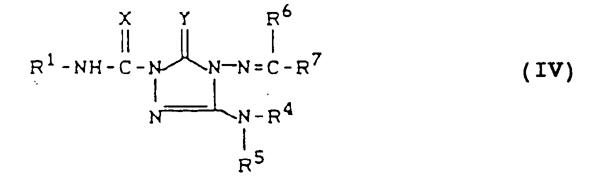 in which
R¹, R⁴, R⁵, X and Y have the abovementioned meanings and
R⁶ and R⁷ are identical or different and independently of one another represent hydrogen, straight-chain or branched alkyl having 1 to 4 carbon atoms, phenyl or benzyl,
are reacted with aqueous acids, if appropriate in the presence of organic solvents,
or in that
(c) substituted 1,2,4-triazol(e)-3-(thi)ones of the general formula (V) 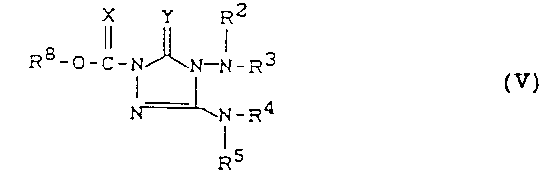 in which
R², R³, R⁴, R⁵, X and Y have the abovementioned meanings and
R⁸ represents methyl, ethyl, propyl, isopropyl, butyl, isobutyl, sec.-butyl, tert.-butyl, phenyl or benzyl,
are reacted with amino compounds of the general formula (VI) 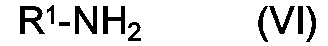 in which
R¹ has the abovementioned meaning,
if appropriate in the presence of a diluent and if appropriate in the presence of a reaction auxiliary,
or in that
(d) 4,5-diamino-1,2,4-triazol(e)-3-(thi)ones of the general formula (II) 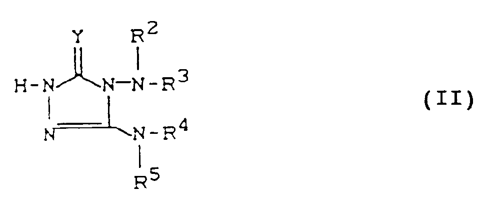 in which
R², R³, R⁴, R⁵ and Y have the abovementioned meanings,
are reacted with (thio)urethanes of the general formula (VII) 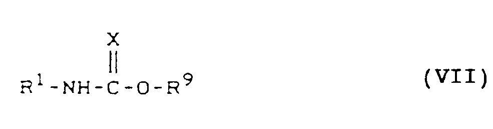 in which
R¹ and X have the abovementioned meanings and
R⁹ represents methyl, ethyl, propyl, isopropyl, butyl, isobutyl, sec-butyl, tert.-butyl, phenyl or benzyl,
if appropriate in the presence of a diluent and if appropriate in the presence of a reaction auxiliary,
or in that
(e) 4-oxyalkylideneamino-5-amino-1,2,4-triazol(e)-3-(thi)ones of the general formula (VIII) 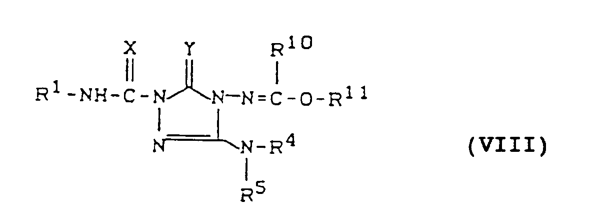 in which
R¹, R⁴, R⁵, X and Y have the abovementioned meanings and
R¹⁰ represents hydrogen, methyl, ethyl, propyl, phenyl or benzyl and
R¹¹ represents methyl, ethyl, propyl or benzyl, are reacted with hydride complexes of the general formula (IX) 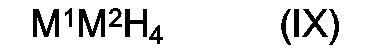 in which
M¹ represents lithium, sodium or potassium and
M² represents boron or aluminium,
if appropriate in the presence of a diluent,
or in that
(f) 4-alkylideneamino-5-amino-1,2,4-triazol(e)-3-(thi)ones of the general formula (IV) 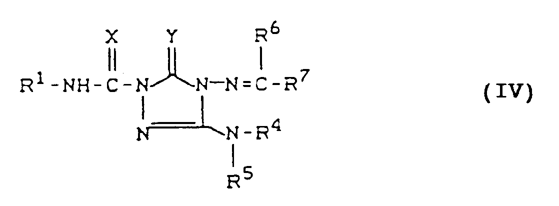 in which
R¹, R⁴, R⁵, X and Y have the abovementioned meanings and
R⁶ and R⁷ are identical or different and independently of one another have the abovementioned meanings,
are reacted with reducing agents, if appropriate in the presence of catalysts and if appropriate in the presence of diluents.

4. Herbicidal agents, characterised in that they contain at least one substituted 4,5-diamino-1,2,4-triazol(e)-3-(thi)one of the formula (I) according to Claim 1 or 3.

5. Method for combating undesired plants, characterised in that substituted 4,5-diamino-1,2,4-triazol(e)-3-(thi)ones of the formula (I) according to Claim 1 or 3 are allowed to act on the plants and/or their environment.

6. Use of substituted 4,5-diamino-1,2,4-triazol(e)-3-(thi)ones of the formula (I) according to Claim 1 or 3 for combating undesired plants.

7. Process for the preparation of herbicidal agents, characterised in that substituted 4,5-diamino-1,2,4-triazol(e)-3-(thi)ones of the formula (I) according to Claim 1 or 4 are mixed with extenders and/or surface- active substances.

8. 4,5-Diamino-1,2,4-triazol-3-ones of the general formula (Ila) 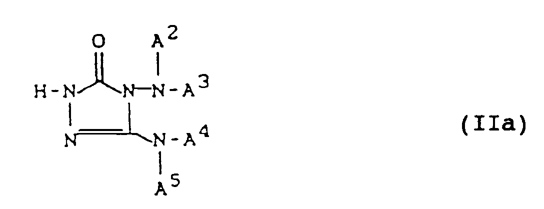 in which
A², A3, A4 and A⁵ are identical or different and represent hydrogen, or represent in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, in each case having 2 to 8 carbon atoms and 1 to 15, or 13, identical or different halogen atoms, cyanoalkyl having 1 to 8 carbon atoms, or alkoxyalkyl or alkylthioalkyl, in each case having up to 4 carbon atoms in the individual alkyl moieties, or represent cycloalkyl or cycloalkylalkyl, each of which has 3 to 8 carbon atoms in the cycloalkyl moiety and, where appropriate, 1 to 6 carbon atoms in the alkyl moiety and each of which is optionally monosubstituted to trisubstituted by identical or different substituents, suitable substituents in each case being: halogen, cyano as well as in each case straight-chain or branched alkyl or halogenoalkyl, in each case having 1 to 4 carbon atoms and, where appropriate, 1 to 9 identical or different halogen atoms, in which formula it is furthermore also possible for in each case two of these radicals together - A² and A3 or A4 and A^{5 -} to represent straight-chain or branched alkanediyl or oxaalkanediyl, in each case having 2 to 6 carbon atoms, and A⁵ can also represent straight-chain or branched alkoxy having 1 to 8 carbon atoms, with the proviso that at least one of the radicals A², A3, A4 or A⁵ is other than hydrogen.

9. 4-Alkylideneamino-5-amino-1,2,4-triazol(e)-3-(thi)ones of the general formula (IV) 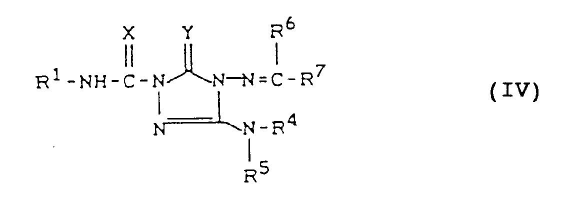 in which
R¹ represents hydrogen, or represents in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, in each case having 2 to 8 carbon atoms and 1 to 15, or 13, identical or different halogen atoms, cyanoalkyl having 1 to 8 carbon atoms or hydroxyalkyl having 1 to 8 carbon atoms and 1 to 6 hydroxyl groups, or represents phenoxyalkyl having 1 to 4 carbon atoms in the alkyl moiety, alkoxyalkyl, alkoxycarbonylalkyl or alkoxycarbonylalkenyl, in each case having up to 6 carbon atoms in the individual alkyl or alkenyl moieties, or alkylaminoalkyl or dialkylaminoalkyl, in each case having 1 to 6 carbon atoms in the individual alkyl moieties, or represents cycloalkyl, cycloalkylalkyl, cycloalkenyl or cycloalkenylalkyl, each of which has 3 to 8 carbon atoms in the cycloalkyl or cycloalkenyl moiety and, where appropriate, 1 to 6 carbon atoms in the alkyl moiety and each of which is optionally monosubstituted to trisubstituted by identical or different substituents, suitable substituents in each case being: halogen, cyano as well as in each case straight-chain or branched alkyl or halogenoalkyl, in each case having 1 to 4 carbon atoms and, where appropriate, 1 to 9 identical ordifferent halogen atoms, and straight-chain or branched halogenoalkenyl having up to 4 carbon atoms and 1 to 5 identical or different halogen atoms, or in each case double- linked alkanediyl or alkenediyl, in each case having up to 4 carbon atoms; R¹ furthermore represents heterocyclylalkyl which has 1 to 6 carbon atoms in the straight-chain or branched alkyl moiety and 2 to 9 carbon atoms as well as 1 to 3 heteroatoms-in particular nitrogen, oxygen and/orsulphur- in the heterocyclyl moiety and which is optionally monosubstituted to trisubstituted in the heterocyclyl moiety by identical or different substituents, suitable substituents being: halogen, cyano, nitro, as well as in each case straight-chain or branched alkyl, alkoxy, alkylthio, halogenoalkyl, halogenoalkoxy, halogenoalkylthio or alkoxycarbonyl, in each case having 1 to 5 carbon atoms and, where appropriate, 1 to 9 identical or different halogen atoms; R¹ furthermore represents in each case straight-chain or branched alkoxy having 1 to 8 carbon atoms, alkenyloxy having 2 to 8 carbon atoms or alkinyloxy having 2 to 8 carbon atoms, or represents aralkyl, aroyl, aryl, aralkyloxy or aryloxy, each of which has 6 to 10 carbon atoms in the aryl moiety and, where appropriate, 1 to 8 carbon atoms in the straight-chain or branched alkyl moiety and each of which is optionally monosubstituted to trisubstitued by identical or different substituents, suitable alkyl substituents, where appropriate, being halogen and cyano and suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl,alkylsulphonyl,halogenoalkylsulphinyl, halogenoalkylsulphonyl, alkanoyl or alkoxycarbonyl, in each case having 1 to 6 carbon atoms in the alkyl moiety and, where appropriate, 1 to 9 identical or different halogen atoms, or cycloalkyl having 3 to 6 carbon atoms, and phenoxy; or R¹ represents benzyl with an -O-CH_{Z}-O- group fused to the phenyl moiety,
R⁴ and R⁵ are identical or different and independently of one another represent hydrogen, or represent in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical ordifferent halogen atoms, halogenoalkenyl or halogenoalkinyl, in each case having 2 to 8 carbon atoms and 1 to 15, or 13, identical or different halogen atoms, cyanoalkyl having 1 to 8 carbon atoms, or alkoxyalkyl or alkylthioalkyl, in each case having up to 4 carbon atoms in the individual alkyl moieties, or represent cycloalkyl or cycloalkylalkyl, each of which has 3 to 8 carbon atoms in the cycloalkyl moiety and, where appropriate, 1 to 6 carbon atoms in the alkyl moiety and each of which is optionally monosubstituted to trisubstituted by identical or different substituents, suitable substituents in each case being: halogen, cyano as well as in each case straight-chain or branched alkyl or halogenoalkyl, in each case having 1 to 4 carbon atoms and, where appropriate, 1 to 9 identical or different halogen atoms, the radicals R⁴ and R⁵ furthermore represent aryl or aralkyl, each of which has 6 or 10 carbon atoms in the aryl moiety and, where appropriate, 1 to 4 carbon atoms in the straight-chain or branched alkyl moiety and each of which is optionally monosubstituted to trisubstituted by identical or different substituents, suitable substituents being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl,halogenoalkylsulphinyl,halogenoalkylsulphonyl, alkanoyl or alkoxycarbonyl, in each case having 1 to 6 carbon atoms in the alkyl moiety and, where appropriate, 1 to 9 halogen atoms, in which formula it is furthermore also possible for in each case two of these radicals together- R² and R³ or R⁴ and R^{5 -} to represent straight-chain or branched alkanediyl or oxaalkanediyl, in each case having 2 to 6 carbon atoms, and R⁵ can also represent straight-chain or branched alkoxy having 1 to 8 carbon atoms,
X represents oxygen or sulphur, and
Y represents oxygen or sulphur,
R⁶ and R⁷ are identical or different and independently of one another represent hydrogen, straight-chain or branched alkyl having 1 to 4 carbon atoms, phenyl or benzyl.

10. 4-Alkylideneamino-5-amino-1,2,4-triazol(e)-3-(thi)ones of the formula (XXI) 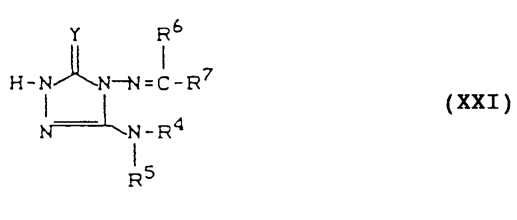 in which
R⁴ and R⁵ are identical or different and independently of one another represent hydrogen, or represent in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical ordifferent halogen atoms, halogenoalkenyl or halogenoalkinyl, in each case having 2 to 8 carbon atoms and 1 to 15, or 13, identical or different halogen atoms, cyanoalkyl having 1 to 8 carbon atoms, or alkoxyalkyl or alkylthioalkyl, in each case having up to 4 carbon atoms in the individual alkyl moieties, or represent cycloalkyl or cycloalkylalkyl, each of which has 3 to 8 carbon atoms in the cycloalkyl moiety and, where appropriate, 1 to 6 carbon atoms in the alkyl moiety and each of which is optionally monosubstituted to trisubstituted by identical or different substituents, suitable substituents in each case being: halogen, cyano as well as in each case straight-chain or branched alkyl or halogenoalkyl, in each case having 1 to 4 carbon atoms and, where appropriate, 1 to 9 identical or different halogen atoms, the radicals R⁴ and R⁵ furthermore represent aryl or aralkyl, each of which has 6 or 10 carbon atoms in the aryl moiety and, where appropriate, 1 to 4 carbon atoms in the straight-chain or branched alkyl moiety and each of which is optionally monosubstituted to trisubstituted by identical or different substituents, suitable substituents being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl,halogenoalkylsulphinyl, halogenoalkylsulphonyl, alkanoyl or alkoxycarbonyl, in each case having 1 to 6 carbon atoms in the alkyl moiety and, where appropriate, 1 to 9 halogen atoms, in which formula it is furthermore possible for R⁴ and R⁵ together to represent straight-chain or branched alkanediyl or oxaalkanediyl, in each case having 2 to 6 carbon atoms, and R⁵ can also represent straight-chain or branched alkoxy having 1 to 8 carbon atoms, with the proviso that R⁴ and/or R⁵ are other than hydrogen,
Y represents oxygen or sulphur,
R⁶ and R⁷ are identical or different and independently of one another represent hydrogen, straight-chain or branched alkyl having 1 to 4 carbon atoms, phenyl or benzyl.

11. 2-Oxy-(thio)-carbonyl-4-alkylideneamino-5-amino-1,2,4-triazol(e)-3-(thi)ones of the formula (XXIII) 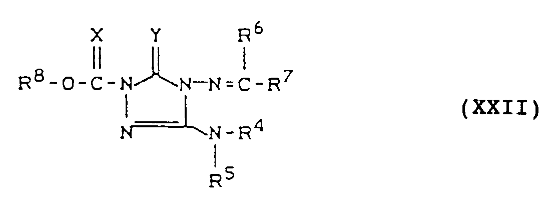 in which
R⁴ and R⁵ are identical or different and independently of one another represent hydrogen, or represent in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical ordifferent halogen atoms, halogenoalkenyl or halogenoalkinyl, in each case having 2 to 8 carbon atoms and 1 to 15, or 13, identical or different halogen atoms, cyanoalkyl having 1 to 8 carbon atoms, or alkoxyalkyl or alkylthioalkyl, in each case having up to 4 carbon atoms in the individual alkyl moieties, or represent cycloalkyl or cycloalkylalkyl, each of which has 3 to 8 carbon atoms in the cycloalkyl moiety and, where appropriate, 1 to 6 carbon atoms in the alkyl moiety and each of which is optionally monosubstituted to trisubstituted by identical or different substituents, suitable substituents in each case being: halogen, cyano as well as in each case straight-chain or branched alkyl or halogenoalkyl, in each case having 1 to 4 carbon atoms and, where appropriate, 1 to 9 identical or different halogen atoms, the radicals R⁴ and R⁵ furthermore represent aryl or aralkyl, each of which has 6 or 10 carbon atoms in the aryl moiety and, where appropriate, 1 to 4 carbon atoms in the straight-chain or branched alkyl moiety and each of which is optionally monosubstituted to trisubstituted by identical or different substituents, suitable substituents being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl,halogenoalkylsulphinyl,halogenoalkylsulphonyl, alkanoyl or alkoxycarbonyl, in each case having 1 to 6 carbon atoms in the alkyl moiety and, where appropriate, 1 to 9 halogen atoms, in which formula it is furthermore possible for R⁴ and R⁵ together to represent straight-chain or branched alkanediyl or oxaalkanediyl, in each case having 2 to 6 carbon atoms, and R⁵ can also represent straight-chain or branched alkoxy having 1 to 8 carbon atoms,
X represents oxygen or sulphur,
Y represents oxygen or sulphur,
R⁶ and R⁷ are identical or different and independently of one another represent hydrogen, straight-chain or branched alkyl having 1 to 4 carbon atoms, phenyl or benzyl, and
R⁸ represents methyl, ethyl, propyl, isopropyl, butyl, isobutyl, sec-butyl, tert.-butyl, phenyl or benzyl.

12. Oxyalkylidene compounds of the formula (XXV) 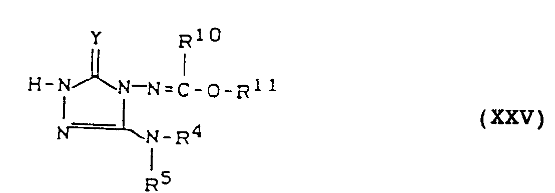 in which
R⁴ and R⁵ are identical or different and independently of one another represent hydrogen, or represent in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical ordifferent halogen atoms, halogenoalkenyl or halogenoalkinyl, in each case having 2 to 8 carbon atoms and 1 to 15, or 13, identical or different halogen atoms, cyanoalkyl having 1 to 8 carbon atoms, or alkoxyalkyl or alkylthioalkyl, in each case having up to 4 carbon atoms in the individual alkyl moieties, or represent cycloalkyl or cycloalkylalkyl, each of which has 3 to 8 carbon atoms in the cycloalkyl moiety and, where appropriate, 1 to 6 carbon atoms in the alkyl moiety and each of which is optionally monosubstituted to trisubstituted by identical or different substituents, suitable substituents in each case being: halogen, cyano as well as in each case straight-chain or branched alkyl or halogenoalkyl, in each case having 1 to 4 carbon atoms and, where appropriate, 1 to 9 identical or different halogen atoms, the radicals R⁴ and R⁵ furthermore represent aryl or aralkyl, each of which has 6 or 10 carbon atoms in the aryl moiety and, where appropriate, 1 to 4 carbon atoms in the straight-chain or branched alkyl moiety and each of which is optionally monosubstituted to trisubstituted by identical or different substituents, suitable substituents being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl,halogenoalkylsulphinyl,halogenoalkylsulphonyl, alkanoyl or alkoxycarbonyl, in each case having 1 to 6 carbon atoms in the alkyl moiety and, where appropriate, 1 to 9 halogen atoms, in which formula it is furthermore possible for R⁴ and R⁵ together to represent straight-chain or branched alkanediyl or oxaalkanediyl, in each case having 2 to 6 carbon atoms, and R⁵ can also represent straight-chain or branched alkoxy having 1 to 8 carbon atoms,
Y represents oxygen or sulphur,
R¹⁰ and R¹¹ represent hydrogen, Cₗ-C₆-alkyl, C₂-C₆-alkenyl, C₂-C₆-alkinyl, C₃-Cₛ-cycloalkyl, phenyl or benzyl.

## Revendications

1. 4,5-diamino-1,2,4-triazol-3-(thi)ones substituées répondant à la formule générale (I) 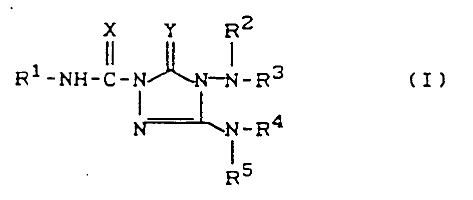 dans laquelle
R¹ représente un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanoalkyle contenant de 1 à 8 atomes de carbone, un groupe hydroxyalkyle contenant de 1 à 8 atomes de carbone et de 1 à 6 groupes hydroxyle, chacun de ces groupes étant à chaîne droite ou ramifiée, un groupe phé- noxyalkyle contenant de 1 à 4 atomes de carbone dans la fraction alkyle, un groupe alcoxyalkyle, un groupe alcoxycarbonylalkyle ou un groupe alcoxycarbonylalcényle contenant chacun jusqu'à 6 atomes de carbone dans les fractions individuelles alkyle ou alcényle, un groupe alkylaminoalkyle ou dialkylaminoalkyle contenant chacun de 1 à 6 atomes de carbone dans les fractions alkyle individuelles, un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou cycloalcényle et éventuellement de 1 à 6 atomes de carbone dans la fraction alkyle, ces groupes étant éventuellement substitués de 1 à 3 fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; et un groupe halogénalcényle à chaîne droite ou ramifiée contenant jusqu'à 4 atomes de carbone et de 1 à 5 atomes d'halogène identiques ou différents ou encore un groupe alcanediyle ou alcènediyle contenant chacun jusqu'à 4 atomes de carbone et étant chacun à liaison double; R¹ représente, en outre, un groupe hétérocyclylalkyle contenant de 1 à 6 atomes de carbone dans la fraction alkyle à chaîne droite ou ramifiée et de 2 à 9 atomes de carbone, ainsi que de 1 à 3 hétéro-atomes - en particulier un atome d'azote, un atome d'oxygène et/ou un atome de soufre - dans la fraction hétérocyclyle, éventuellement substitué de 1 à 3 fois de manière identique ou différente dans la fraction hétérocyclyle, dans lesquels, comme substituants, entrent en ligne de compte : un atome d'halogène, un groupe cyano, un groupe nitro, ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée; R¹ représente, en outre, un groupe alcoxy contenant de 1 à 8 atomes de carbone, un groupe alcényloxy contenant de 2 à 8 atomes de carbone ou un groupe alcynyloxy contenant de 2 à 8 atomes de carbone, chacun étant à chaîne droite ou ramifiée; ou encore un groupe aralkyle, un groupe aroyle, un groupe aryle, un groupe aralkyloxy ou un groupe aryloxy contenant chacun de 6 à 10 atomes de carbone dans la fraction aryle et éventuellement de 1 à 8 atomes de carbone dans la fraction alkyle à chaîne droite ou ramifiée, chacun de ces groupes étant éventuellement substitué de 1 à 3 fois de manière identique ou différente, dans lesquels, comme substituants du groupe alkyle, on envisage éventuellement un atome d'halogène et un groupe cyano, et dans lesquels, comme substituants du groupe aryle, on envisage chaque fois : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkyl- sulfonyle, un groupe alcanoyle ou un groupe alcoxycarbonyle contenant chacun de 1 à 6 atomes de carbone dans la fraction alkyle et éventuellement de 1 à 9 atomes d'halogène identiques ou différents, chacun de ces groupes étant à chaîne droite ou ramifiée, un groupe cycloalkyle contenant de 3 à 6 atomes de carbone et un groupe phénoxy; ou encore R¹ représente un groupe benzyle contenant un groupe -0-CH₂-O- condensé dans la fraction phényle,
R², R³, R⁴ et R⁵ sont identiques ou différents et représentent, indépendamment l'un de l'autre, un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanoalkyle contenant de 1 à 8 atomes de carbone, un groupe alcoxyalkyle ou alkylthioalkyle contenant chacun jusqu'à 4 atomes de carbone dans les fractions alkyle individuelles, chacun de ces groupes étant à chaîne droite ou ramifiée, un groupe cycloalkyle ou un groupe cycloalkylalkyle contenant chacun de 3 à 8 atomes de carbone dans la fraction cycloalkyle et éventuellement de 1 à 6 atomes de carbone dans la fraction alkyle, chacun de ces groupes étant éventuellement substitué de 1 à 3 fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée, les radicaux R² à R⁵ représentant, en outre, un groupe aryle ou un groupe aralkyle contenant chacun 6 ou 10 atomes de carbone dans la fraction aryle et éventuellement de 1 à 4 atomes de carbone dans la fraction alkyle à chaîne droite ou ramifiée, chacun de ces groupes étant éventuellement substitué de 1 à 3 fois de manière identique ou différente, dans lesquels, comme substituants, on envisage : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halo- génalkylsulfonyle, un groupe alcanoyle ou un groupe alcoxycarbonyle contenant chacun de 1 à 6 atomes de carbone dans la fraction alkyle et éventuellement de 1 à 9 atomes d'halogène, chacun de ces groupes étant à chaîne droite ou ramifiée, dans lesquels, en outre, chaque fois deux de ces radicaux - R² et R³ ou R⁴ et R^{5 -} peuvent représenter ensemble un groupe oxaalcanediyle ou un groupe alcanediyle à chaîne droite ou ramifiée contenant chacun de 2 à 6 atomes de carbone, et R⁵ peut également représenter un groupe alcoxy à chaîne droite ou ramifiée contenant de 1 à 8 atomes de carbone,
X représente un atome d'oxygène ou un atome de soufre et
Y représente un atome d'oxygène ou un atome de soufre.

2. 4,5-diamino-1,2,4-triazol-3-(thi)ones substituées de formule (I) selon la revendication 1, dans lesquelles
R¹ représente un atome d'hydrogène; un groupe méthyle, un groupe éthyle, un groupe n- ou i- propyle, un groupe n-, i-, s- ou t-butyle; un groupe pentyle, un groupe hexyle, un groupe heptyle, un groupe octyle, un groupe nonyle, un groupe décyle, un groupe dodécyle, chacun étant à chaîne droite ou ramifiée; un groupe allyle; un groupe butényle, un groupe pentényle ou un groupe hexényle, chacun étant à chaîne droite ou ramifiée; un groupe propargyle; un groupe butynyle, un groupe pentynyle ou un groupe hexynyle, chacun étant à chaîne droite ou ramifiée; un groupe halogénalkyle à chaîne droite ou ramifiée contenant de 1 à 8 atomes de carbone et de 1 à 9 atomes d'halogène identiques ou différents; un groupe halogénalcényle ou un groupe halogénalcynyle contenant chacun de 3 à 8 atomes de carbone et de 1 à 3 atomes d'halogène, chacun de ces groupes étant à chaîne droite ou ramifiée; un groupe cyanoalkyle contenant de 1 à 6 atomes de carbone dans la fraction alkyle, un groupe hydroxyalkyle contenant de 1 à 6 atomes de carbone et de 1 à 3 groupes hydroxyle, un groupe alcoxyalkyle, un groupe alcoxycarbonylalkyle ou un groupe alcoxycarbonylalcényle, un groupe alkylaminoalkyle ou un groupe dialkylaminoalkyle contenant chacun jusqu'à 4 atomes de carbone dans les fractions alkyle ou alcényle individuelles, chacun de ces groupes étant à chaîne droite ou ramifiée; ou encore représente un groupe cyclopropyle, un groupe cy- clopropylméthyle, un groupe cyclopropyléthyle, un groupe cyclopentyle, un groupe cyclohexyle, un groupe cycloheptyle, un groupe cyclooctyle, un groupe cyclohexylméthyle, un groupe cyclohexyléthyle, un groupe cyclohexényle ou un groupe cyclohexénylméthyle, chacun de ces groupes étant éventuellement substitué de 1 à 3 fois de manière identique ou différente, dans lesquels, comme substituants, on envisage chaque fois : un atome de fluor, un atome de chlore, un atome de brome, un groupe méthyle, un groupe éthyle, un groupe n- ou i-propyle, un groupe n-, i-, s- ou t-butyle, un groupe cyano, un groupe méthane- diyle, un groupe éthanediyle, un groupe butanediyle ou un groupe butadiènediyle ou encore un groupe dichloroallyle;
R¹ représente, en outre, un groupe hétérocyclylméthyle, un groupe hétérocyclylpropyle ou un groupe hétérocyclyléthyle éventuellement substitué de 1 à 3 fois de manière identique ou différente dans la fraction hétérocyclyle, dans lesquels, comme composés hétérocycliques, on envisage chaque fois : 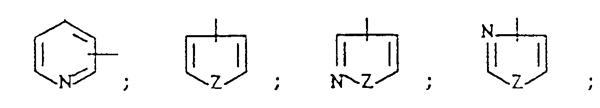 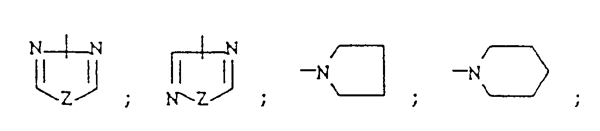 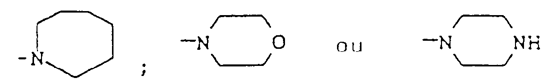 dans lesquels Z représente chaque fois un atome d'oxygène ou un atome de soufre, et dans lesquels, comme substituants, on envisage chaque fois : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy ou un groupe trifluorométhylthio;
R¹ représente, en outre, un groupe alcoxy contenant de 1 à 6 atomes de carbone, un groupe alcényloxy contenant de 3 à 6 atomes de carbone ou un groupe alcynyloxy contenant de 3 à 6 atomes de carbone, chacun de ces groupes étant à chaîne droite ou ramifiée; ou représente un groupe benzyle, un groupe phényléthyle, un groupe phénylpropyle, un groupe phénylbutyle, un groupe phénylpentyle, un groupe phénylhexyle, un groupe phénylheptyle, un groupe phénylcyanométhyle, un groupe phénylcya- noéthyle, un groupe phénylcyanopropyle, un groupe benzyloxy, un groupe phényléthyloxy, un groupe phénoxy, un groupe benzoyle, un groupe phényle ou un groupe naphtyle, chacun de ces groupes étant éventuellement substitué de 1 à 3 fois de manière identique ou différente, dans lesquels, comme substituants du groupe phényle, on envisage chaque fois : un atome de fluor, un atome de chlore, un atome de brome, un groupe hydroxyle, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy, un groupe trifluorométhylthio, un groupe trifluorométhylsulfinyle, un groupe trifluorométhylsulfonyle, un groupe méthylsulfinyle, un groupe méthylsulfonyle, un groupe acétyle, un groupe propionyle, un groupe méthoxycarbonyle, un groupe éthoxycarbonyle, un groupe cyclohexyle ou un groupe phénoxy,
R² représente un atome d'hydrogène, un groupe méthyle, un groupe éthyle, un groupe propyle, un groupe isopropyle, un groupe butyle, un groupe isobutyle, un groupe sec.-butyle, un groupe allyle, un groupe propargyle, un groupe cyanoéthyle, un groupe méthoxyéthyle, un groupe cyclopropyle, un groupe cyclopentyle, un groupe cyclohexyle, un groupe phényle ou un groupe benzyle,
R³ représente un atome d'hydrogène, un groupe méthyle, un groupe éthyle, un groupe propyle, un groupe butyle, un groupe allyle, un groupe propargyle, un groupe cyanoéthyle, un groupe méthoxyéthyle ou encore un groupe benzyle éventuellement substitué par un atome de chlore ou bien représente, conjointement avec R², un groupe butane-1,4-diyle ou un groupe pentane-1,5-diyle,
R⁴ représente un atome d'hydrogène, un groupe méthyle, un groupe éthyle, un groupe propyle, un groupe isopropyle, un groupe butyle, un groupe isobutyle, un groupe sec.-butyle, un groupe allyle, un groupe propargyle, un groupe cyanoéthyle, un groupe méthoxyéthyle, un groupe cyclopropyle, un groupe cyclopentyle, un groupe cyclohexyle, un groupe phényle ou un groupe benzyle,
R⁵ représente un atome d'hydrogène, un groupe méthyle, un groupe éthyle, un groupe propyle, un groupe butyle, un groupe allyle, un groupe propargyle, un groupe cyanoéthyle, un groupe méthoxyéthyle; un groupe méthoxy ou un groupe benzyle, ou bien représente, conjointement avec R⁴, un groupe butane-1,4-diyle, un groupe pentane-1,5-diyle ou un groupe 3-oxapentane-1,5-diyle,
X représente un atome d'oxygène ou un atome de soufre, et
Y représente un atome d'oxygène ou un atome de soufre.

3. Procédé pour la préparation de 4,5-diamino-1,2,4-triazol-3-(thi)ones substituées répondant à la formule générale (I) 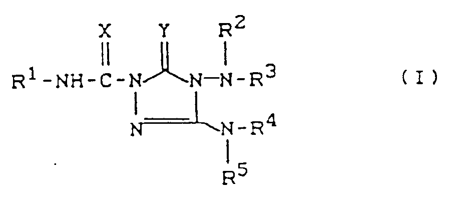 dans laquelle R¹, R², R³, R⁴, R⁵, X et Y ont les significations mentionnées à la revendication 1,
caractérisé en ce qu'on fait réagir
(a) des 4,5-diamino-1,2,4-triazol-3-(thi)ones de formule générale (II) 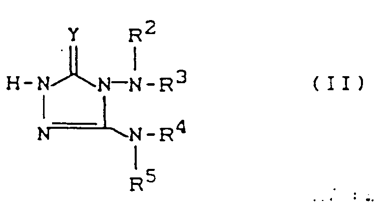 dans laquelle
R², R³, R⁴, R⁵ et Y ont les significations mentionnées ci-dessus,
avec des iso(thio)cyanates répondant à la formule générale (III) 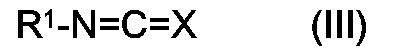 dans laquelle
R¹ et X ont les significations mentionnées ci-dessus,
éventuellement en présence d'un diluant et éventuellement en présence d'un adjuvant réactionnel,
ou en ce qu'on fait réagir
(b) des 4-alkylidènamino-5-amino-1,2,4-triazol-3-(thi)ones de formule générale (IV) 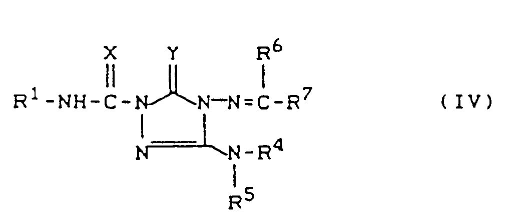 dans laquelle
R¹, R⁴, R⁵, X et Y ont les significations mentionnées ci-dessus, et
R⁶ et R⁷ sont identiques ou différents et représentent, indépendamment l'un de l'autre, un atome d'hydrogène, un groupe alkyle à chaîne droite ou ramifiée contenant de 1 à 4 atomes de carbone, un groupe phényle ou un groupe benzyle
avec des acides aqueux éventuellement en présence de solvants organiques,
ou bien en ce qu'on fait réagir
(c) des 1,2,4-triazol-3-(thi)ones substituées de formule générale (V) 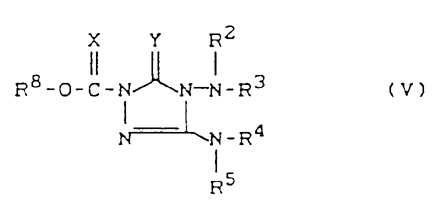 dans laquelle
R², R³, R⁴, R⁵, X et Y ont les significations mentionnées ci-dessus, et
R⁸ représente un groupe méthyle, un groupe éthyle, un groupe propyle, un groupe isopropyle, un groupe butyle, un groupe isobutyle, un groupe sec.butyle, un groupe tert.-butyle, un groupe phényle ou un groupe benzyle
avec des composés amino répondant à la formule générale (VI) 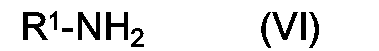 dans laquelle
R¹ a la signification mentionnée ci-dessus,
éventuellement en présence d'un diluant et éventuellement en présence d'un adjuvant réactionnel,
ou bien en ce qu'on fait réagir
(d) des 4,5-diamino-1,2,4-triazol-3-(thi)ones de formule générale (II) 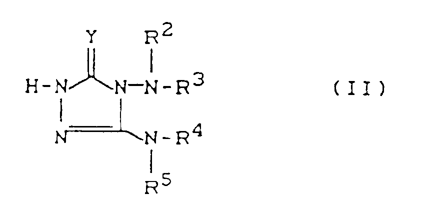 dans laquelle
R², R³, R⁴, R⁵ et Y ont les significations indiquées ci-dessus,
avec des (thio)uréthannes répondant à la formule générale (VII) 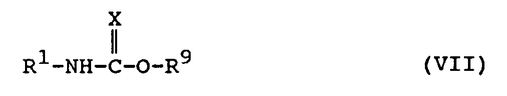 dans laquelle
R¹ et X ont les significations indiquées ci-dessus, et
R⁹ représente un groupe méthyle, un groupe éthyle, un groupe propyle, un groupe isopropyle, un groupe butyle, un groupe isobutyle, un groupe sec.butyle, un groupe tert.-butyle, un groupe phényle ou un groupe benzyle,
éventuellement en présence d'un diluant et éventuellement en présence d'un adjuvant réactionnel,
ou bien en ce qu'on fait réagir
(e) des 4-oxyalkylidènamino-5-amino-1,2,4-triazol-3-(thi)ones de formule générale (VIII) 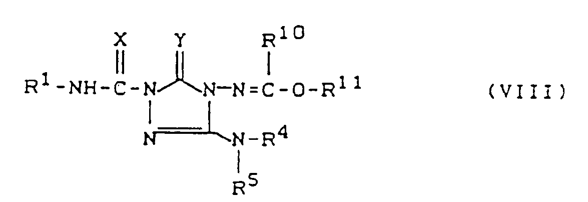 dans laquelle
R¹, R⁴, R⁵, X et Y ont les significations indiquées ci-dessus, et
R¹⁰ représente un atome d'hydrogène, un groupe méthyle, un groupe éthyle, un groupe propyle, un groupe phényle ou un groupe benzyle, et
R¹¹ représente un groupe méthyle, un groupe éthyle, un groupe propyle ou un groupe benzyle,
avec des complexes d'hydrures répondant à la formule générale (IX) 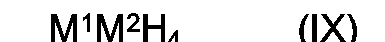 dans laquelle
M¹ représente le lithium, le sodium ou le potassium, et
M² représente le bore ou l'aluminium,
éventuellement en présence d'un diluant,
ou bien en ce qu'on fait réagir
(f) des 4-alkylidènamino-5-amino-1,2,4-triazol-3-(thi)ones de formule générale (IV) 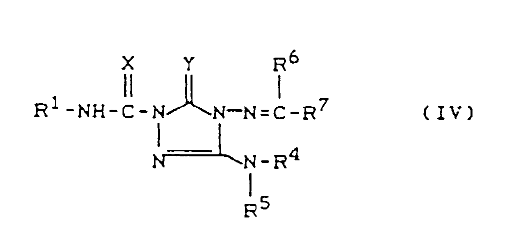 dans laquelle
R¹, R⁴, R⁵, X et Y ont les significations indiquées ci-dessus, et
R⁶ et R⁷ sont identiques ou différents et ont, indépendamment l'un de l'autre, les significations indiquées ci-dessus,
avec des agents de réduction, éventuellement en présence de catalyseurs et éventuellement en présence de diluants.

4. Agents herbicides caractérisés par une teneur en au moins une 4,5-diamino-1,2,4-triazol-3-(thi)one substituée de formule (I) selon la revendication 1 ou 3.

5. Procédé pour lutter contre des plantes non désirées, caractérisé en ce qu'on laisse agir des 4,5-diamino-1,2,4-triazol-3-(thi)ones substituées de formule (I) selon la revendication 1 ou 3, sur les plantes et/ou sur leur biotope.

6. Utilisation de 4,5-diamino-1,2,4-triazol-3-(thi)ones substituées de formule (I) selon la revendication 1 ou 3, pour lutter contre des plantes non désirées.

7. Procédé pour la préparation d'agents herbicides, caractérisé en ce qu'on mélange des 4,5-diamino-1,2,4-triazol-3-(thi)ones substituées de formule (I) selon la revendication 1 ou 4, avec des diluants et/ou des substances tensioactives.

8. 4,5-diamino-1,2,4-triazol-3-ones répondant à la formule générale (Ila) 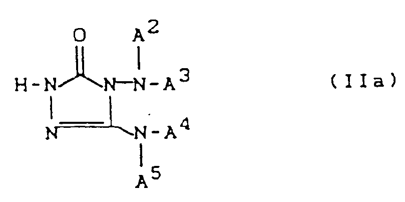 dans laquelle
A², A³, A⁴ etA⁵ sont identiques ou différents et représentent un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou un groupe halogénalcynyle contenant chacun de 2 à 8 atomes de carbone et de 1 à 15 ou 13 atomes d'halogène identiques ou différents, un groupe cyanoalkyle contenant de 1 à 8 atomes de carbone, un groupe alcoxyalkyle ou un groupe alkylthioalkyle contenant chacun jusqu'à 4 atomes de carbone dans les fractions alkyle individuelles, chacun de ces groupes étant à chaîne droite ou ramifiée; un groupe cycloalkyle ou un groupe cycloalkylalkyle contenant chacun de 3 à 8 atomes de carbone dans la fraction cycloalkyle et éventuellement de 1 à 6 atomes de carbone dans la fraction alkyle, chacun de ces groupes étant éventuellement substitué de 1 à 3 fois de manière identique ou différente, dans lesquels, comme substituants, entrent chaque fois en ligne de compte : un atome d'halogène, un groupe cyano; ainsi qu'un groupe alkyle ou un groupe halogénalkyle, chacun étant à chaîne droite ou ramifiée et contenant chacun de 1 à 4 atomes de carbone et éventuellement de 1 à 9 atomes d'halogène identiques ou différents, dans lesquels, en outre, chaque fois deux de ces radicaux - A² et A³ ou A⁴ et A5 ⁻ peuvent représenter ensemble un groupe oxaalcanediyle ou un groupe alcanediyle à chaîne droite ou ramifiée contenant chacun de 2 à 6 atomes de carbone, et A5 peut également représenter un groupe alcoxy à chaîne droite ou ramifiée contenant de 1 à 8 atomes de carbone, avec cette mesure qu'au moins un des radicaux A², A³, A⁴ ou A5 est différent d'un atome d'hydrogène.

9. 4-alkylidènamino-5-amino-1,2,4-triazol-3-(thi)ones répondant à la formule générale (IV) 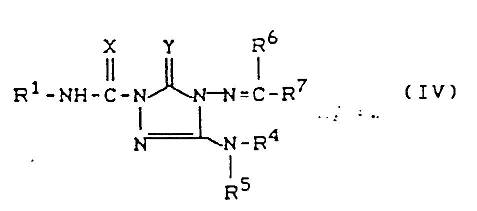 dans laquelle
R¹ représente un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanoalkyle contenant de 1 à 8 atomes de carbone, un groupe hydroxyalkyle contenant de 1 à 8 atomes de carbone et de 1 à 6 groupes hydroxyle, chacun de ces groupes étant à chaîne droite ou ramifiée, un groupe phé- noxyalkyle contenant de 1 à 4 atomes de carbone dans la fraction alkyle, un groupe alcoxyalkyle, un groupe alcoxycarbonylalkyle ou un groupe alcoxycarbonylalcényle contenant chacun jusqu'à 6 atomes de carbone dans les fractions individuelles alkyle ou alcényle, un groupe alkylaminoalkyle ou dialkylaminoalkyle contenant chacun de 1 à 6 atomes de carbone dans les fractions alkyle individuelles, un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou cycloalcényle et éventuellement de 1 à 6 atomes de carbone dans la fraction alkyle, ces groupes étant éventuellement substitués de 1 à 3 fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; et un groupe halogénalcényle à chaîne droite ou ramifiée contenant jusqu'à 4 atomes de carbone et de 1 à 5 atomes d'halogène identiques ou différents ou encore un groupe alcanediyle ou alcènediyle contenant chacun jusqu'à 4 atomes de carbone et étant chacun à liaison double; R¹ représente, en outre, un groupe hétérocyclylalkyle contenant de 1 à 6 atomes de carbone dans la fraction alkyle à chaîne droite ou ramifiée et de 2 à 9 atomes de carbone, ainsi que de 1 à 3 hétéro-atomes - en particulier un atome d'azote, un atome d'oxygène et/ou un atome de soufre - dans la fraction hétérocyclyle, éventuellement substitué de 1 à 3 fois de manière identique ou différente dans la fraction hétérocyclyle, dans lesquels, comme substituants, entrent en ligne de compte : un atome d'halogène, un groupe cyano, un groupe nitro, ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée; R¹ représente, en outre, un groupe alcoxy contenant de 1 à 8 atomes de carbone, un groupe alcényloxy contenant de 2 à 8 atomes de carbone ou un groupe alcynyloxy contenant de 2 à 8 atomes de carbone, chacun étant à chaîne droite ou ramifiée; ou encore un groupe aralkyle, un groupe aroyle, un groupe aryle, un groupe aralkyloxy ou un groupe aryloxy contenant chacun de 6 à 10 atomes de carbone dans la fraction aryle et éventuellement de 1 à 8 atomes de carbone dans la fraction alkyle à chaîne droite ou ramifiée, chacun de ces groupes étant éventuellement substitué de 1 à 3 fois de manière identique ou différente, dans lesquels, comme substituants du groupe alkyle, on envisage éventuellement un atome d'halogène et un groupe cyano, et dans lesquels, comme substituants du groupe aryle, on envisage chaque fois : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkyl- sulfonyle, un groupe alcanoyle ou un groupe alcoxycarbonyle contenant chacun de 1 à 6 atomes de carbone dans la fraction alkyle et éventuellement de 1 à 9 atomes d'halogène identiques ou différents, chacun de ces groupes étant à chaîne droite ou ramifiée, un groupe cycloalkyle contenant de 3 à 6 atomes de carbone et un groupe phénoxy; ou encore R¹ représente un groupe benzyle contenant un groupe -0-CH₂-O- condensé dans la fraction phényle,
R⁴ et R⁵ sont identiques ou différents et représentent, indépendamment l'un de l'autre, un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'hàlogène identiques ou différents, un groupe halogénalcényle ou halogénalcynyle contenant chacun de 2 à 8 atomes de carbone et de 1 à 15 ou 13 atomes d'halogène identiques ou différents, un groupe cyanoalkyle contenant de 1 à 8 atomes de carbone, un groupe alcoxyalkyle ou alkylthioalkyle contenant chacun jusqu'à 4 atomes de carbone dans les fractions alkyle individuelles, chacun de ces groupes étant à chaîne droite ou ramifiée, un groupe cycloalkyle ou un groupe cycloalkylalkyle contenant chacun de 3 à 8 atomes de carbone dans la fraction cycloalkyle et éventuellement de 1 à 6 atomes de carbone dans la fraction alkyle, chacun de ces groupes étant éventuellement substitué de 1 à 3 fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée, les radicaux R⁴ et R⁵ représentant, en outre, un groupe aryle ou un groupe aralkyle contenant chacun 6 ou 10 atomes de carbone dans la fraction aryle et éventuellement de 1 à 4 atomes de carbone dans la fraction alkyle à chaîne droite ou ramifiée, chacun de ces groupes étant éventuellement substitué de 1 à 3 fois de manière identique ou différente, dans lesquels, comme substituants, on envisage : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halo- génalkylsulfonyle, un groupe alcanoyle ou un groupe alcoxycarbonyle contenant chacun de 1 à 6 atomes de carbone dans la fraction alkyle et éventuellement de 1 à 9 atomes d'halogène, chacun de ces groupes étant à chaîne droite ou ramifiée, dans lesquels, en outre, (chaque fois deux de ces radicaux - R² et R³ ou) R⁴ et R^{5 -} peuvent représenter ensemble un groupe oxaalcanediyle ou un groupe alcanediyle à chaîne droite ou ramifiée contenant chacun de 2 à 6 atomes de carbone, et R⁵ peut également représenter un groupe alcoxy à chaîne droite ou ramifiée contenant de 1 à 8 atomes de carbone,
X représente un atome d'oxygène ou un atome de soufre et
Y représente un atome d'oxygène ou un atome de soufre.
R⁶ et R⁷ sont identiques ou différents et représentent, indépendamment l'un de l'autre, un atome d'hydrogène, un groupe alkyle à chaîne droite ou ramifiée contenant de 1 à 4 atomes de carbone, un groupe phényle ou un groupe benzyle.

10. 4-alkylidènamino-5-amino-1,2,4-triazol-3-(thi)ones de formule (XXI) 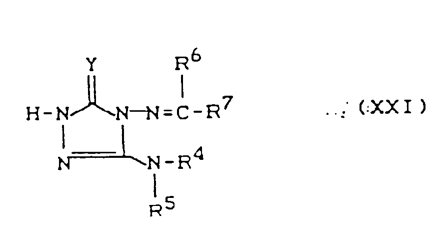 dans laquelle
R⁴ et R⁵ sont identiques ou différents et représentent, indépendamment l'un de l'autre, un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanoalkyle contenant de 1 à 8 atomes de carbone, un groupe alcoxyalkyle ou alkylthioalkyle contenant chacun jusqu'à 4 atomes de carbone dans les fractions alkyle individuelles, chacun de ces groupes étant à chaîne droite ou ramifiée, un groupe cycloalkyle ou un groupe cycloalkylalkyle contenant chacun de 3 à 8 atomes de carbone dans la fraction cycloalkyle et éventuellement de 1 à 6 atomes de carbone dans la fraction alkyle, chacun de ces groupes étant éventuellement substitué de 1 à 3 fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée, les radicaux R⁴ et R⁵ représentant, en outre, un groupe aryle ou un groupe aralkyle contenant chacun 6 ou 10 atomes de carbone dans la fraction aryle et éventuellement de 1 à 4 atomes de carbone dans la fraction alkyle à chaîne droite ou ramifiée, chacun de ces groupes étant éventuellement substitué de 1 à 3 fois de manière identique ou différente, dans lesquels, comme substituants, on envisage : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halo- génalkylsulfonyle, un groupe alcanoyle ou un groupe alcoxycarbonyle contenant chacun de 1 à 6 atomes de carbone dans la fraction alkyle et éventuellement de 1 à 9 atomes d'halogène, chacun de ces groupes étant à chaîne droite ou ramifiée, dans lesquels, en outre, R⁴ et R⁵ peuvent représenter ensemble un groupe oxaalcanediyle ou un groupe alcanediyle à chaîne droite ou ramifiée contenant chacun de 2 à 6 atomes de carbone, et R⁵ peut également représenter un groupe alcoxy à chaîne droite ou ramifiée contenant de 1 à 8 atomes de carbone, avec cette mesure que R⁴ et/ou R⁵ sont différents d'un atome d'hydrogène,
Y représente un atome d'oxygène ou un atome de soufre.
R⁶ et R⁷ sont identiques ou différents et représentent, indépendamment l'un de l'autre, un atome d'hydrogène, un groupe alkyle à chaîne droite ou ramifiée contenant de 1 à 4 atomes de carbone, un groupe phényle ou un groupe benzyle.

11. 2-oxy-(thio)-carbonyl-4-alkylidènamino-5-amino-1,2,4-triazol-3-(thi)ones de formule (XXIII) 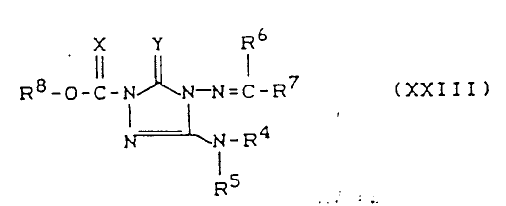 dans laquelle
R⁴ et R⁵ sont identiques ou différents et représentent, indépendamment l'un de l'autre, un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanoalkyle contenant de 1 à 8 atomes de carbone, un groupe alcoxyalkyle ou alkylthioalkyle contenant chacun jusqu'à 4 atomes de carbone dans les fractions alkyle individuelles, chacun de ces groupes étant à chaîne droite ou ramifiée, un groupe cycloalkyle ou un groupe cycloalkylalkyle contenant chacun de 3 à 8 atomes de carbone dans la fraction cycloalkyle et éventuellement de 1 à 6 atomes de carbone dans la fraction alkyle, chacun de ces groupes étant éventuellement substitué de 1 à 3 fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée, les radicaux R⁴ et R⁵ représentant, en outre, un groupe aryle ou un groupe aralkyle contenant chacun 6 ou 10 atomes de carbone dans la fraction aryle et éventuellement de 1 à 4 atomes de carbone dans la fraction alkyle à chaîne droite ou ramifiée, chacun de ces groupes étant éventuellement substitué de 1 à 3 fois de manière identique ou différente, dans lesquels, comme substituants, on envisage : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halo- génalkylsulfonyle, un groupe alcanoyle ou un groupe alcoxycarbonyle contenant chacun de 1 à 6 atomes de carbone dans la fraction alkyle et éventuellement de 1 à 9 atomes d'halogène, chacun de ces groupes étant à chaîne droite ou ramifiée, dans lesquels, en outre, R⁴ et R⁵ peuvent représenter ensemble un groupe oxaalcanediyle ou un groupe alcanediyle à chaîne droite ou ramifiée contenant chacun de 2 à 6 atomes de carbone, et R⁵ peut également représenter un groupe alcoxy à chaîne droite ou ramifiée contenant de 1 à 8 atomes de carbone,
X représente un atome d'oxygène ou un atome de soufre et
Y représente un atome d'oxygène ou un atome de soufre.
R⁶ et R⁷ sont identiques ou différents et représentent, indépendamment l'un de l'autre, un atome d'hydrogène, un groupe alkyle à chaîne droite ou ramifiée contenant de 1 à 4 atomes de carbone, un groupe phényle ou un groupe benzyle.
R⁸ représente un groupe méthyle, un groupe éthyle, un groupe propyle, un groupe isopropyle, un groupe butyle, un groupe isobutyle, un groupe sec.butyle, un groupe tert.-butyle, un groupe phényle ou un groupe benzyle.

12. Composés d'oxyalkylidènes de formule (XXV) 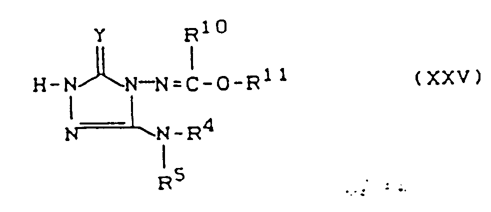 dans laquelle
R⁴ et R⁵ sont identiques ou différents et représentent, indépendamment l'un de l'autre, un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanoalkyle contenant de 1 à 8 atomes de carbone, un groupe alcoxyalkyle ou alkylthioalkyle contenant chacun jusqu'à 4 atomes de carbone dans les fractions alkyle individuelles, chacun de ces groupes étant à chaîne droite ou ramifiée, un groupe cycloalkyle ou un groupe cycloalkylalkyle contenant chacun de 3 à 8 atomes de carbone dans la fraction cycloalkyle et éventuellement de 1 à 6 atomes de carbone dans la fraction alkyle, chacun de ces groupes étant éventuellement substitué de 1 à 3 fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée, les radicaux R⁴ et R⁵ représentant, en outre, un groupe aryle ou un groupe aralkyle contenant chacun 6 ou 10 atomes de carbone dans la fraction aryle et éventuellement de 1 à 4 atomes de carbone dans la fraction alkyle à chaîne droite ou ramifiée, chacun de ces groupes étant éventuellement substitué de 1 à 3 fois de manière identique ou différente, dans lesquels, comme substituants, on envisage : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halo- génalkylsulfonyle, un groupe alcanoyle ou un groupe alcoxycarbonyle contenant chacun de 1 à 6 atomes de carbone dans la fraction alkyle et éventuellement de 1 à 9 atomes d'halogène, chacun de ces groupes étant à chaîne droite ou ramifiée, dans lesquels, en outre, R⁴ et R⁵ peuvent représenter ensemble un groupe oxaalcanediyle ou un groupe alcanediyle à chaîne droite ou ramifiée contenant chacun de 2 à 6 atomes de carbone, et R⁵ peut également représenter un groupe alcoxy à chaîne droite ou ramifiée contenant de 1 à 8 atomes de carbone,
Y représente un atome d'oxygène ou un atome de soufre.
R¹⁰ et pu représentent un atome d'hydrogène, un groupe alkyle en Ci-Ce, un groupe alcényle en C₂-C₆, un groupe alcynyle en C₂-C₆, un groupe cycloalkyle en C₃-C₆, un groupe phényle ou un groupe benzyle.
